# EUROPEAN PATENT SPECIFICATION

(11) **EP 3 913 000 B1**
(45) Date of publication and mention of the grant of the patent: **28.10.2026**
(21) Application number: 21168389.1
(22) Date of filing: 28.09.2016
(51) Int. Cl.: C07K 16/28, C07K 16/46, A61K 39/395, A61P 35/00

(54) **BISPECIFIC ANTI-CD19XCD3 T CELL ACTIVATING ANTIGEN BINDING MOLECULES**
BISPEZIFISCHE ANTI-CD19XCD3 ANTIGENBINDENDE MOLEKÜLE ZUR T-ZELLEN-AKTIVIERUNG
ANTI-CD19XCD3 MOLÉCULES BISPÉCIFIQUES DE LIAISON À L'ANTIGÈNE ACTIVANT DES LYMPHOCYTES T

(30) Priority: 02.10.2015 EP 15188093; 11.05.2016 EP 16169160
(43) Date of publication of application: 24.11.2021
(62) Document number(s) of the earlier application(s) in accordance with Art. 76 EPC: 16775194.0 / 3 356 407
(73) Proprietor: F. Hoffmann-La Roche AG, 4070 Basel (CH)
(72) Inventor: BACAC, Marina, 8046 Zuerich (CH); KLEIN, Christian, 8906 Bonstetten (CH); SCHAEFER, Wolfgang, 68199 Mannheim (DE); KLOSTERMANN, Stefan, 82061 Neuried (DE); IMHOF-JUNG, Sabine, 82152 Planegg (DE); MOLHOJ, Michael, 80686 Muenchen (DE); REGULA, Joerg Thomas, 81377 Muenchen (DE); UMAÑA, Pablo, 8832 Wollerau (CH); HERTER, Sylvia, 8105 Regensdorf (CH); NEUMANN, Christiane, 8166 Niederweningen (CH)
(74) Representative: Cueni, Leah Noëmi

(56) References cited:
- WO-A1-2004/106381
- WO-A1-2009/080254
- WO-A1-2014/131694
- WO-A1-2014/131711
- WO-A1-2015/101588
- WO-A2-2014/012085
- WO-A2-2015/006749
- REUSCH UWE ET AL: "A tetravalent bispecific TandAb (CD19/CD3), AFM11, efficiently recruits T cells for the potent lysis of CD19(+) tumor cells", MABS, LANDES BIOSCIENCE, US, vol. 7, no. 3, 4 May 2015 (2015-05-04), pages 584 - 604, XP009184710, ISSN: 1942-0870, DOI: 10.1080/19420862.2015.1029216
- DIRK NAGORSEN ET AL: "Immunomodulatory therapy of cancer with T cell-engaging BiTE antibody blinatumomab", EXPERIMENTAL CELL RESEARCH, ELSEVIER, AMSTERDAM, NL, vol. 317, no. 9, 10 March 2011 (2011-03-10), pages 1255 - 1260, XP028205663, ISSN: 0014-4827, [retrieved on 20110316], DOI: 10.1016/J.YEXCR.2011.03.010
- P. A. MOORE ET AL: "Application of dual affinity retargeting molecules to achieve optimal redirected T-cell killing of B-cell lymphoma", BLOOD, vol. 117, no. 17, 28 April 2011 (2011-04-28), pages 4542 - 4551, XP055031067, ISSN: 0006-4971, DOI: 10.1182/blood-2010-09-306449
- KIPRIYANOV S M ET AL: "Bispecific CD3 x CD19 diabody for T cell-mediated lysis of malignant human B cells", INTERNATIONAL JOURNAL OF CANCER, JOHN WILEY & SONS, INC, US, vol. 77, no. 5, 31 August 1998 (1998-08-31), pages 763 - 771, XP002115487, ISSN: 0020-7136, DOI: 10.1002/(SICI)1097-0215(19980831)77:5<763::AID-IJC16>3.0.CO;2-2
- HONEYCHURCH J ET AL: "Bispecific Ab therapy of B-cell lymphoma: target specificity of antibody derivatives appears critical in determining therapeutic outcome", CANCER IMMUNOLOGY, IMMUNOTHERAPY, SPRINGER, BERLIN/HEIDELBERG, vol. 45, no. 3-4, 1 November 1997 (1997-11-01), pages 171 - 173, XP002724814, ISSN: 0340-7004, DOI: 10.1007/S002620050425
- RIDGWAY ET AL: "'KNOBS-INTO-HOLES' ENGINEERING OF ANTIBODY CH3 DOMAINS FOR HEAVY CHAIN HETERODIMERIZATION", PROTEIN ENGINEERING, OXFORD UNIVERSITY PRESS, SURREY, GB, vol. 9, no. 7, 1 January 1996 (1996-01-01), pages 617 - 621, XP002084766, ISSN: 0269-2139

## Description

### Field of the Invention

The present invention generally relates to bispecific antigen binding molecules for activating T cells. In addition, the present invention relates to polynucleotides encoding such bispecific antigen binding molecules, and vectors and host cells comprising such polynucleotides. The invention further relates to methods for producing the bispecific antigen binding molecules of the invention, and to these bispecific antigen binding molecules for use in the treatment of disease.

### Background

The selective destruction of an individual cell or a specific cell type is often desirable in a variety of clinical settings. For example, it is a primary goal of cancer therapy to specifically destroy tumor cells, while leaving healthy cells and tissues intact and undamaged.

An attractive way of achieving this is by inducing an immune response against the tumor, to make immune effector cells such as natural killer (NK) cells or cytotoxic T lymphocytes (CTLs) attack and destroy tumor cells. CTLs constitute the most potent effector cells of the immune system, however they cannot be activated by the effector mechanism mediated by the Fc domain of conventional therapeutic antibodies.

In this regard, bispecific antibodies designed to bind with one "arm" to a surface antigen on target cells, and with the second "arm" to an activating, invariant component of the T cell receptor (TCR) complex, have become of interest in recent years. The simultaneous binding of such an antibody to both of its targets will force a temporary interaction between target cell and T cell, causing activation of any cytotoxic T cell and subsequent lysis of the target cell. Hence, the immune response is re-directed to the target cells and is independent of peptide antigen presentation by the target cell or the specificity of the T cell as would be relevant for normal MHC-restricted activation of CTLs. In this context it is crucial that CTLs are only activated when a target cell is presenting the bispecific antibody to them, i.e. the immunological synapse is mimicked. Particularly desirable are bispecific antibodies that do not require lymphocyte preconditioning or co-stimulation in order to elicit efficient lysis of target cells.

Several bispecific antibody formats have been developed and their suitability for T cell mediated immunotherapy investigated. Out of these, the so-called BiTE (bispecific T cell engager) molecules have been very well characterized and already shown some promise in the clinic (reviewed in Nagorsen and Bäuerle, Exp Cell Res 317, 1255-1260 (2011)). BiTEs are tandem scFv molecules wherein two scFv molecules are fused by a flexible linker. Further bispecific formats being evaluated for T cell engagement include diabodies (Holliger et al., Prot Eng 9, 299-305 (1996)) and derivatives thereof, such as tandem diabodies (Kipriyanov et al., J Mol Biol 293, 41-66 (1999)). A more recent development are the so-called DART (dual affinity retargeting) molecules, which are based on the diabody format but feature a C-terminal disulfide bridge for additional stabilization (Moore et al., Blood 117, 4542-51 (2011)). The so-called triomabs, which are whole hybrid mouse/rat IgG molecules and also currently being evaluated in clinical trials, represent a larger sized format (reviewed in Seimetz et al., Cancer Treat Rev 36, 458-467 (2010)). Other larger sized sized formats are disclosed e.g. in WO 2013/026833 or WO 2014/131694.

The variety of formats that are being developed shows the great potential attributed to T cell re-direction and activation in immunotherapy. The task of generating bispecific antibodies suitable therefor is, however, by no means trivial, but involves a number of challenges that have to be met related to efficacy, toxicity, applicability and produceability of the antibodies.

Small constructs such as, for example, BiTE molecules - while being able to efficiently crosslink effector and target cells - have a very short serum half life requiring them to be administered to patients by continuous infusion. IgG-like formats on the other hand - while having the great benefit of a long half life - suffer from toxicity associated with the native effector functions inherent to IgG molecules. Their immunogenic potential constitutes another unfavorable feature of IgG-like bispecific antibodies, especially non-human formats, for successful therapeutic development. Finally, a major challenge in the general development of bispecific antibodies has been the production of bispecific antibody constructs at a clinically sufficient quantity and purity, due to the mispairing of antibody heavy and light chains of different specificities upon co-expression, which decreases the yield of the correctly assembled construct and results in a number of non-functional side products from which the desired bispecific antibody may be difficult to separate.

Different approaches have been taken to overcome the chain association issue in bispecific antibodies (see e.g. Klein et al., mAbs 6, 653-663 (2012)). For example, the 'knobs-into-holes' strategy aims at forcing the pairing of two different antibody heavy chains by introducing mutations into the CH3 domains to modify the contact interface. On one chain bulky amino acids are replaced by amino acids with short side chains to create a 'hole'. Conversely, amino acids with large side chains are introduced into the other CH3 domain, to create a 'knob'. By coexpressing these two heavy chains (and two identical light chains, which have to be appropriate for both heavy chains), high yields of heterodimer ('knob-hole') versus homodimer ('hole-hole' or 'knob-knob') are observed (Ridgway, J.B., et al., Protein Eng. 9 (1996) 617-621; and WO 96/027011). The percentage of heterodimer could be further increased by remodeling the interaction surfaces of the two CH3 domains using a phage display approach and the introduction of a disulfide bridge to stabilize the heterodimers (Merchant, A.M., et al., Nature Biotech. 16 (1998) 677-681; Atwell, S., et al., J. Mol. Biol. 270 (1997) 26-35). New approaches for the knobs-into-holes technology are described in e.g. in EP 1870459 A1.

The 'knobs-into-holes' strategy does, however, not solve the problem of heavy chain-light chain mispairing, which occurs in bispecific antibodies comprising different light chains for binding to the different target antigens.

A strategy to prevent heavy chain-light chain mispairing is to exchange domains between the heavy and light chains of one of the binding arms of a bispecific antibody (see WO 2009/080251, WO 2009/080252, WO 2009/080253, WO 2009/080254 and Schaefer, W. et al, PNAS, 108 (2011) 11187-11191, which relate to bispecific IgG antibodies with a domain crossover).

Exchanging the heavy and light chain variable domains VH and VL in one of the binding arms of the bispecific antibody (WO2009/080252, see also Schaefer, W. et al, PNAS, 108 (2011) 11187-11191) clearly reduces the side products caused by the mispairing of a light chain against a first antigen with the wrong heavy chain against the second antigen (compared to approaches without such domain exchange). Nevertheless, these antibody preparations are not completely free of side products. The main side product is based on a Bence Jones-type interaction (Schaefer, W. et al, PNAS, 108 (2011) 11187-11191; in Fig. S1I of the Supplement). A further reduction of such side products is thus desirable to improve e.g. the yield of such bispecific antibodies.

The choice of target antigens and appropriate binders for both the T cell antigen and the target cell antigen is a further crucial aspect in the generation of T cell bispecific (TCB) antibodies for therapeutic application.

Human CD19 is a 95 kDa transmembrane protein (B-cell co-receptor) exclusively expressed on B-cells and on follicular dendritic cells. CD 19 is found in association with CD21 and CD81. CD19 and CD21 are required for normal B-cell differentiation (Carter, R.H., et al., Immunol. Res. 26 (2002) 45-54). CD19 is expressed on most B-cells (pan-B-cell marker) with the exception of stem cells and plasma cells, and is frequently expressed on most human B-cell malignancies (tumor associated antigen), such as lymphoma and leukemias except for multiple myeloma, e.g. in non-Hodgkin lymphoma and acute lymphoblastic leukemia. Antibodies against CD19 have been used in several clinical trials (see e. g. Hekman, A., et al., Cancer Immunol. Immunother. 32 (191) 364-372; Vlasfeld, L.T., et al., Cancer Immunol. Immunother. 40 (1995) 37-47; Conry, R.M., et al., J. Immunother. Emphasis Tumor Immunol. 18 (1995) 231-241; Manzke, O., et al., Int. J. Cancer 91 (2001) 516-522). In WO 2011/147834 antibodies against CD19 and uses thereof are reported. In WO 99/54440 and WO 2004/106381 pharmaceutical compositions comprising bispecific anti-CD3, anti-CD19 antibody constructs for the treatment of B-cell related disorders are reported.

The present invention provides novel, improved bispecific antigen binding molecules designed for T cell activation and re-direction, targeting CD3 and CD19, that combine good efficacy and produceability with low toxicity and favorable pharmacokinetic properties.

### Summary of the Invention

The present inventors have developed a novel T cell activating bispecific antigen binding molecule with unexpected, improved properties using a particular anti-CD19 antibody.

Thus, the present invention provides a T cell activating bispecific antigen binding molecule comprising
(a) a first antigen binding moiety which specifically binds to CD19, wherein the first antigen binding moiety is a conventional Fab molecule;
(b) a second antigen binding moiety which specifically binds to CD3, wherein the second antigen binding moiety is a crossover Fab molecule wherein the variable domains VL and VH of the Fab light chain and the Fab heavy chain are replaced by each other;
(c) a third antigen binding moiety which specifically binds to CD19, wherein the third antigen binding moiety is a conventional Fab molecule;
(d) an Fc domain composed of a first and a second subunit capable of stable association;

wherein in the first and the third antigen binding moiety in the constant domain CL the amino acid at position 124 is substituted independently by lysine (K), arginine (R) or histidine (H) (numbering according to Kabat) and the amino acid at position 123 is substituted independently by lysine (K), arginine (R) or histidine (H) (numbering according to Kabat), and in the constant domain CH1 the amino acid at position 147 is substituted independently by glutamic acid (E) or aspartic acid (D) (numbering according to Kabat EU index) and the amino acid at position 213 is substituted independently by glutamic acid (E) or aspartic acid (D) (numbering according to Kabat EU index); and
wherein the first and the third antigen binding moiety comprise a heavy chain variable region, particularly a humanized heavy chain variable region, comprising the heavy chain complementarity determining region (HCDR) 1 of SEQ ID NO: 50, the HCDR 2 of SEQ ID NO: 51 and the HCDR 3 of SEQ ID NO: 52, and a light chain variable region, particularly a humanized light chain variable region, comprising the light chain complementarity determining region (LCDR) 1 of SEQ ID NO: 53, the LCDR 2 of SEQ ID NO: 54 and the LCDR 3 of SEQ ID NO: 55; and
wherein (i) the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety, or (ii) the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety.

In one aspect, the first and the third antigen binding moiety comprise a heavy chain variable region comprising an amino acid sequence that is at least about 95%, 96%, 97%, 98%, 99% or 100% identical to the amino acid sequence of SEQ ID NO: 56 and a light chain variable region comprising an amino acid sequence that is at least about 95%, 96%, 97%, 98%, 99% or 100% identical to the amino acid sequence of SEQ ID NO: 57.

In a particular aspect, not more than one Fab molecule capable of specific binding to an activating T cell antigen is present in the T cell activating bispecific antigen binding molecule (i.e. the T cell activating bispecific antigen binding molecule provides monovalent binding to the activating T cell antigen).

According to the invention, the activating T cell antigen is CD3, particularly CD3 epsilon.

According to an aspect of the invention, the ratio of a desired bispecific antibody compared to undesired side products, in particular Bence Jones-type side products occurring in bispecific antibodies with a VH/VL domain exchange in one of their binding arms, can be improved by the introduction of charged amino acids with opposite charges at specific amino acid positions in the CH1 and CL domains (sometimes referred to herein as "charge modifications").

Thus, in the constant domain CL of the first and the third Fab molecule the amino acid at position 124 is substituted independently by lysine (K), arginine (R) or histidine (H) (numbering according to Kabat) (in one preferred aspect independently by lysine (K) or arginine (R)) and the amino acid at position 123 is substituted independently by lysine (K), arginine (R) or histidine (H) (numbering according to Kabat) (in one preferred aspect independently by lysine (K) or arginine (R)), and in the constant domain CH1 of the first and the third Fab molecule the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In a particular aspect, in the constant domain CL of the first and the third Fab molecule the amino acid at position 124 is substituted by lysine (K) (numbering according to Kabat) and the amino acid at position 123 is substituted by lysine (K) (numbering according to Kabat), and in the constant domain CH1 of the first and the third Fab molecule the amino acid at position 147 is substituted by glutamic acid (E) (numbering according to Kabat EU index) and the amino acid at position 213 is substituted by glutamic acid (E) (numbering according to Kabat EU index).

In another particular aspect, in the constant domain CL of the first and the third Fab molecule the amino acid at position 124 is substituted by lysine (K) (numbering according to Kabat) and the amino acid at position 123 is substituted by arginine (R) (numbering according to Kabat), and in the constant domain CH1 of the first and the third Fab molecule the amino acid at position 147 is substituted by glutamic acid (E) (numbering according to Kabat EU index) and the amino acid at position 213 is substituted by glutamic acid (E) (numbering according to Kabat EU index).

The T cell activating bispecific antigen binding molecule according to the invention comprises a third antigen binding moiety which specifically binds to CD19. In particular aspects, the third antigen binding moiety is identical to the first antigen binding moiety.

In particular aspects, the third and the first antigen binding moiety are each a Fab molecule and the third Fab molecule is identical to the first Fab molecule. In these aspects, the third Fab molecule thus comprises the same amino acid substitutions, if any, as the first Fab molecule. Like the first Fab molecule, the third Fab molecule is a conventional Fab molecule.

The T cell activating bispecific antigen binding molecule according to the invention comprises an Fc domain composed of a first and a second subunit capable of stable association.

The components of the T cell activating antigen binding molecule of the invention may be fused to each other directly or, preferably, via one or more suitable peptide linkers. Where fusion of a Fab molecule is to the N-terminus of a subunit of the Fc domain, it is typically via an immunoglobulin hinge region.

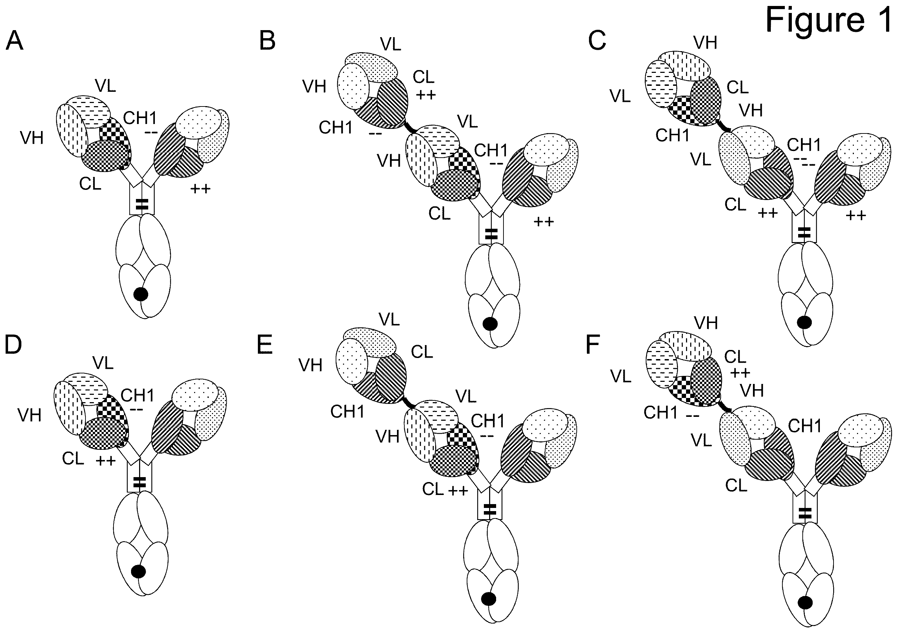

In the T cell activating antigen binding molecule of the invention, a third antigen binding moiety, specifically a third Fab molecule, is fused at the C-terminus of the Fab heavy chain to the N-terminus of the second subunit of the Fc domain. In one such aspect, the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second Fab molecule. In this aspect, the T cell activating bispecific antigen binding molecule essentially comprises an immunoglobulin molecule, wherein in one of the Fab arms the heavy and light chain variable regions VH and VL are exchanged/replaced by each other, and wherein an additional (conventional) Fab molecule is N-terminally fused to said Fab arm (see Figure 1B, E). In another such aspect, the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. In this aspect, the T cell activating bispecific antigen binding molecule essentially comprises an immunoglobulin molecule with an additional Fab molecule N-terminally fused to one of the immunoglobulin Fab arms, wherein in said additional Fab molecule the heavy and light chain variable regions VH and VL are exchanged/replaced by each other (see Figure 1C, F).

In a particular aspect, the immunoglobulin molecule comprised in the T cell activating bispecific antigen binding molecule according to the invention is an IgG class immunoglobulin. In an even more particular aspect the immunoglobulin is an IgG₁ subclass immunoglobulin. In another aspect, the immunoglobulin is an IgG₄ subclass immunoglobulin.

In the T cell activating bispecific antigen binding molecule according to the invention, the amino acid substitutions described hereinare in the CH1 and CL domains of the first and the third Fab molecule. In accordance with the concept of the invention, if amino acid substitutions as described herein are made in the first and the third Fab molecule, no such amino acid substitutions are made in the second Fab molecule.

In particular aspects of the T cell activating bispecific antigen binding molecule according to the invention, wherein amino acid substitutions as described herein are made in the first and the third Fab molecule, the constant domain CL of the first and the third Fab molecule is of kappa isotype. In some aspects, the constant domain CL of the first and the third Fab molecule and the constant domain CL of the second Fab molecule are of kappa isotype.

In particular aspects of the T cell activating bispecific antigen binding molecule, the Fc domain is an IgG Fc domain. In a specific aspect, the Fc domain is an IgG₁ Fc domain. In another specific aspect, the Fc domain is an IgG₄ Fc domain. In an even more specific aspect, the Fc domain is an IgG₄ Fc domain comprising the amino acid substitution S228P (Kabat numbering). In particular aspects the Fc domain is a human Fc domain.

In particular aspects, the Fc domain comprises a modification promoting the association of the first and the second Fc domain subunit. In a specific such aspect, an amino acid residue in the CH3 domain of the first subunit of the Fc domain is replaced with an amino acid residue having a larger side chain volume, thereby generating a protuberance within the CH3 domain of the first subunit which is positionable in a cavity within the CH3 domain of the second subunit, and an amino acid residue in the CH3 domain of the second subunit of the Fc domain is replaced with an amino acid residue having a smaller side chain volume, thereby generating a cavity within the CH3 domain of the second subunit within which the protuberance within the CH3 domain of the first subunit is positionable.

In a particular aspect the Fc domain exhibits reduced binding affinity to an Fc receptor and/or reduced effector function, as compared to a native IgG₁ Fc domain. In one aspect, the Fc domain comprises one or more amino acid substitution that reduces binding to an Fc receptor and/or effector function. In one aspect, the one or more amino acid substitution in the Fc domain that reduces binding to an Fc receptor and/or effector function is at one or more position selected from the group of L234, L235, and P329 (Kabat EU index numbering). In particular aspects, each subunit of the Fc domain comprises three amino acid substitutions that reduce binding to an Fc receptor and/or effector function wherein said amino acid substitutions are L234A, L235A and P329G (Kabat EU index numbering). In one such aspect, the Fc domain is an IgG₁ Fc domain, particularly a human IgG₁ Fc domain. In other aspects, each subunit of the Fc domain comprises two amino acid substitutions that reduce binding to an Fc receptor and/or effector function wherein said amino acid substitutions are L235E and P329G (Kabat EU index numbering). In one such aspect, the Fc domain is an IgG₄ Fc domain, particularly a human IgG₄ Fc domain. In one aspect, the Fc domain of the T cell activating bispecific antigen binding molecule is an IgG₄ Fc domain and comprises the amino acid substitutions L235E and S228P (SPLE) (Kabat EU index numbering).

In one aspect the Fc receptor is an Fcγ receptor. In one aspect the Fc receptor is a human Fc receptor. In one aspect, the Fc receptor is an activating Fc receptor. In a specific aspect, the Fc receptor is human FcγRIIa, FcγRI, and/or FcγRIIIa. In one aspect, the effector function is antibody-dependent cell-mediated cytotoxicity (ADCC).

In a specific aspect of the T cell activating bispecific antigen binding molecule according to the invention, the second antigen binding moiety comprises a heavy chain variable region comprising the heavy chain complementarity determining region (HCDR) 1 of SEQ ID NO: 4, the HCDR 2 of SEQ ID NO: 5, the HCDR 3 of SEQ ID NO: 6, and a light chain variable region comprising the light chain complementarity determining region (LCDR) 1 of SEQ ID NO: 8, the LCDR 2 of SEQ ID NO: 9 and the LCDR 3 of SEQ ID NO: 10. In an even more specific aspect, the second antigen binding moiety comprises a heavy chain variable region comprising an amino acid sequence that is at least about 95%, 96%, 97%, 98%, 99% or 100% identical to the amino acid sequence of SEQ ID NO: 3 and a light chain variable region comprising an amino acid sequence that is at least about 95%, 96%, 97%, 98%, 99% or 100% identical to the amino acid sequence of SEQ ID NO: 7. In one specific aspect, the second antigen binding moiety comprised in the T cell activating bispecific antigen binding molecule according to the invention comprises the heavy chain complementarity determining region (CDR) 1 of SEQ ID NO: 4, the heavy chain CDR 2 of SEQ ID NO: 5, the heavy chain CDR 3 of SEQ ID NO: 6, the light chain CDR 1 of SEQ ID NO: 8, the light chain CDR 2 of SEQ ID NO: 9 and the light chain CDR 3 of SEQ ID NO: 10. In an even more specific aspect, said second antigen binding moiety, particularly Fab molecule, comprises a heavy chain variable region comprising the amino acid sequence of SEQ ID NO: 3 and a light chain variable region comprising the amino acid sequence of SEQ ID NO: 7.

In the T cell activating bispecific antigen binding molecule according to the invention, the first and third antigen binding moiety comprises the heavy chain complementarity determining region (CDR) 1 of SEQ ID NO: 50, the heavy chain CDR 2 of SEQ ID NO: 51, the heavy chain CDR 3 of SEQ ID NO: 52, the light chain CDR 1 of SEQ ID NO: 53, the light chain CDR 2 of SEQ ID NO: 54 and the light chain CDR 3 of SEQ ID NO: 55. In a more specific aspect, the first and the third antigen binding moiety comprises a heavy chain variable region comprising an amino acid sequence that is at least about 95%, 96%, 97%, 98%, 99% or 100% identical to the amino acid sequence of SEQ ID NO: 56 and a light chain variable region comprising an amino acid sequence that is at least about 95%, 96%, 97%, 98%, 99% or 100% identical to the amino acid sequence of SEQ ID NO: 57. In an even more specific aspect, said first and said third antigen binding moiety comprises a heavy chain variable region comprising the amino acid sequence of SEQ ID NO: 56 and a light chain variable region comprising the amino acid sequence of SEQ ID NO: 57.

According to another aspect of the invention there is provided one or more isolated polynucleotide(s) encoding a T cell activating bispecific antigen binding molecule of the invention. The invention further provides one or more expression vector(s) comprising the isolated polynucleotide(s) of the invention, and a host cell comprising the isolated polynucleotide(s) or the expression vector(s) of the invention. In some aspects the host cell is a eukaryotic cell, particularly a mammalian cell.

The invention also provides a method of producing the T cell activating bispecific antigen binding molecule capable of specific binding to CD19 and CD3, comprising the steps of a) culturing the host cell of the invention under conditions suitable for the expression of the T cell activating bispecific antigen binding molecule and b) recovering the T cell activating bispecific antigen binding molecule. The invention also encompasses a T cell activating bispecific antigen binding molecule produced by the method of the invention.

The invention further provides a pharmaceutical composition comprising the T cell activating bispecific antigen binding molecule of the invention and a pharmaceutically acceptable carrier.

Also encompassed by the invention are methods of using the T cell activating bispecific antigen binding molecule and pharmaceutical composition of the invention. The invention also provides a T cell activating bispecific antigen binding molecule or a pharmaceutical composition of the invention for use as a medicament. Furthermore, the invention provides a T cell activating bispecific antigen binding molecule or a pharmaceutical composition according to the invention for use in the treatment of a disease in an individual in need thereof. In a specific aspect the disease is cancer.

In any of the above aspects the individual preferably is a mammal, particularly a human.

### Brief Description of the Drawings

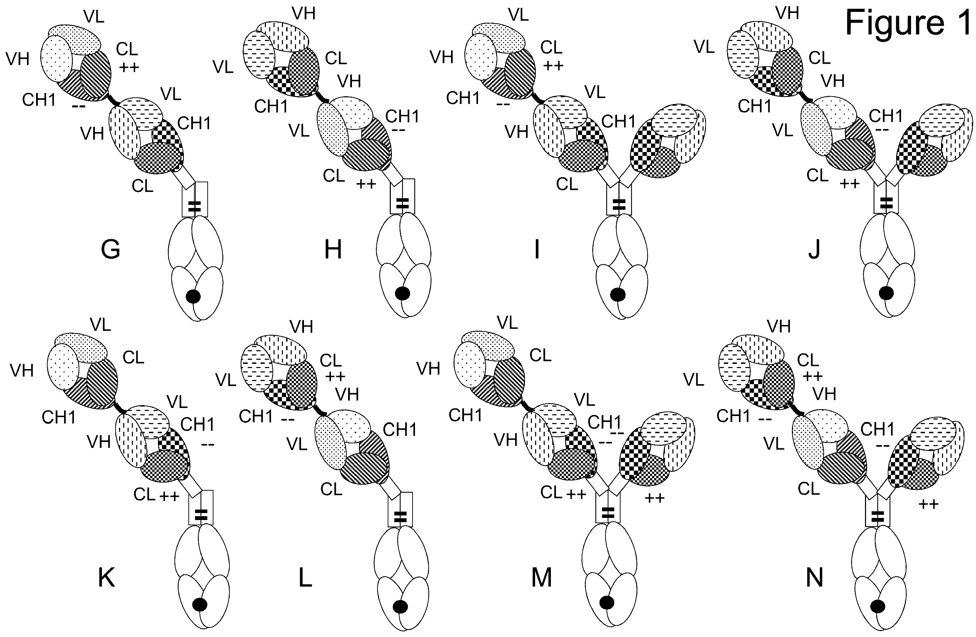

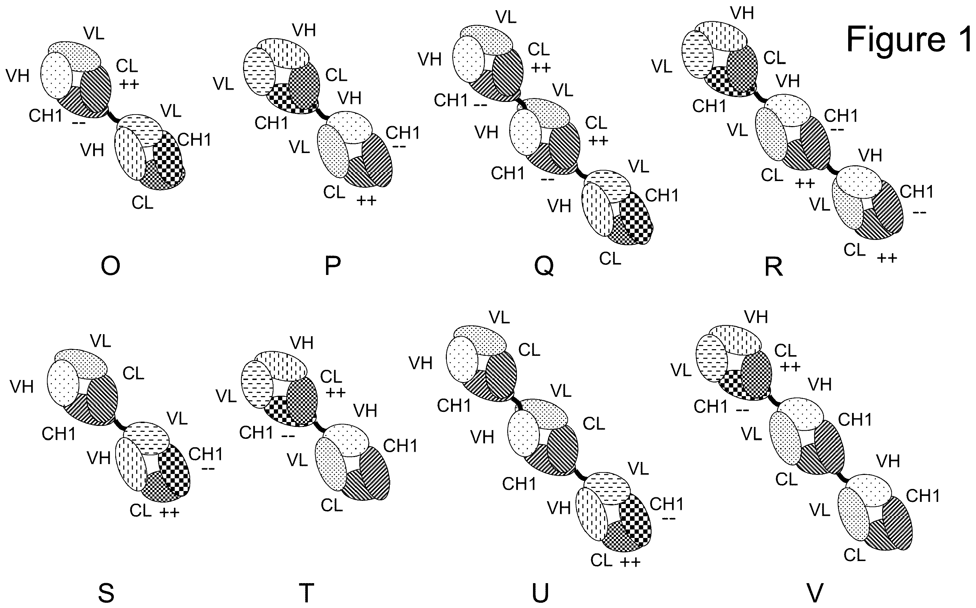

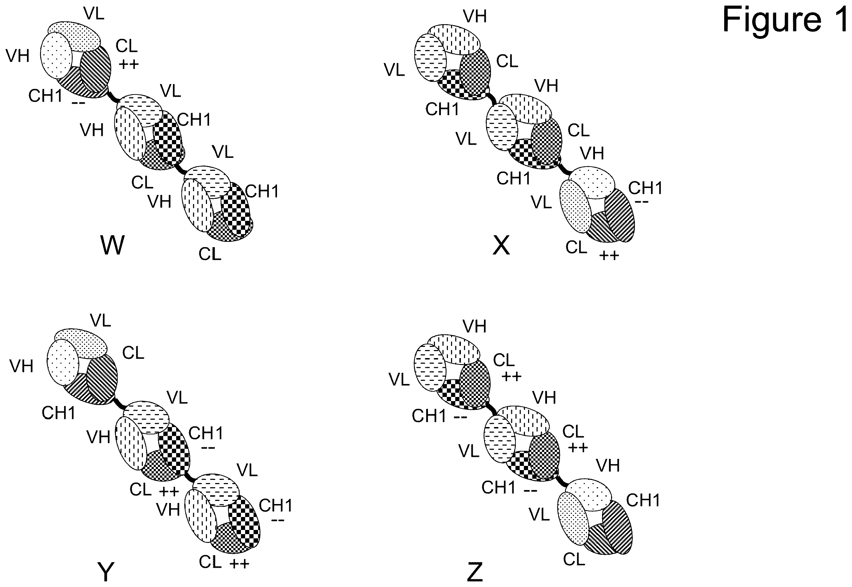

FIGURE 1. Exemplary configurations of the T cell activating bispecific antigen binding molecules (TCBs)disclosed herein. (A, D) Illustration of the "1+1 CrossMab" molecule. (B, E) Illustration of the "2+1 IgG Crossfab" molecule with alternative order of Crossfab and Fab components ("inverted"). (C, F) Illustration of the "2+1 IgG Crossfab" molecule. (G, K) Illustration of the "1+1 IgG Crossfab" molecule with alternative order of Crossfab and Fab components ("inverted"). (H, L) Illustration of the "1+1 IgG Crossfab" molecule. (I, M) Illustration of the "2+1 IgG Crossfab" molecule with two CrossFabs. (J, N) Illustration of the "2+1 IgG Crossfab" molecule with two CrossFabs and alternative order of Crossfab and Fab components ("inverted"). (O, S) Illustration of the "Fab-Crossfab" molecule. (P, T) Illustration of the "Crossfab-Fab" molecule. (Q, U) Illustration of the "(Fab)₂-Crossfab" molecule. (R, V) Illustration of the "Crossfab-(Fab)₂" molecule. (W, Y) Illustration of the "Fab-(Crossfab)₂" molecule. (X, Z) Illustration of the "(Crossfab)₂-Fab" molecule. Black dot: optional modification in the Fc domain promoting heterodimerization. ++, --: amino acids of opposite charges optionally introduced in the CH1 and CL domains. Crossfab molecules are depicted as comprising an exchange of VH and VL regions, but may - in aspects wherein no charge modifications are introduced in CH1 and CL domains - alternatively comprise an exchange of the CH1 and CL domains.

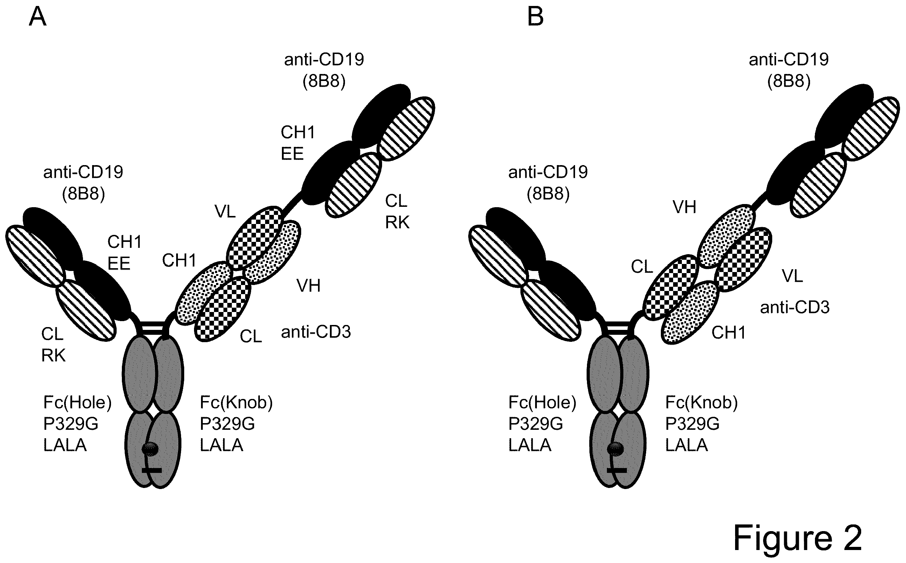

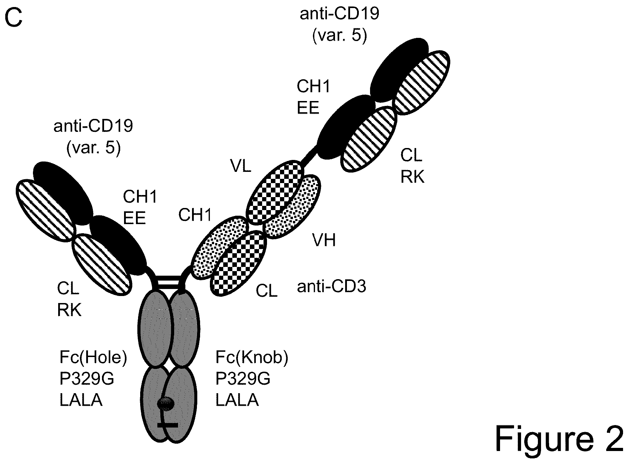

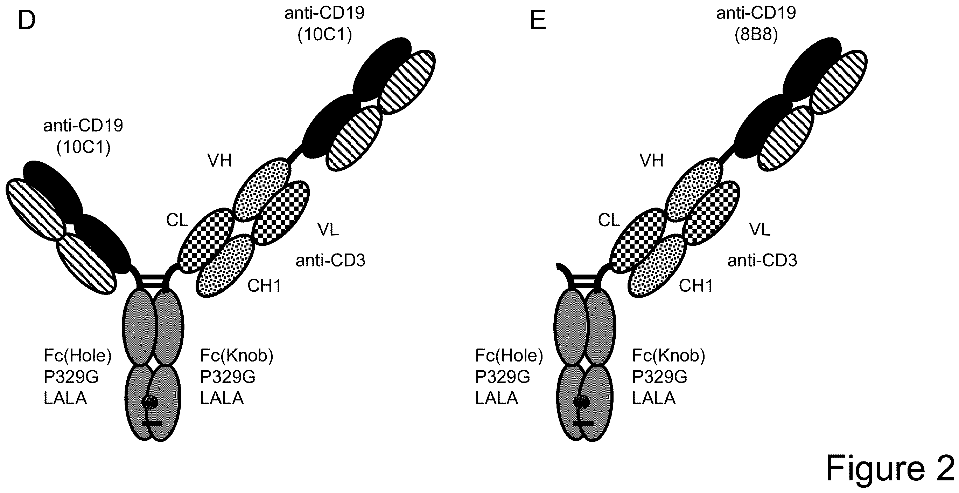

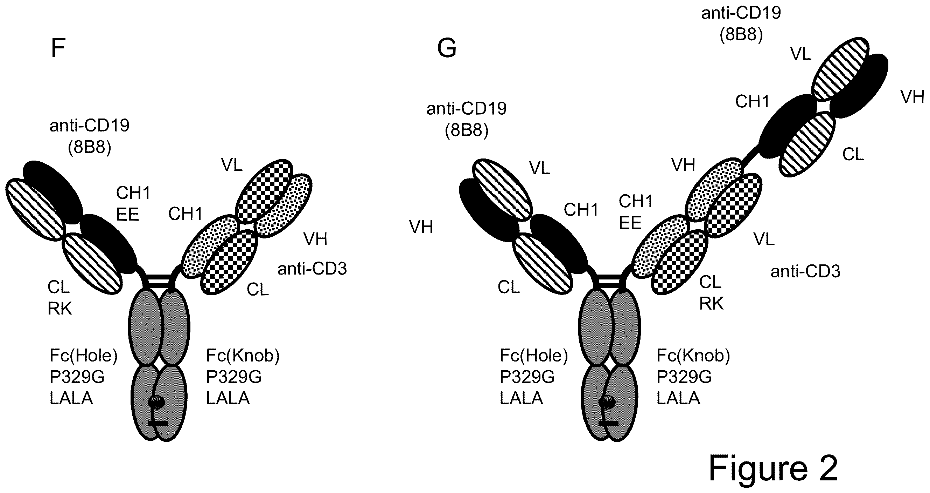

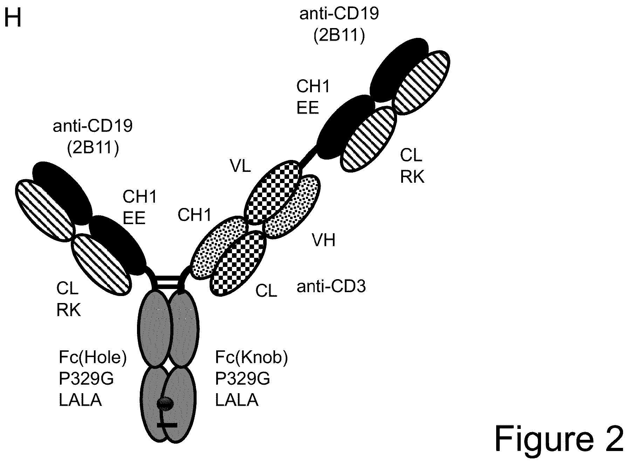

FIGURE 2. Illustration of the TCBs prepared in the Examples. (A) Molecule A: "2+1 IgG CrossFab, inverted" with charge modifications (VH/VL exchange in CD3 binder, charge modification in CD19 binders (parental CD19 binder 8B8), EE = 147E, 213E; RK = 123R, 124K), (B) Molecule B: "2+1 IgG CrossFab, inverted" without charge modifications (CH1/CL exchange in CD3 binder, parental CD19 binder 8B8), (C) Molecule C: "2+1 IgG CrossFab" with charge modifications (VH/VL exchange in CD3 binder, charge modification in CD19 binders (humanized CD19 binder var. 5), (D) Molecule D: "2+1 IgG CrossFab, inverted" without charge modifications (CH1/CL exchange in CD3 binder, CD19 binder 10C1), (E) Molecule E: "1+1 IgG CrossFab, inverted" without charge modifications (CH1/CL exchange in CD3 binder, parental CD19 binder 8B8), (F) Molecule F: "1+1 IgG CrossMab", with charge modifications (VH/VL exchange in CD3 binder, charge modification in CD19 binder (parental CD19 binder 8B8), (G) "2+1 IgG CrossFab, inverted" with charge modifications (VH/VL exchange in CD19 binders (parental CD19 binder 8B8), charge modification in CD3 binder), (H) Molecule H: "2+1 IgG CrossFab" with charge modifications (VH/VL exchange in CD3 binder, charge modification in CD19 binders (humanized CD19 binder 2B11). EE = 147E, 213E; RK = 123R, 124K.

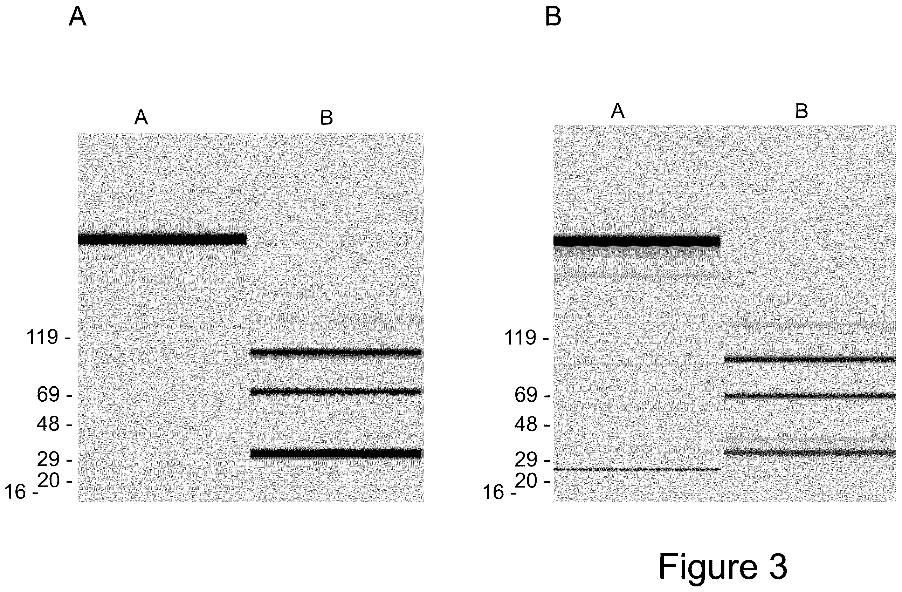

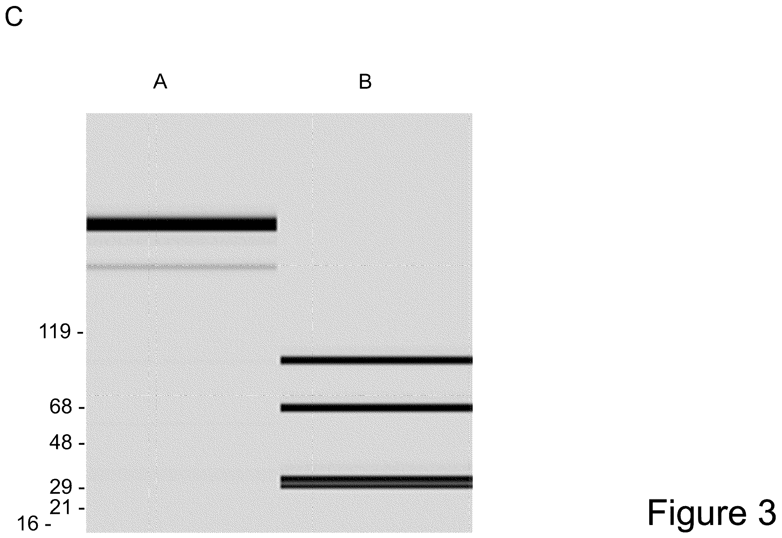

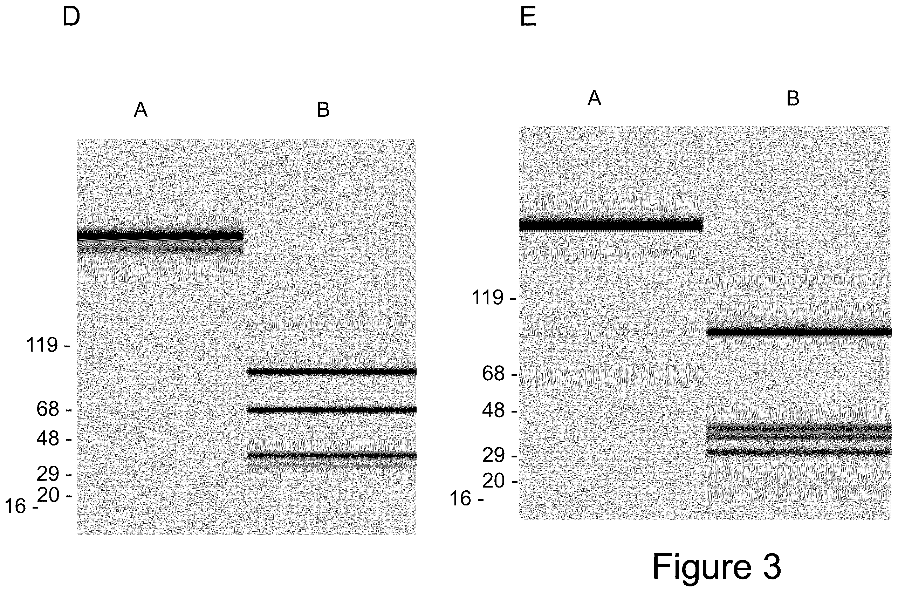

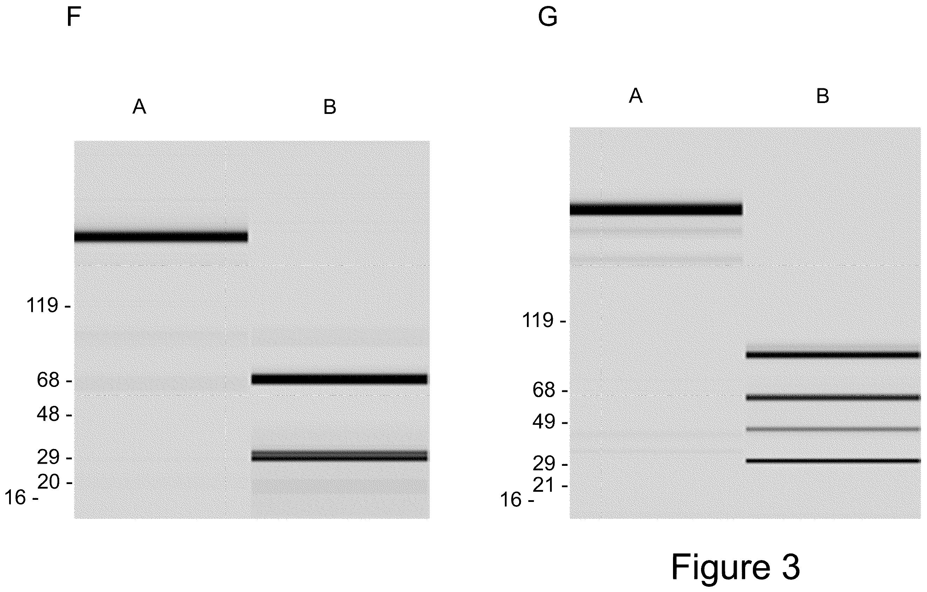

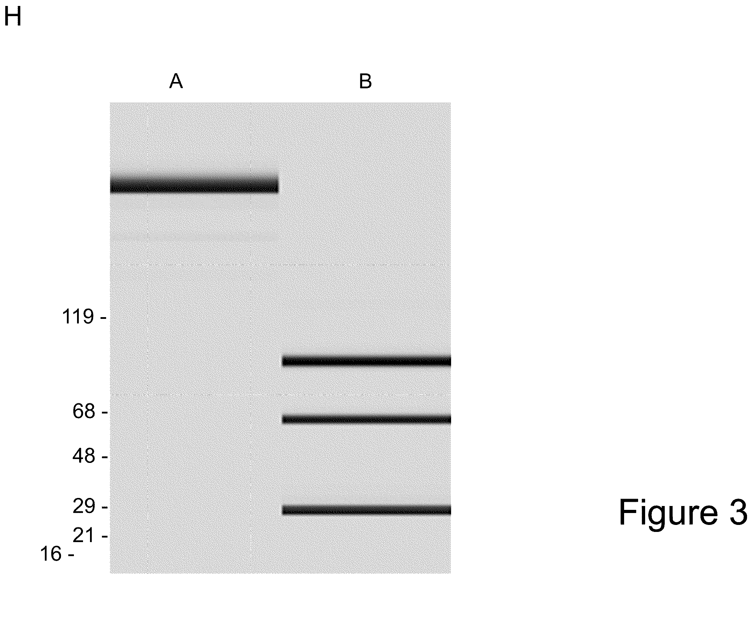

FIGURE 3. CE-SDS analysis of the TCBs prepared in the Examples (final purified preparations). (A) Electropherogram of molecule "A", shown in Figure 2A, (B) electropherogram of molecule "B", shown in Figure 2B, (C) electropherogram of molecule "C", shown in Figure 2C, (D) electropherogram of molecule "D", shown in Figure 2D, (E) electropherogram of molecule "E", shown in Figure 2E, (F) electropherogram of molecule "F", shown in Figure 2F, (G) electropherogram of molecule "G", shown in Figure 2G. Lane A = non-reduced, lane B = reduced, (H) electropherogram of molecule "H", shown in Figure 2H. Lane A = non-reduced, lane B = reduced.

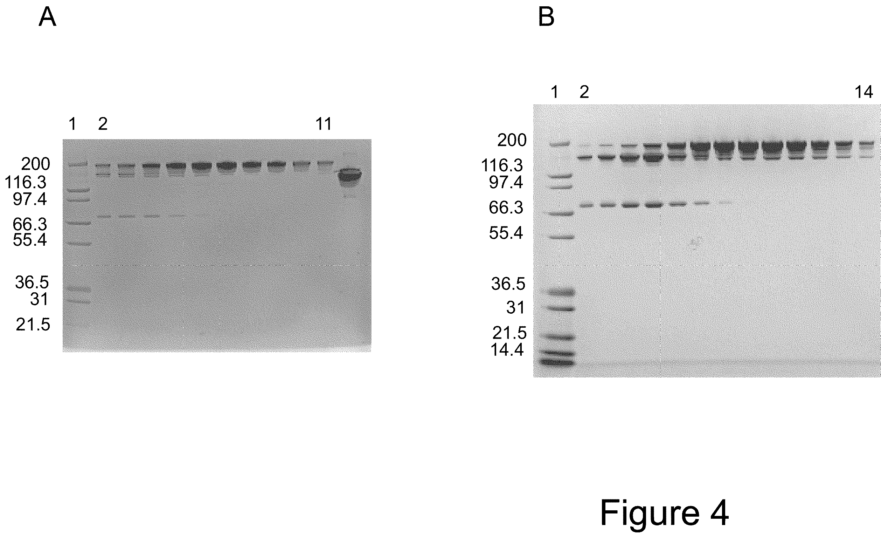

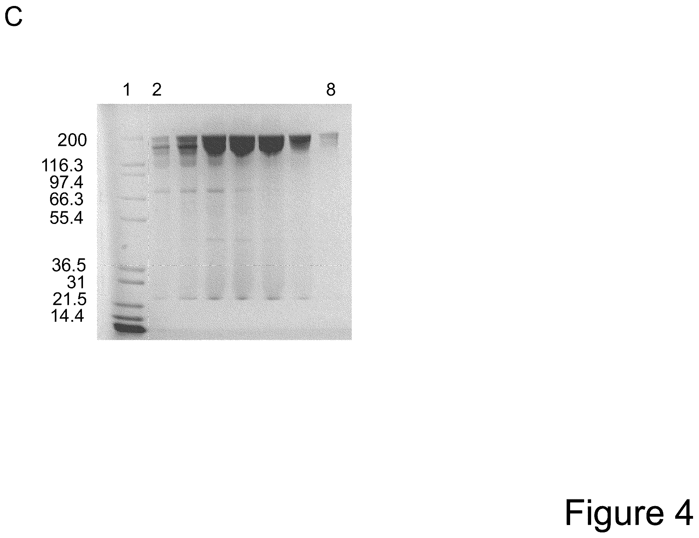

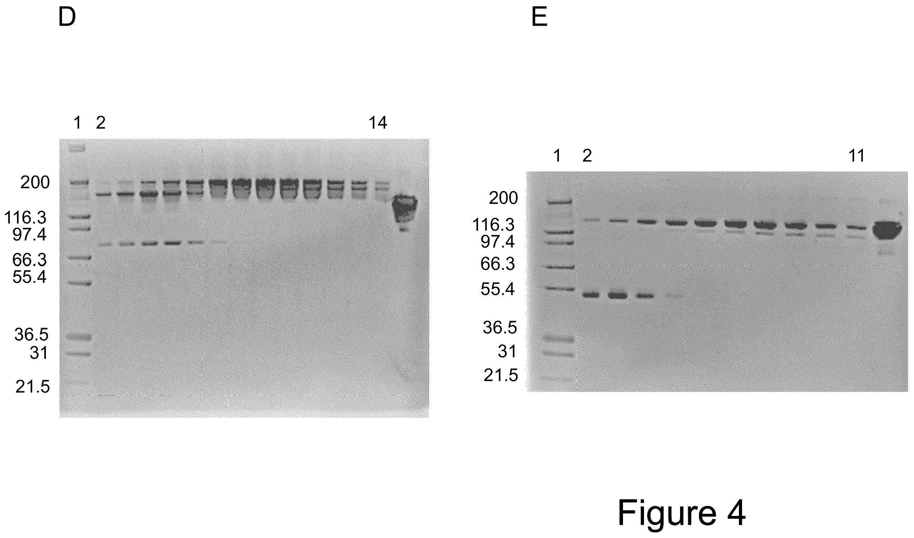

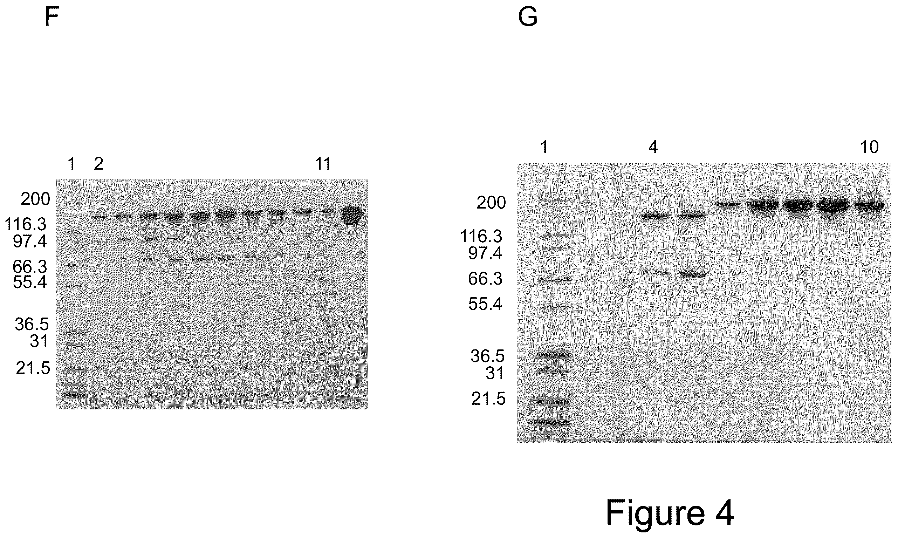

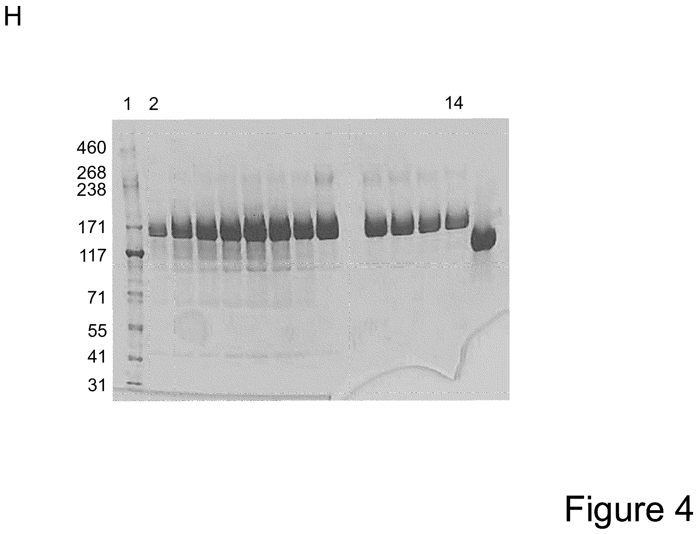

FIGURE 4. SDS-PAGE analysis (4-12% Bis-Tris, Coomassie stained, non reduced) of TCBs prepared in the Examples after the first purification step (Protein A affinity chromatography). (A) lane 1 = marker (Mark 12, unstained standard, Invitrogen); lane 2-11 = fractions from Protein A affinity chromatography of molecule A, (B) lane 1 = marker (Mark 12, unstained standard, Invitrogen); lane 2-14 = fractions from Protein A affinity chromatography of molecule B, (C) lane 1 = marker (Mark 12, unstained standard, Invitrogen); lane 2-8 = fractions from Protein A affinity chromatography of molecule C, (D) lane 1 = marker (Mark 12, unstained standard, Invitrogen); lane 2-14 = fractions from Protein A affinity chromatography of molecule D, (E) lane 1 = marker (Mark 12, unstained standard, Invitrogen); lane 2-11 = fractions from Protein A affinity chromatography of molecule E, (F) lane 1 = marker (Mark 12, unstained standard, Invitrogen); lane 2-11 = fractions from Protein A affinity chromatography of molecule F, (G) lane 1 = marker (Mark 12, unstained standard, Invitrogen); lane 4-10 = fractions from Protein A affinity chromatography of molecule G, (H) lane 1 = marker (HiMark HMW, Invitrogen); lane 2-14 = fractions from Protein A affinity chromatography of molecule H.

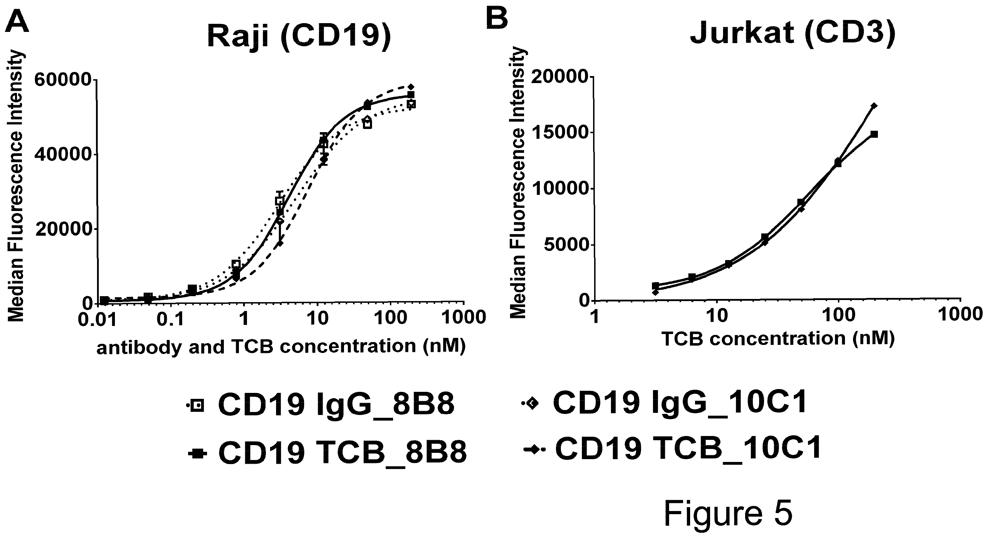

FIGURE 5. (A) Binding of CD19 IgGs and CD19 TCBs to Raji cells. (B) Binding of CD19 TCBs (generated using 8B8 and 10C1 clones) to CD3-expressing Jurkat cells.

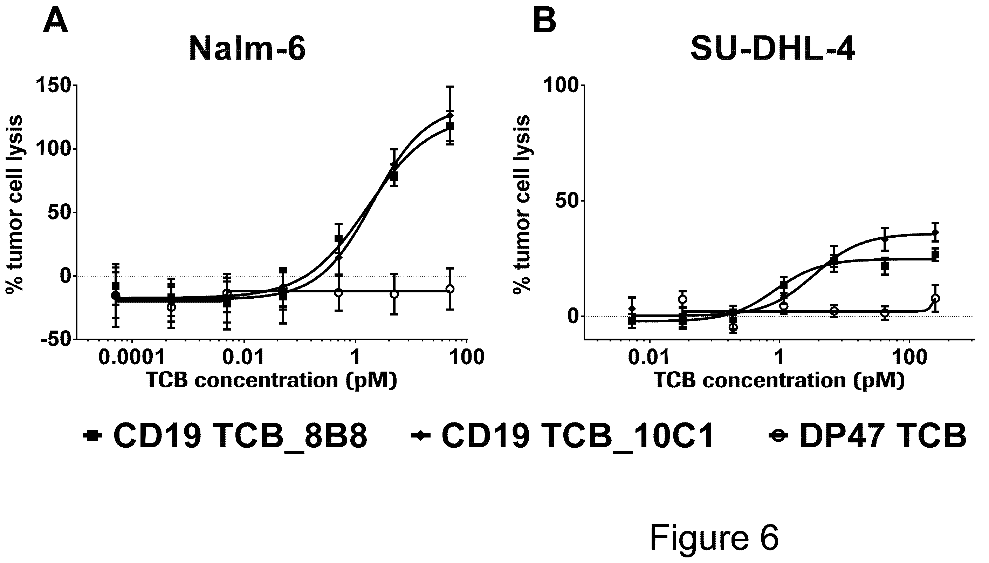

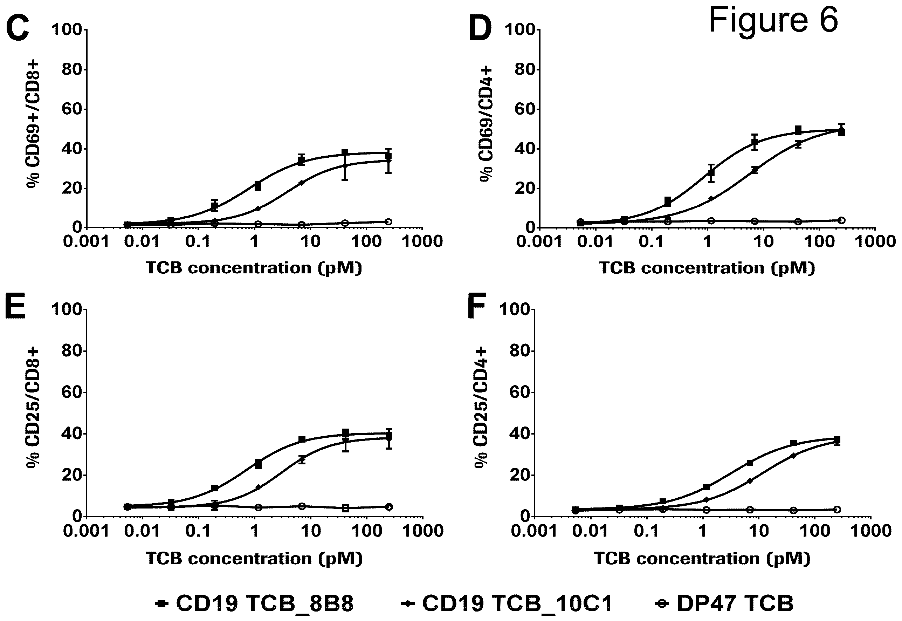

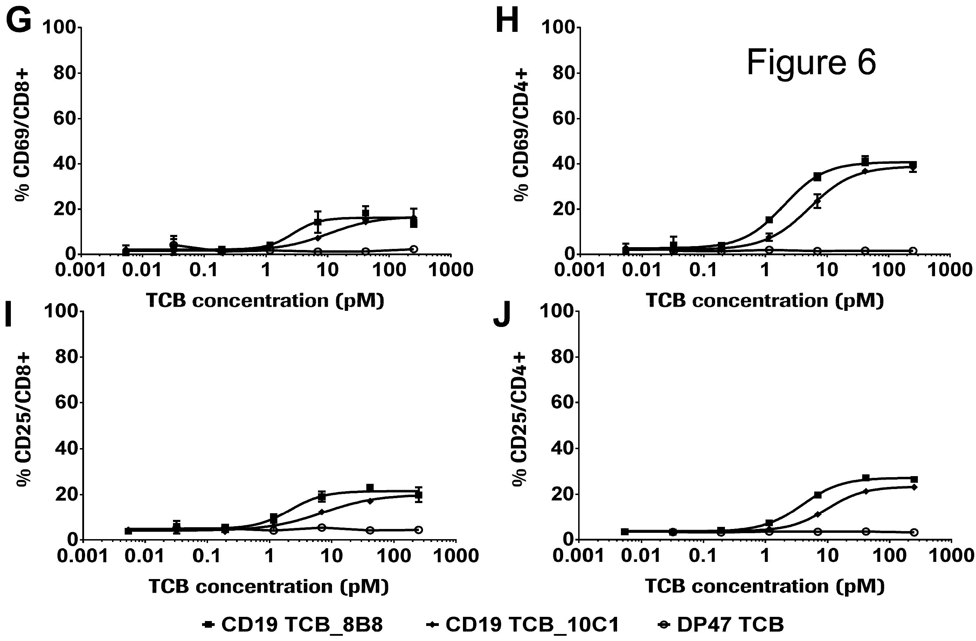

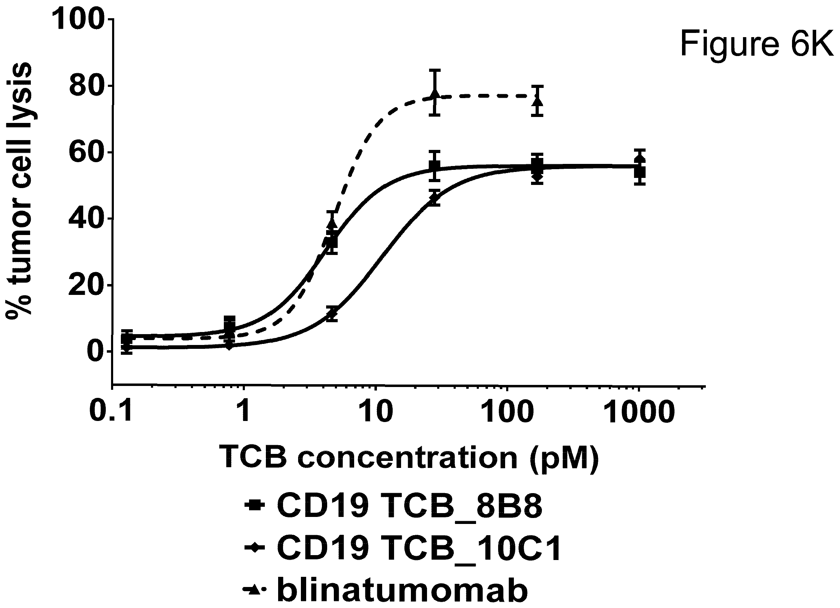

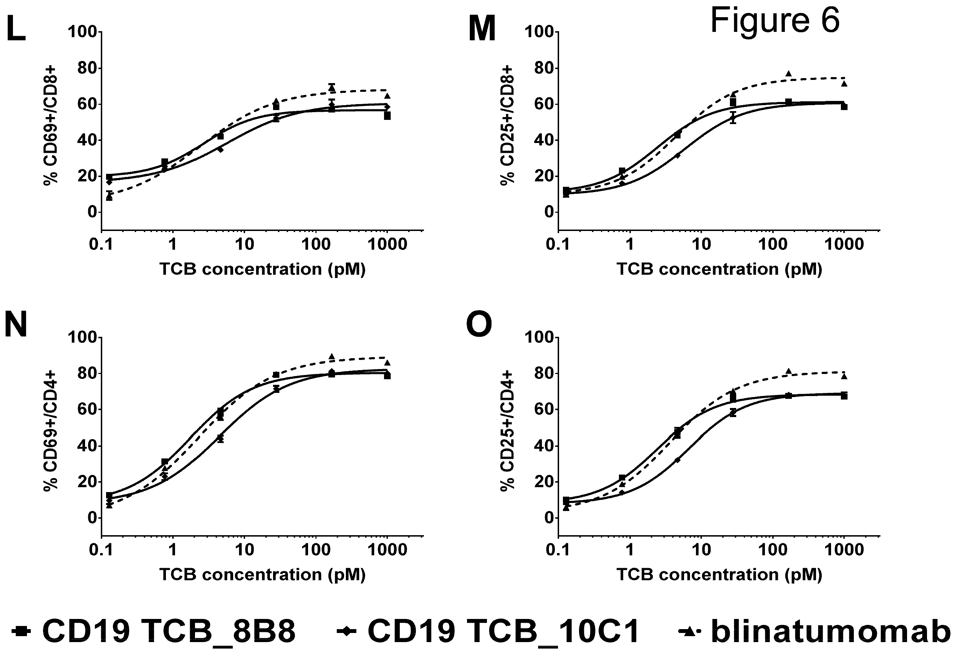

FIGURE 6. (A-B) Lysis of CD19-expressing tumor cells ((A) Nalm-6, (B) SUDHL-4) mediated by CD19 TCB antibodies. (C-F) CD69 (C, D) and CD25 (D, E) expression on CD8 (C, E) and CD4 (D, F) T cells upon killing of Nalm-6 target cells mediated by CD19 TCB antibodies. (G-J) CD69 (G, H) and CD25 (I, J) expression on CD8 (G, I) and CD4 (H, J) T cells upon killing of SUDHL-4 target cells mediated by CD19 TCB antibodies. (K) Lysis of Z-138 cells mediated by CD19 TCB antibodies (having 8B8 and 10C1 clone) and blinatumomab. (L-M) CD69 (L) and CD25 (M) expression on CD8 T cells after Z-138 tumor cell killing mediated by CD19 TCB antibodies. (N-O) CD69 (N) and CD25 (O) expression on CD4 T cells after Z-138 tumor cell killing mediated by CD19 TCB antibodies.

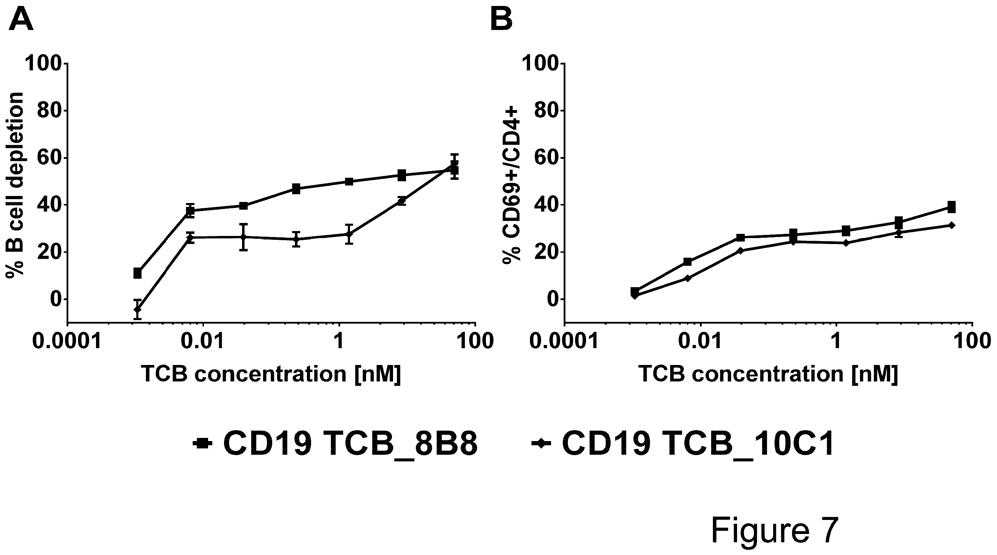

FIGURE 7. (A) B cell depletion mediated by CD19 TCB antibodies based on 8B8 and 10C1 clone. (B) T cell activation (CD69 marker) after B cell depletion mediated by CD19 TCB antibodies based on 8B8 and 10C1 clone.

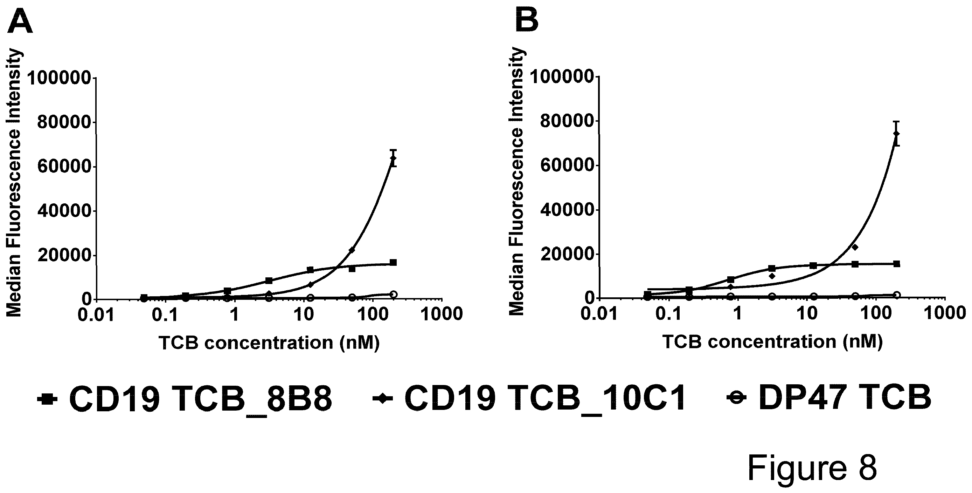

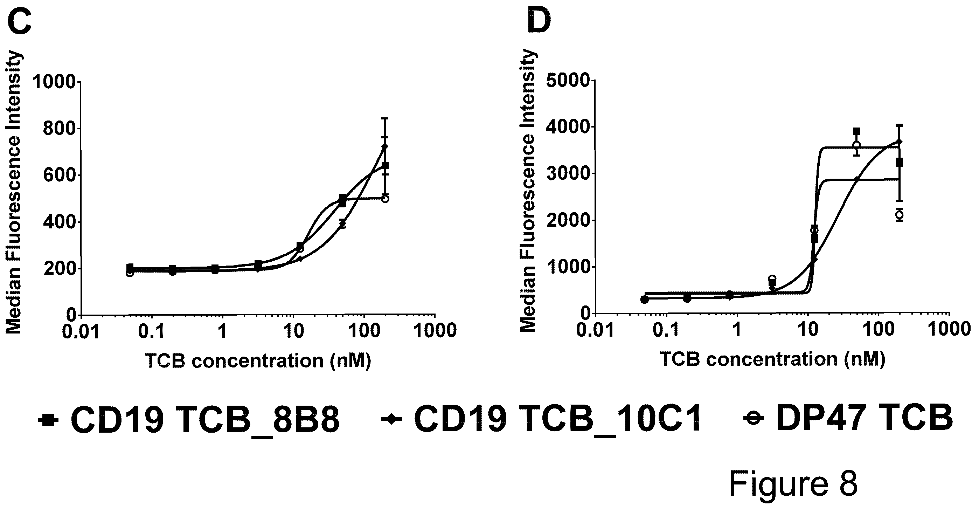

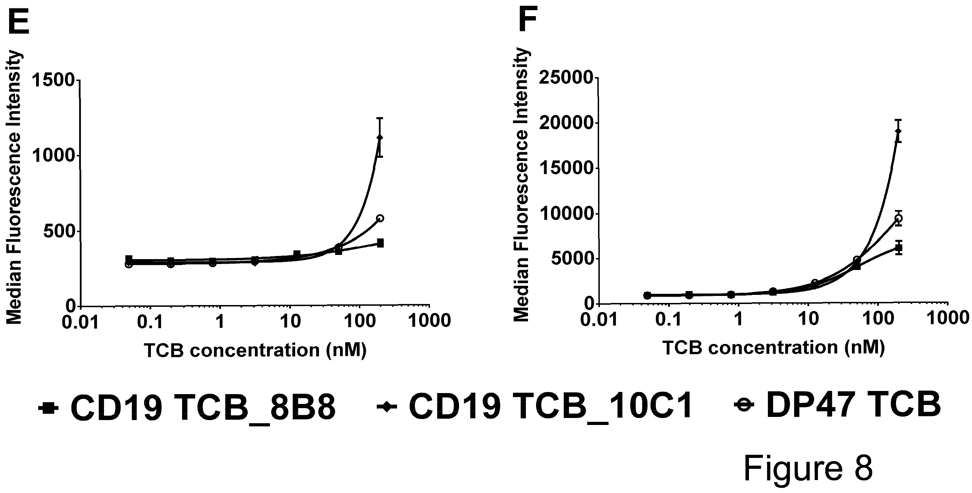

FIGURE 8. Binding of CD19 TCB antibodies (based on 8B8 and 10C1 clone) to cynomolgus monkey (A) and human (B) B cells, to cynomolgus monkey (C) and human (D) CD4⁺T cells, and to cynomolgus monkey (E) and human (F) CD8⁺ T cells.

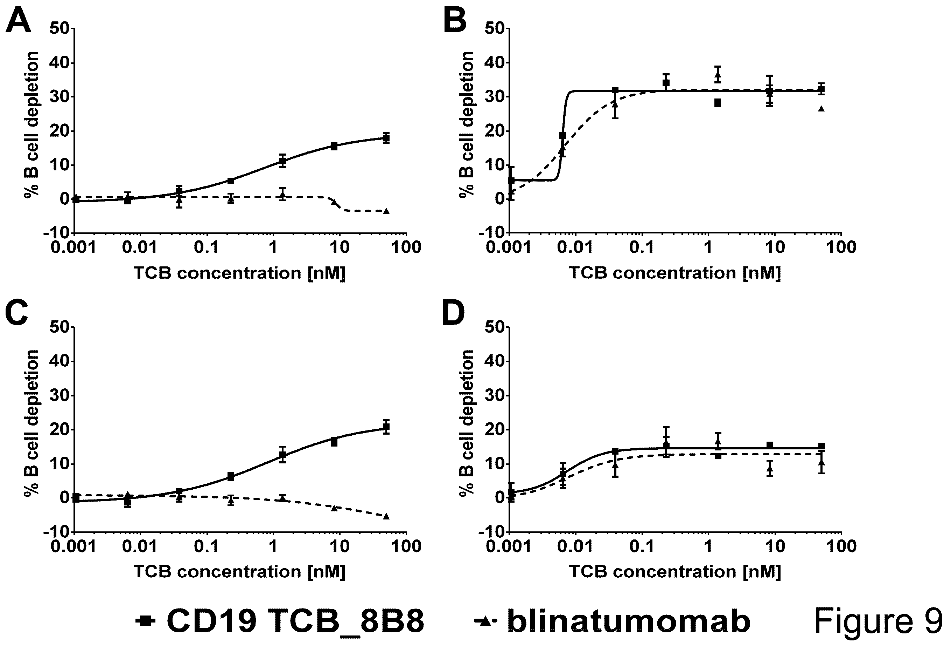

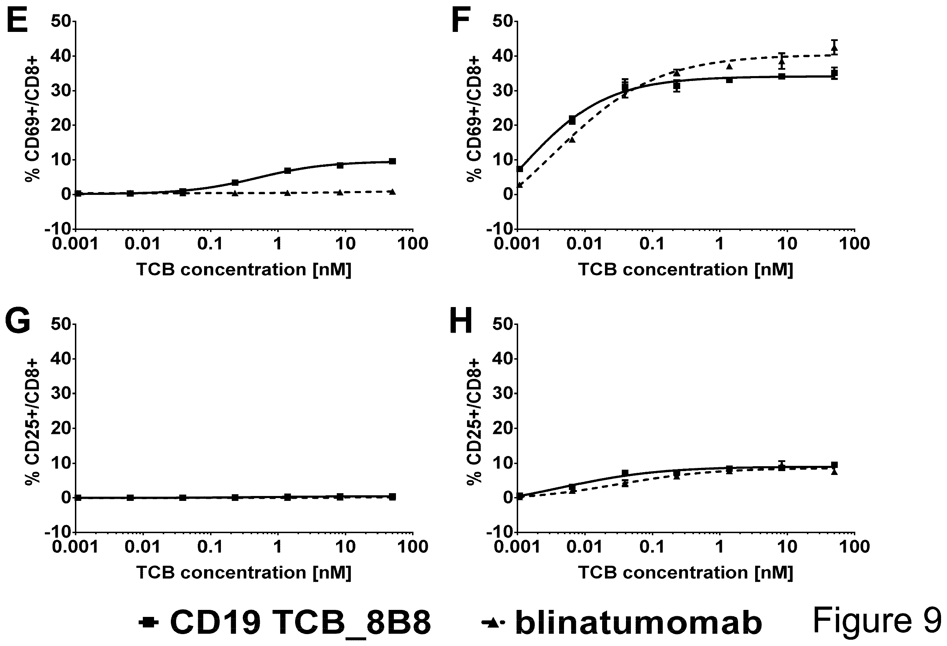

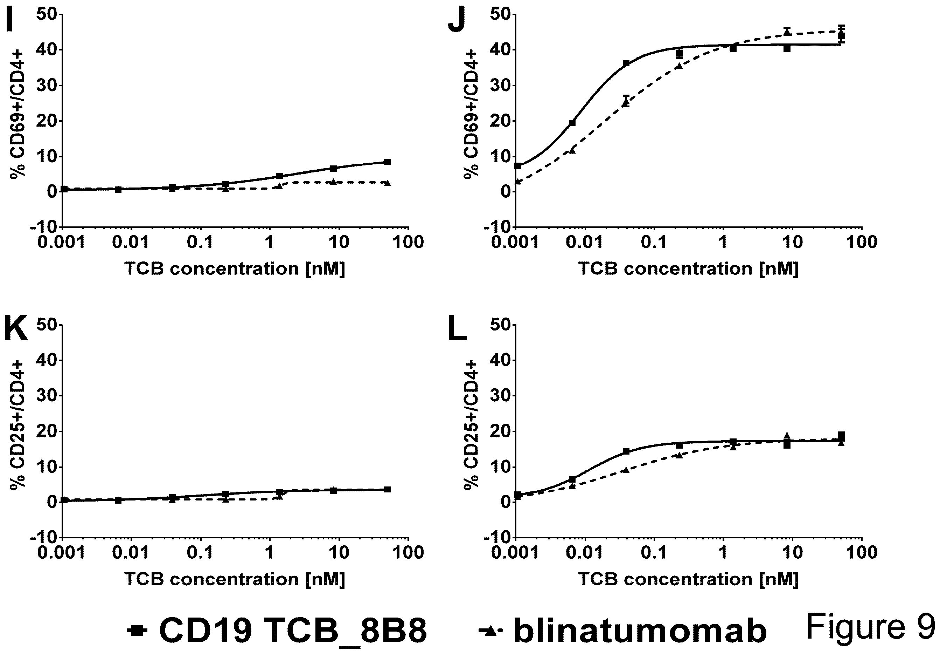

FIGURE 9. (A-D) B cell depletion in cynomolgus monkey (A, C) and human (B, D) whole blood mediated by CD19 TCB antibody (8B8 clone) and blinatumomab: (A, B) % B cell depletion obtained by normalizing the B cell values to CD4 T cells, (C, D) % B cell depletion obtained by normalizing the B cell values to CD8 T cells. (E-L) T cell activation occurring upon CD19 TCB (8B8 clone) and blinatumomab B cell depletion in cynomolgus monkey (E, G, I, K) and human (F, H, J, L) whole blood: (E-F) % CD69 on cynomolgus monkey (E) and human (F) CD8 T cells, (G-H) % CD25 on cynomolgus monkey (G) and human (H) CD8 T cells, (I-J) % CD69 on cynomolgus monkey (I) and human (J) CD4 T cells, (K-L) % CD25 on cynomolgus monkey (K) and human (L) CD4 T cells.

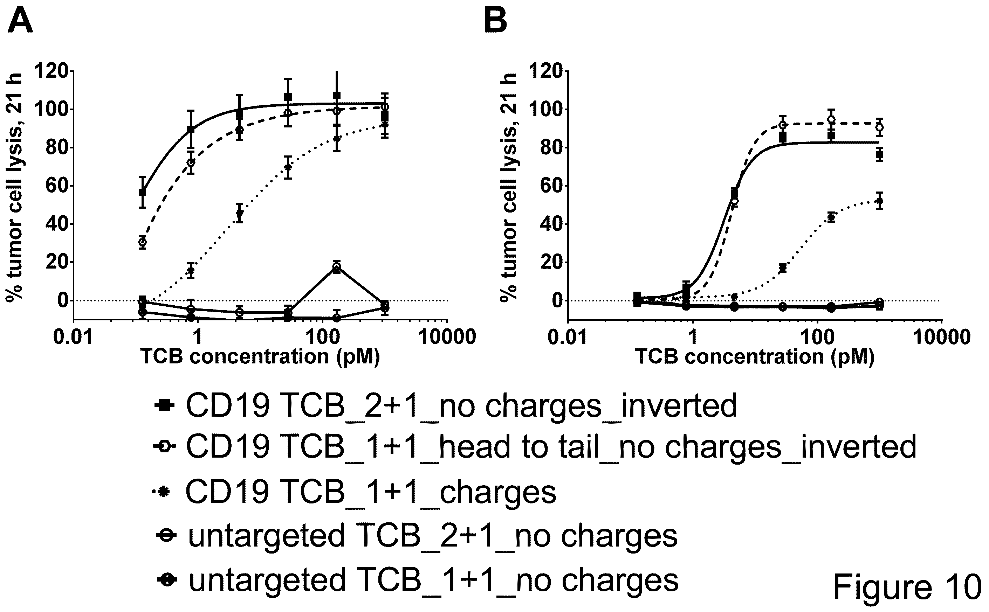

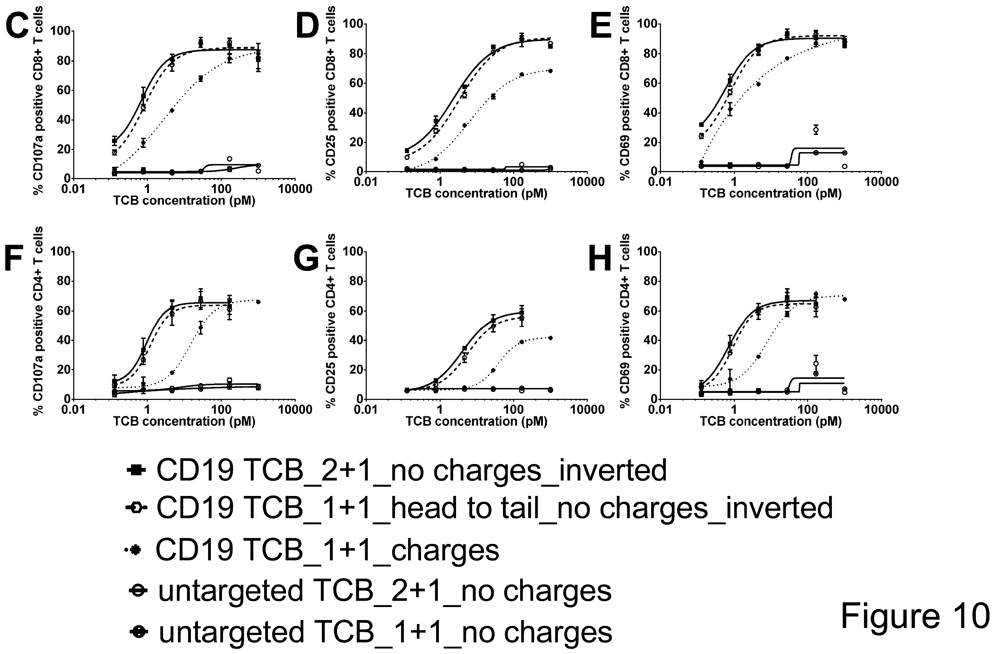

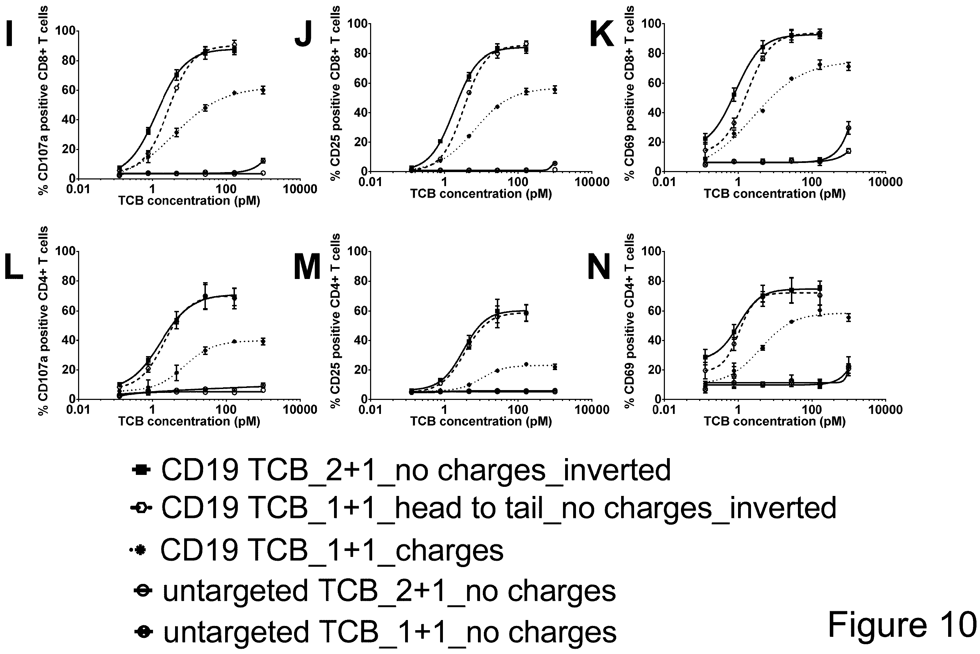

FIGURE 10. Comparison of tumor cell lysis and subsequent T cell activation mediated by different CD19 TCB antibody formats (A) Lysis of Nalm-6 tumor cells. (B) Lysis of Z-138 tumor cells. (C-E) CD107a (C), CD25 (D) and CD69 (E) expression on CD8 T cells upon killing of Nalm-6 targets. (F-H) CD107a (F), CD25 (G) and CD69 (H) expression on CD4 T cells upon killing of Nalm-6 targets. (I-K) CD107a (I), CD25 (J) and CD69 (K) expression on CD8 T cells upon killing of Z-138 targets. (L-N) CD107a (L), CD25 (M) and CD69 (N) expression on CD4 T cells upon killing of Z-138 targets.

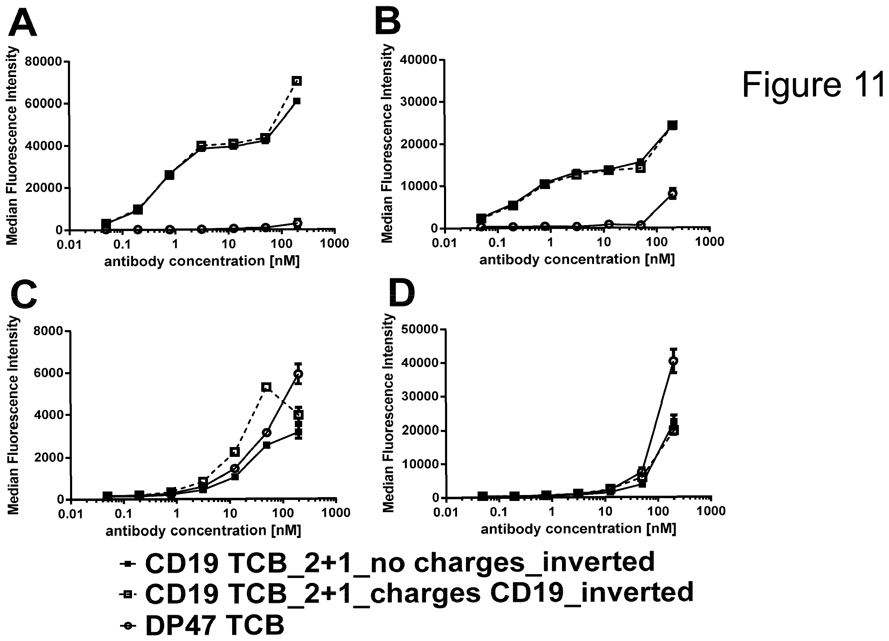

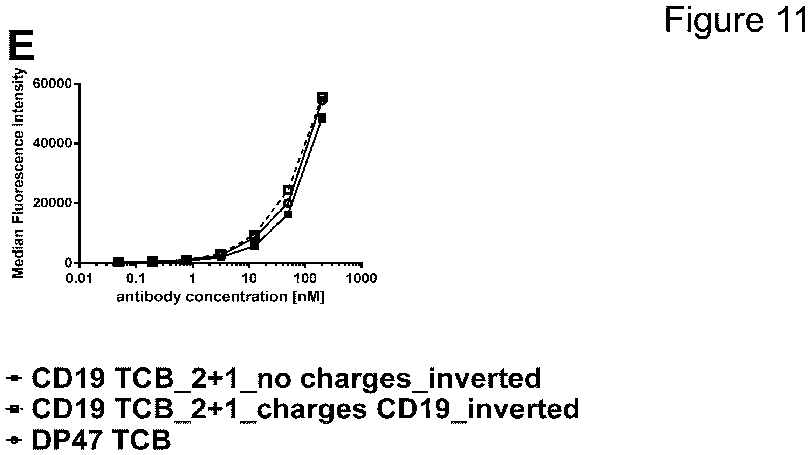

FIGURE 11. Binding of different CD19 TCB antibody formats (based on 8B8 clone) to human CD19- and CD3-expressing target cells. (A) Nalm-6 cells, (B) normal human B cells, (C) normal human CD4 T cells, (D) normal human CD8 T cells, (E) Jurkat cells.

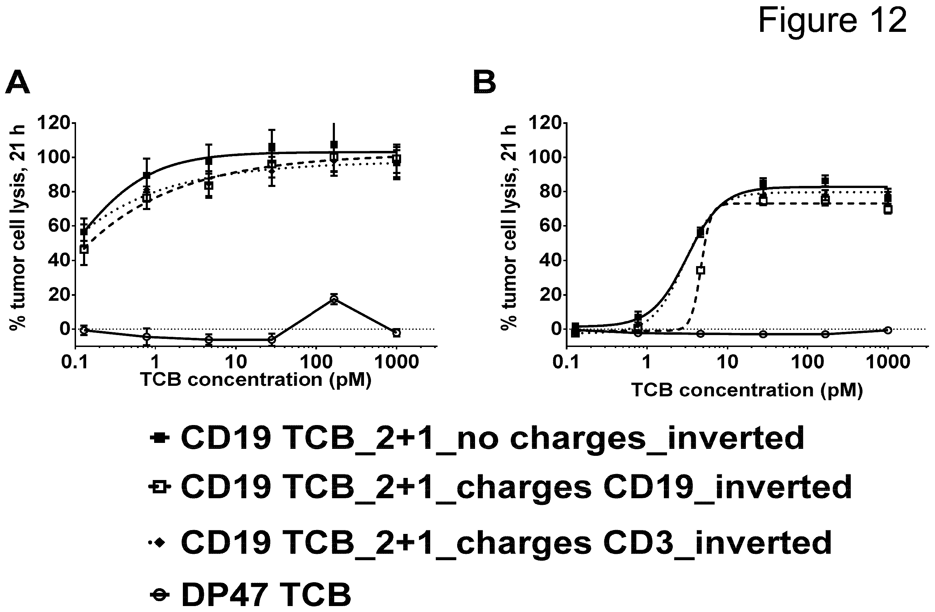

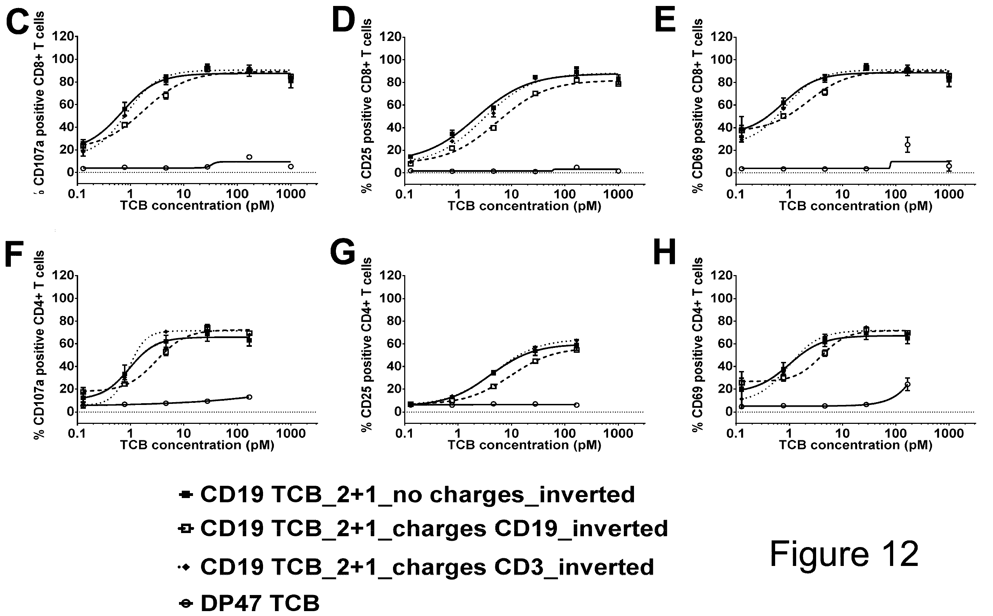

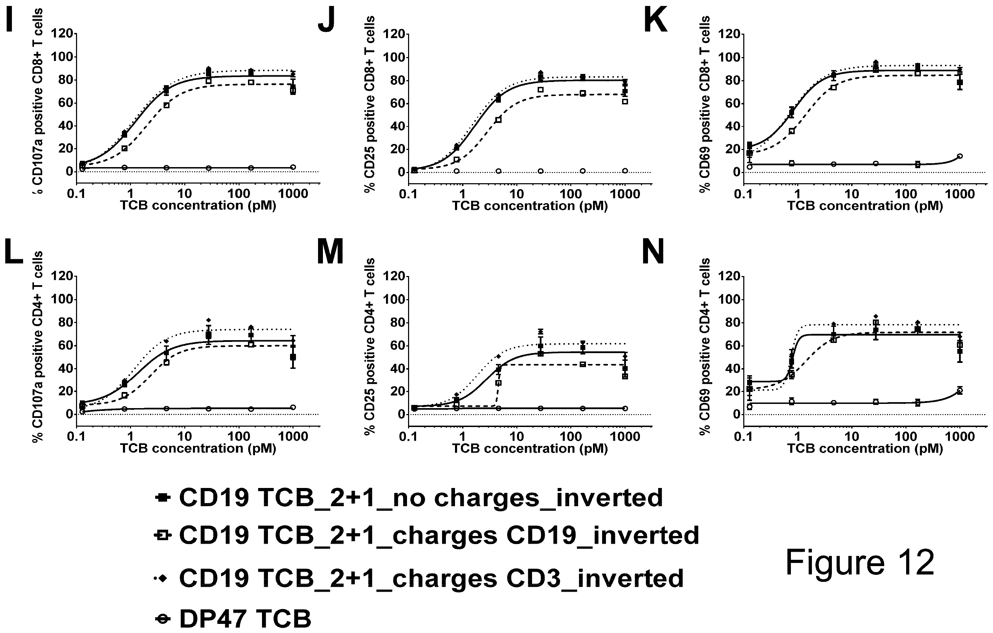

FIGURE 12. Comparison of tumor cell lysis and subsequent T cell activation mediated by different CD19 TCB antibody formats. (A) Lysis of Nalm-6 tumor cells. (B) Lysis of Z-138 tumor cells. (C-E) CD107a (C), CD25 (D) and CD69 (E) expression on CD8 T cells upon killing of Nalm-6 targets. (F-H) CD107a (F), CD25 (G) and CD69 (H) expression on CD4 T cells upon killing of Nalm-6 targets. (I-K) CD107a I), CD25 (J) and CD69 (K) expression on CD8 T cells upon killing of Z-138 targets. (L-N) CD107a (L), CD25 (M) and CD69 (N) expression on CD4 T cells upon killing of Z-138 targets.

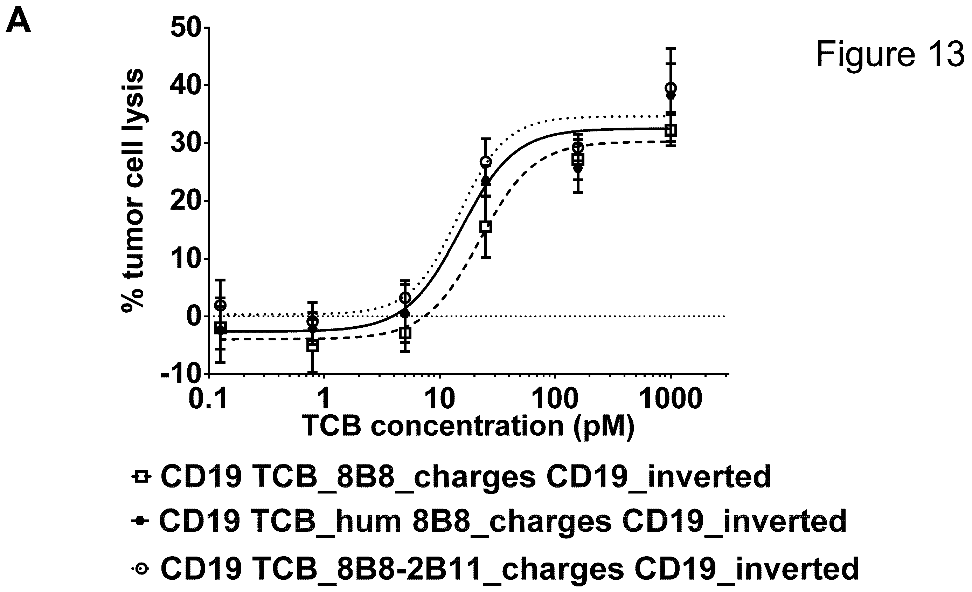

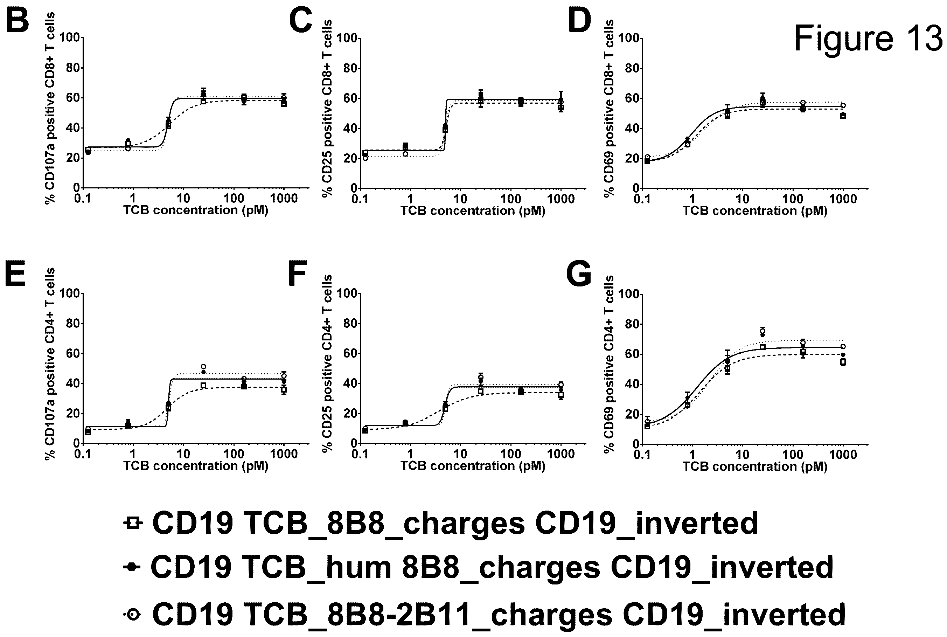

FIGURE 13. Lysis of CD19-expressing tumor cells and subsequent T cell activation mediated by CD19 TCB antibody containing parental and humanized CD19 binders. (A) Lysis of Z-138 tumor cells. (B-D) CD107a (B), CD25 (C) and CD69 (D) expression on CD8 T cells upon killing of Z-138 targets. (E-G) CD107a (E), CD25 (F) and CD69 (G) expression on CD4 T cells upon killing of Z-13 8 targets.

### Detailed Description

### Definitions

Terms are used herein as generally used in the art, unless otherwise defined in the following.

As used herein, the term "antigen binding molecule" refers in its broadest sense to a molecule that specifically binds an antigenic determinant. Examples of antigen binding molecules are immunoglobulins and derivatives, e.g. fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain aspects the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigen binding moiety" refers to a polypeptide molecule that specifically binds to an antigenic determinant. In one aspect, an antigen binding moiety is able to direct the entity to which it is attached (e.g. a second antigen binding moiety) to a target site, for example to a specific type of tumor cell or tumor stroma bearing the antigenic determinant. In another aspect an antigen binding moiety is able to activate signaling through its target antigen, for example a T cell receptor complex antigen. Antigen binding moieties include antibodies and fragments thereof as further defined herein. Particular antigen binding moieties include an antigen binding domain of an antibody, comprising an antibody heavy chain variable region and an antibody light chain variable region. In certain aspects, the antigen binding moieties may comprise antibody constant regions as further defined herein and known in the art. Useful heavy chain constant regions include any of the five isotypes: α, δ, ε, γ, or µ. Useful light chain constant regions include any of the two isotypes: κ and λ.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. CD3) can be any native form the proteins from any vertebrate source, including mammals such as primates (e.g. humans) and rodents (e.g. mice and rats), unless otherwise indicated. In a particular aspect the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. An exemplary human protein useful as antigen is CD3, particularly the epsilon subunit of CD3 (see UniProt no. P07766 (version 130), NCBI RefSeq no. NP_000724.1, SEQ ID NO: 1 for the human sequence; or UniProt no. Q95LI5 (version 49), NCBI GenBank no. BAB71849.1, SEQ ID NO: 2 for the cynomolgus [Macaca fascicularis] sequence), or CD19, also known as B-lymphocyte antigen CD19 or B-lymphocyte surface antigen B4 (see for the human protein UniProt no. P15391, NCBI RefSeq no. NP_001761.3). In certain aspects the T cell activating bispecific antigen binding molecule disclosed herein binds to an epitope of CD3 or CD19 that is conserved among the CD3 or CD19 antigens from different species.

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one aspect, the extent of binding of an antigen binding moiety to an unrelated protein is less than about 10% of the binding of the antigen binding moiety to the antigen as measured, e.g., by SPR. In certain aspects, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

An "activating T cell antigen" as used herein refers to an antigenic determinant expressed on the surface of a T lymphocyte, particularly a cytotoxic T lymphocyte, which is capable of inducing T cell activation upon interaction with an antigen binding molecule. Specifically, interaction of an antigen binding molecule with an activating T cell antigen may induce T cell activation by triggering the signaling cascade of the T cell receptor complex. In a particular aspect the activating T cell antigen is CD3, particularly the epsilon subunit of CD3 (see UniProt no. P07766 (version 130), NCBI RefSeq no. NP_000724.1, SEQ ID NO: 1 for the human sequence; or UniProt no. Q95LI5 (version 49), NCBI GenBank no. BAB71849.1, SEQ ID NO: 2 for the cynomolgus [Macaca fascicularis] sequence).

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The T cell activating bispecific antigen binding molecules of the disclosed herein are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma. In a particular aspect, the target cell antigen is CD19, particularly human CD19.

As used herein, the terms "first", "second" or "third" with respect to Fab molecules etc., are used for convenience of distinguishing when there is more than one of each type of moiety. Use of these terms is not intended to confer a specific order or orientation of the T cell activating bispecific antigen binding molecule unless explicitly so stated.

A "Fab molecule" refers to a protein consisting of the VH and CH1 domain of the heavy chain (the "Fab heavy chain") and the VL and CL domain of the light chain (the "Fab light chain") of an immunoglobulin.

By "fused" is meant that the components (e.g. a Fab molecule and an Fc domain subunit) are linked by peptide bonds, either directly or via one or more peptide linkers.

As used herein, the term "single-chain" refers to a molecule comprising amino acid monomers linearly linked by peptide bonds. In certain aspects, one of the antigen binding moieties is a single-chain Fab molecule, i.e. a Fab molecule wherein the Fab light chain and the Fab heavy chain are connected by a peptide linker to form a single peptide chain. In a particular such aspect, the C-terminus of the Fab light chain is connected to the N-terminus of the Fab heavy chain in the single-chain Fab molecule.

By a "crossover" Fab molecule (also termed "Crossfab") is meant a Fab molecule wherein the variable domains or the constant domains of the Fab heavy and light chain are exchanged (i.e. replaced by each other), i.e. the crossover Fab molecule comprises a peptide chain composed of the light chain variable domain VL and the heavy chain constant domain 1 CH1 (VL-CH1, in N-to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e. comprising a heavy chain composed of the heavy chain variable and constant domains (VH-CH1, in N- to C-terminal direction), and a light chain composed of the light chain variable and constant domains (VL-CL, in N- to C-terminal direction).

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable domain (VH), also called a variable heavy domain or a heavy chain variable region, followed by three constant domains (CH1, CH2, and CH3), also called a heavy chain constant region. Similarly, from N- to C-terminus, each light chain has a variable domain (VL), also called a variable light domain or a light chain variable region, followed by a constant light (CL) domain, also called a light chain constant region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain aspects, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable domain (VL) and an antibody heavy chain variable domain (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the complementarity determining regions (CDRs), the latter being of highest sequence variability and/or involved in antigen recognition. With the exception of CDR1 in VH, CDRs generally comprise the amino acid residues that form the hypervariable loops. Hypervariable regions (HVRs) are also referred to as "complementarity determining regions" (CDRs), and these terms are used herein interchangeably in reference to portions of the variable region that form the antigen binding regions. This particular region has been described by Kabat et al., Sequences of Proteins of Immunological Interest, 5th Ed. Public Health Service, National Institutes of Health, Bethesda, MD (1991) and by Chothia et al., J Mol Biol 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody. The CDR sequences given herein are generally according to the Kabat definition.

**TABLE A. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹Numbering of all CDR definitions in Table A is according to the numbering conventions set forth by Kabat et al. (see below). ² "AbM" with a lowercase "b" as used in Table A refers to the CDRs as defined by Oxford Molecular's "AbM" antibody modeling software. | | | |

Kabat et al. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein in connection with variable region seqeunces, "Kabat numbering" refers to the numbering system set forth by Kabat et al., *Sequences of Proteins of Immunological Interest,* 5th Ed. Public Health Service, National Institutes of Health, Bethesda, MD (1991). Unless otherwise specified, references to the numbering of specific amino acid residue positions in an antibody variable region are according to the Kabat numbering system. As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" or "Kabat numbering" herein. Specifically the Kabat numbering system (see pages 647-660 of Kabat, et al., Sequences of Proteins of Immunological Interest, 5th ed., Public Health Service, National Institutes of Health, Bethesda, MD (1991)) is used for the light chain constant domain CL of kappa and lambda isotype and the Kabat EU index numbering system (see pages 661-723) is used for the heavy chain constant domains (CH1, Hinge, CH2 and CH3), which is herein further clarified by referring to "numbering according to Kabat EU index" in this case.

The polypeptide sequences of the sequence listing are not numbered according to the Kabat numbering system. However, it is well within the ordinary skill of one in the art to convert the numbering of the sequences of the Sequence Listing to Kabat numbering.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

A "humanized" antibody refers to a chimeric antibody comprising amino acid residues from non-human HVRs and amino acid residues from human FRs. In certain aspects, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. Such variable domains are referred to herein as "humanized variable region". A humanized antibody optionally may comprise at least a portion of an antibody constant region derived from a human antibody. A "humanized form" of an antibody, e.g., a non-human antibody, refers to an antibody that has undergone humanization. Other forms of "humanized antibodies" encompassed by the present disclosure are those in which the constant region has been additionally modified or changed from that of the original antibody to generate the properties according to the disclosure, especially in regard to C1q binding and/or Fc receptor (FcR) binding.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (K447), of the Fc region may or may not be present. Amino acid sequences of heavy chains including Fc domains (or a subunit of an Fc domain as defined herein) are denoted herein without C-terminal glycine-lysine dipeptide if not indicated otherwise. In one aspect, a heavy chain including a subunit of an Fc domain as specified herein, comprised in a T cell activating bispecific antigen binding molecule disclosed herein, comprises an additional C-terminal glycine-lysine dipeptide (G446 and K447, numbering according to EU index of Kabat). In one aspect, a heavy chain including a subunit of an Fc domain as specified herein, comprised in a T cell activating bispecific antigen binding moleculeas disclosed herein, comprises an additional C-terminal glycine residue (G446, numbering according to EU index of Kabat). Compositions disclosed herein, such as the pharmaceutical compositions described herein, comprise a population of T cell activating bispecific antigen binding molecules. The population of T cell activating bispecific antigen binding molecule may comprise molecules having a full-length heavy chain and molecules having a cleaved variant heavy chain. The population of T cell activating bispecific antigen binding molecules may consist of a mixture of molecules having a full-length heavy chain and molecules having a cleaved variant heavy chain, wherein at least 50%, at least 60%, at least 70%, at least 80% or at least 90% of the T cell activating bispecific antigen binding molecules have a cleaved variant heavy chain. In one aspect, a composition comprising a population of T cell activating bispecific antigen binding molecules comprises an T cell activating bispecific antigen binding molecule comprising a heavy chain including a subunit of an Fc domain as specified herein with an additional C-terminal glycine-lysine dipeptide (G446 and K447, numbering according to EU index of Kabat). In one aspect, a composition comprising a population of T cell activating bispecific antigen binding molecules comprises an T cell activating bispecific antigen binding molecule comprising a heavy chain including a subunit of an Fc domain as specified herein with an additional C-terminal glycine residue (G446, numbering according to EU index of Kabat). In one aspect, such a composition comprises a population of T cell activating bispecific antigen binding molecules comprised of molecules comprising a heavy chain including a subunit of an Fc domain as specified herein; molecules comprising a heavy chain including a subunit of a Fc domain as specified herein with an additional C-terminal glycine residue (G446, numbering according to EU index of Kabat); and molecules comprising a heavy chain including a subunit of an Fc domain as specified herein with an additional C-terminal glycine-lysine dipeptide (G446 and K447, numbering according to EU index of Kabat). Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some aspects the modification promoting association comprises an amino acid mutation in the Fc domain, specifically an amino acid substitution. In a particular aspect, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G₃₂₉, P329G, or Pro329Gly.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the present disclosure, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: $100 times the fraction X / Y$ where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present disclosure. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts, as well as positive and negative strand forms, and double-stranded forms. Isolated polynucleotides or nucleic acids according to the present disclosure further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present disclosure, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present disclosure can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain aspects, the expression cassette comprises polynucleotide sequences that encode bispecific antigen binding molecules as disclosed herein or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector disclosed herein comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one aspect, the expression vector disclosed herein comprises an expression cassette that comprises polynucleotide sequences that encode bispecific antigen binding molecules disclosed herein or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding moleculesdisclosed herein. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcγRI (CD64), FcγRIIa (CD32), and FcαRI (CD89).

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced ADCC" is defined as either a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g. PCT publication no. WO 2006/082515 or PCT publication no. WO 2012/130831).

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some aspects, T cell activating bispecific antigen binding molecules disclosed herein are used to delay development of a disease or to slow the progression of a disease.

The term "package insert" is used to refer to instructions customarily included in commercial packages of therapeutic products, that contain information about the indications, usage, dosage, administration, combination therapy, contraindications and/or warnings concerning the use of such therapeutic products.

### Detailed Description of the Aspects

The invention provides a T cell activating bispecific antigen binding molecule with favorable properties for therapeutic application, in particular with respect to efficacy and safety as well as produceability (e.g. with respect to purity, yield, stability).

The inventors have discovered that T cell activating bispecific antigen binding molecules comprising an antigen binding moiety with the binding specificity of the anti-CD19 antibody 8B8 (WO 2011/147834) provide high potency in mediating killing of CD19-expressing cells by T cells. Moreover, T cell activating bispecific antigen binding molecules comprising a particular humanized version of antibody 8B8 were found to exhibit high potency and good produceability.

### Charge modifications

The T cell activating bispecific antigen binding molecules disclosed herein may comprise amino acid substitutions in Fab molecules comprised therein which are particularly efficient in reducing mispairing of light chains with non-matching heavy chains (Bence-Jones-type side products), which can occur in the production of Fab-based bi-/multispecific antigen binding molecules with a VH/VL exchange in one (or more, in case of molecules comprising more than two antigen-binding Fab molecules) of their binding arms (see also PCT publication no. WO 2015/150447, particularly the examples therein.

Accordingly, in particular aspects, the T cell activating bispecific antigen binding molecule disclosed herein comprises
(a) a first Fab molecule which specifically binds to a first antigen
(b) a second Fab molecule which specifically binds to a second antigen, and wherein the variable domains VL and VH of the Fab light chain and the Fab heavy chain are replaced by each other, wherein the first antigen is an activating T cell antigen and the second antigen is CD19, or the first antigen is CD19 and the second antigen is an activating T cell antigen; and
wherein
i) in the constant domain CL of the first Fab molecule under a) the amino acid at position 124 is substituted by a positively charged amino acid (numbering according to Kabat), and wherein in the constant domain CH1 of the first Fab molecule under a) the amino acid at position 147 or the amino acid at position 213 is substituted by a negatively charged amino acid (numbering according to Kabat EU index); or
ii) in the constant domain CL of the second Fab molecule under b) the amino acid at position 124 is substituted by a positively charged amino acid (numbering according to Kabat), and wherein in the constant domain CH1 of the second Fab molecule under b) the amino acid at position 147 or the amino acid at position 213 is substituted by a negatively charged amino acid (numbering according to Kabat EU index).

The T cell activating bispecific antigen binding molecule does not comprise both modifications mentioned under i) and ii). The constant domains CL and CH1 of the second Fab molecule are not replaced by each other (i.e. remain unexchanged).

In one aspect of the T cell activating bispecific antigen binding molecule according to the present disclosure, in the constant domain CL of the first Fab molecule under a) the amino acid at position 124 is substituted independently by lysine (K), arginine (R) or histidine (H) (numbering according to Kabat) (in one preferred aspect independently by lysine (K) or arginine (R)), and in the constant domain CH1 of the first Fab molecule under a) the amino acid at position 147 or the amino acid at position 213 is substituted independently by glutamic acid (E), or aspartic acid (D) (numbering according to Kabat EU index).

In a further aspect, in the constant domain CL of the first Fab molecule under a) the amino acid at position 124 is substituted independently by lysine (K), arginine (R) or histidine (H) (numbering according to Kabat), and in the constant domain CH1 of the first Fab molecule under a) the amino acid at position 147 is substituted independently by glutamic acid (E), or aspartic acid (D) (numbering according to Kabat EU index).

In a particular aspect, in the constant domain CL of the first Fab molecule under a) the amino acid at position 124 is substituted independently by lysine (K), arginine (R) or histidine (H) (numbering according to Kabat) (in one preferred aspect independently by lysine (K) or arginine (R)) and the amino acid at position 123 is substituted independently by lysine (K), arginine (R) or histidine (H) (numbering according to Kabat) (in one preferred aspect independently by lysine (K) or arginine (R)), and in the constant domain CH1 of the first Fab molecule under a) the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In a more particular aspect, in the constant domain CL of the first Fab molecule under a) the amino acid at position 124 is substituted by lysine (K) (numbering according to Kabat) and the amino acid at position 123 is substituted by lysine (K) or arginine (R) (numbering according to Kabat), and in the constant domain CH1 of the first Fab molecule under a) the amino acid at position 147 is substituted by glutamic acid (E) (numbering according to Kabat EU index) and the amino acid at position 213 is substituted by glutamic acid (E) (numbering according to Kabat EU index).

In an even more particular aspect, in the constant domain CL of the first Fab molecule under a) the amino acid at position 124 is substituted by lysine (K) (numbering according to Kabat) and the amino acid at position 123 is substituted by arginine (R) (numbering according to Kabat), and in the constant domain CH1 of the first Fab molecule under a) the amino acid at position 147 is substituted by glutamic acid (E) (numbering according to Kabat EU index) and the amino acid at position 213 is substituted by glutamic acid (E) (numbering according to Kabat EU index).

In particular aspects, the constant domain CL of the first Fab molecule under a) is of kappa isotype.

Alternatively, the amino acid substitutions according to the above aspects may be made in the constant domain CL and the constant domain CH1 of the second Fab molecule under b) instead of in the constant domain CL and the constant domain CH1 of the first Fab molecule under a). In particular such aspects, the constant domain CL of the second Fab molecule under b) is of kappa isotype.

The T cell activating bispecific antigen binding molecule according to the present disclosure may further comprise a third Fab molecule which specifically binds to the first antigen. In particular aspects, said third Fab molecule is identical to the first Fab molecule under a). In these aspects, the amino acid substitutions according to the above aspects will be made in the constant domain CL and the constant domain CH1 of each of the first Fab molecule and the third Fab molecule. Alternatively, the amino acid substitutions according to the above aspects may be made in the constant domain CL and the constant domain CH1 of the second Fab molecule under b), but not in the constant domain CL and the constant domain CH1 of the first Fab molecule and the third Fab molecule.

In particular aspects, the T cell activating bispecific antigen binding molecule according to the present disclosure further comprises an Fc domain composed of a first and a second subunit capable of stable association.

### T cell activating bispecific antigen binding molecule formats

The components of the T cell activating bispecific antigen binding molecule can be fused to each other in a variety of configurations. Exemplary configurations are depicted in Figure 1.

In particular aspects, the antigen binding moieties comprised in the T cell activating bispecific antigen binding molecule are Fab molecules. In such aspects, the first, second, third etc. antigen binding moiety may be referred to herein as first, second, third etc. Fab molecule, respectively. Furthermore, in particular aspects, the T cell activating bispecific antigen binding molecule comprises an Fc domain composed of a first and a second subunit capable of stable association.

In some aspects, the second Fab molecule is fused at the C-terminus of the Fab heavy chain to the N-terminus of the first or the second subunit of the Fc domain.

In one such aspect, the first Fab molecule is fused at the C-terminus of the Fab heavy chain to the N-terminus of the Fab heavy chain of the second Fab molecule. In a specific such aspect, the T cell activating bispecific antigen binding molecule essentially consists of the first and the second Fab molecule, the Fc domain composed of a first and a second subunit, and optionally one or more peptide linkers, wherein the first Fab molecule is fused at the C-terminus of the Fab heavy chain to the N-terminus of the Fab heavy chain of the second Fab molecule, and the second Fab molecule is fused at the C-terminus of the Fab heavy chain to the N-terminus of the first or the second subunit of the Fc domain. Such a configuration is schematically depicted in Figures 1G and 1K. Optionally, the Fab light chain of the first Fab molecule and the Fab light chain of the second Fab molecule may additionally be fused to each other.

In another such aspect, the first Fab molecule is fused at the C-terminus of the Fab heavy chain to the N-terminus of the first or second subunit of the Fc domain. In a specific such aspect, the T cell activating bispecific antigen binding molecule essentially consists of the first and the second Fab molecule, the Fc domain composed of a first and a second subunit, and optionally one or more peptide linkers, wherein the first and the second Fab molecule are each fused at the C-terminus of the Fab heavy chain to the N-terminus of one of the subunits of the Fc domain. Such a configuration is schematically depicted in Figures 1A and 1D. The first and the second Fab molecule may be fused to the Fc domain directly or through a peptide linker. In a particular aspect the first and the second Fab molecule are each fused to the Fc domain through an immunoglobulin hinge region. In a specific aspect, the immunoglobulin hinge region is a human IgG₁ hinge region, particularly where the Fc domain is an IgG₁ Fc domain.

In other aspects, the first Fab molecule is fused at the C-terminus of the Fab heavy chain to the N-terminus of the first or second subunit of the Fc domain.

In one such aspect, the second Fab molecule is fused at the C-terminus of the Fab heavy chain to the N-terminus of the Fab heavy chain of the first Fab molecule. In a specific such aspect, the T cell activating bispecific antigen binding molecule essentially consists of the first and the second Fab molecule, the Fc domain composed of a first and a second subunit, and optionally one or more peptide linkers, wherein the second Fab molecule is fused at the C-terminus of the Fab heavy chain to the N-terminus of the Fab heavy chain of the first Fab molecule, and the first Fab molecule is fused at the C-terminus of the Fab heavy chain to the N-terminus of the first or the second subunit of the Fc domain. Such a configuration is schematically depicted in Figures 1H and 1L. Optionally, the Fab light chain of the first Fab molecule and the Fab light chain of the second Fab molecule may additionally be fused to each other.

The Fab molecules may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, (G₄S)ₙ, (SG₄)ₙ, (G₄S)ₙ or G₄(SG₄)ₙ peptide linkers. "n" is generally an integer from 1 to 10, typically from 2 to 4. In one aspect said peptide linker has a length of at least 5 amino acids, in one aspect a length of 5 to 100, in a further aspect of 10 to 50 amino acids. In one aspect said peptide linker is (GxS)ₙ or (GxS)ₙGₘ with G=glycine, S=serine, and (x=3, n= 3, 4, 5 or 6, and m=0, 1, 2 or 3) or (x=4, n=2, 3, 4 or 5 and m= 0, 1, 2 or 3), in one aspect x=4 and n=2 or 3, in a further aspect x=4 and n=2. In one aspect said peptide linker is (G₄S)₂. A particularly suitable peptide linker for fusing the Fab light chains of the first and the second Fab molecule to each other is (G₄S)₂. An exemplary peptide linker suitable for connecting the Fab heavy chains of the first and the second Fab fragments comprises the sequence (D)-(G₄S)₂ (SEQ ID NOs 11 and 12). Another suitable such linker comprises the sequence (G₄S)₄. Additionally, linkers may comprise (a portion of) an immunoglobulin hinge region. Particularly where a Fab molecule is fused to the N-terminus of an Fc domain subunit, it may be fused via an immunoglobulin hinge region or a portion thereof, with or without an additional peptide linker. A T cell activating bispecific antigen binding molecule with a single antigen binding moiety (such as a Fab molecule) capable of specific binding to a target cell antigen (for example as shown in Figure 1A, D, G, H, K, L) is useful, particularly in cases where internalization of the target cell antigen is to be expected following binding of a high affinity antigen binding moiety. In such cases, the presence of more than one antigen binding moiety specific for the target cell antigen may enhance internalization of the target cell antigen, thereby reducing its availablity.

In many other cases, however, it will be advantageous to have a T cell activating bispecific antigen binding molecule comprising two or more antigen binding moieties (such as Fab moelcules) specific for a target cell antigen (see examples shown in Figure 1B, 1C, 1E, 1F, 1I, 1J. 1M or 1N), for example to optimize targeting to the target site or to allow crosslinking of target cell antigens.

Accordingly, in particular aspects, the T cell activating bispecific antigen binding molecule disclosed herein further comprises a third Fab molecule which specifically binds to the first antigen. The first antigen preferably is the target cell antigen, i.e. CD19. In one aspect, the third Fab molecule is a conventional Fab molecule. In one aspect, the third Fab molecule is identical to the first Fab molecule (i.e. the first and the third Fab molecule comprise the same heavy and light chain amino acid sequences and have the same arrangement of domains (i.e. conventional or crossover)). In a particular aspect, the second Fab molecule specifically binds to an activating T cell antigen, particularly CD3, and the first and third Fab molecule specifically bind to CD19. In alternative aspects, the T cell activating bispecific antigen binding molecule disclosed herein further comprises a third Fab molecule which specifically binds to the second antigen. In these aspects, the second antigen preferably is the target cell antigen, i.e. CD19. In one such aspect, the third Fab molecule is a crossover Fab molecule (a Fab molecule wherein the variable domains VH and VL or the constant domains CL and CH1 of the Fab heavy and light chains are exchanged / replaced by each other). In one such aspect, the third Fab molecule is identical to the second Fab molecule (i.e. the second and the third Fab molecule comprise the same heavy and light chain amino acid sequences and have the same arrangement of domains (i.e. conventional or crossover)). In one such aspect, the first Fab molecule specifically binds to an activating T cell antigen, particularly CD3, and the second and third Fab molecule specifically bind to CD19.

In one aspect, the third Fab molecule is fused at the C-terminus of the Fab heavy chain to the N-terminus of the first or second subunit of the Fc domain.

In a particular aspect, the second and the third Fab molecule are each fused at the C-terminus of the Fab heavy chain to the N-terminus of one of the subunits of the Fc domain, and the first Fab molecule is fused at the C-terminus of the Fab heavy chain to the N-terminus of the Fab heavy chain of the second Fab molecule. In a specific such aspect, the T cell activating bispecific antigen binding molecule essentially consists of the first, the second and the third Fab molecule, the Fc domain composed of a first and a second subunit, and optionally one or more peptide linkers, wherein the first Fab molecule is fused at the C-terminus of the Fab heavy chain to the N-terminus of the Fab heavy chain of the second Fab molecule, and the second Fab molecule is fused at the C-terminus of the Fab heavy chain to the N-terminus of the first subunit of the Fc domain, and wherein the third Fab molecule is fused at the C-terminus of the Fab heavy chain to the N-terminus of the second subunit of the Fc domain. Such a configuration is schematically depicted in Figure 1B and 1E (particular aspects, wherein the third Fab molecule is a conventional Fab molecule and preferably identical to the first Fab molecule), and Figure 1I and 1M (alternative aspects, wherein the third Fab molecule is a crossover Fab molecule and preferably identical to the second Fab molecule). The second and the third Fab molecule may be fused to the Fc domain directly or through a peptide linker. In a particular aspect the second and the third Fab molecule are each fused to the Fc domain through an immunoglobulin hinge region. In a specific aspect, the immunoglobulin hinge region is a human IgG₁ hinge region, particularly where the Fc domain is an IgG₁ Fc domain. Optionally, the Fab light chain of the first Fab molecule and the Fab light chain of the second Fab molecule may additionally be fused to each other.

In another aspect, the first and the third Fab molecule are each fused at the C-terminus of the Fab heavy chain to the N-terminus of one of the subunits of the Fc domain, and the second Fab molecule is fused at the C-terminus of the Fab heavy chain to the N-terminus of the Fab heavy chain of the first Fab molecule. In a specific such aspect, the T cell activating bispecific antigen binding molecule essentially consists of the first, the second and the third Fab molecule, the Fc domain composed of a first and a second subunit, and optionally one or more peptide linkers, wherein the second Fab molecule is fused at the C-terminus of the Fab heavy chain to the N-terminus of the Fab heavy chain of the first Fab molecule, and the first Fab molecule is fused at the C-terminus of the Fab heavy chain to the N-terminus of the first subunit of the Fc domain, and wherein the third Fab molecule is fused at the C-terminus of the Fab heavy chain to the N-terminus of the second subunit of the Fc domain. Such a configuration is schematically depicted in Figure 1C and 1F (particular aspects, wherein the third Fab molecule is a conventional Fab molecule and preferably identical to the first Fab molecule) and in Figure 1J and 1N (alternative aspects, wherein the third Fab molecule is a crossover Fab molecule and preferably identical to the second Fab molecule). The first and the third Fab molecule may be fused to the Fc domain directly or through a peptide linker. In a particular aspect the first and the third Fab molecule are each fused to the Fc domain through an immunoglobulin hinge region. In a specific aspect, the immunoglobulin hinge region is a human IgG₁ hinge region, particularly where the Fc domain is an IgG₁ Fc domain. Optionally, the Fab light chain of the first Fab molecule and the Fab light chain of the second Fab molecule may additionally be fused to each other.

In configurations of the T cell activating bispecific antigen binding molecule wherein a Fab molecule is fused at the C-terminus of the Fab heavy chain to the N-terminus of each of the subunits of the Fc domain through an immunoglobulin hinge regions, the two Fab molecules, the hinge regions and the Fc domain essentially form an immunoglobulin molecule. In a particular aspect the immunoglobulin molecule is an IgG class immunoglobulin. In an even more particular aspect the immunoglobulin is an IgG₁ subclass immunoglobulin. In another aspect the immunoglobulin is an IgG₄ subclass immunoglobulin. In a further particular aspect the immunoglobulin is a human immunoglobulin. In other aspects the immunoglobulin is a chimeric immunoglobulin or a humanized immunoglobulin.

In some of the T cell activating bispecific antigen binding moleculedisclosed herein, the Fab light chain of the first Fab molecule and the Fab light chain of the second Fab molecule are fused to each other, optionally via a peptide lnker. Depending on the configuration of the first and the second Fab molecule, the Fab light chain of the first Fab molecule may be fused at its C-terminus to the N-terminus of the Fab light chain of the second Fab molecule, or the Fab light chain of the second Fab molecule may be fused at its C-terminus to the N-terminus of the Fab light chain of the first Fab molecule. Fusion of the Fab light chains of the first and the second Fab molecule further reduces mispairing of unmatched Fab heavy and light chains, and also reduces the number of plasmids needed for expression of some of the T cell activating bispecific antigen binding moleculesdisclosed herein.

In certain aspects the T cell activating bispecific antigen binding molecule according to the present disclosure comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with an Fc domain subunit (VL₍₂₎-CH1₍₂₎-CH2-CH3(-CH4)), and a polypeptide wherein the Fab heavy chain of the first Fab molecule shares a carboxy-terminal peptide bond with an Fc domain subunit (VH₍₁₎-CH1₍₁₎-CH2-CH3(-CH4)). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In certain aspects the polypeptides are covalently linked, e.g., by a disulfide bond. In certain aspects the T cell activating bispecific antigen binding molecule according to the present disclosure comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with an Fc domain subunit (VH₍₂₎-CL₍₂₎-CH2-CH3(-CH4)), and a polypeptide wherein the Fab heavy chain of the first Fab molecule shares a carboxy-terminal peptide bond with an Fc domain subunit (VH₍₁₎-CH1₍₁₎-CH2-CH3(-CH4)). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In certain aspects the polypeptides are covalently linked, e.g., by a disulfide bond.

In some aspects, the T cell activating bispecific antigen binding molecule comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule, which in turn shares a carboxy-terminal peptide bond with an Fc domain subunit (VL₍₂₎-CH1₍₂₎-VH₍₁₎-CH1₍₁₎-CH2-CH3(-CH4)). In other aspects, the T cell activating bispecific antigen binding molecule comprises a polypeptide wherein the Fab heavy chain of the first Fab molecule shares a carboxy-terminal peptide bond with the Fab light chain variable region of the second Fab molecule which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with an Fc domain subunit (VH₍₁₎-CH1₍₁₎-VL₍₂₎-CH1₍₂₎-CH2-CH3(-CH4)).

In some of these aspects the T cell activating bispecific antigen binding molecule further comprises a crossover Fab light chain polypeptide of the second Fab molecule, wherein the Fab heavy chain variable region of the second Fab molecule shares a carboxy-terminal peptide bond with the Fab light chain constant region of the second Fab molecule (VH₍₂₎-CL₍₂₎), and the Fab light chain polypeptide of the first Fab molecule (VL₍₁₎-CL₍₁₎). In others of these aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule which in turn shares a carboxy-terminal peptide bond with the Fab light chain polypeptide of the first Fab molecule (VH₍₂₎-CL₍₂₎-VL₍₁₎-CL₍₁₎), or a polypeptide wherein the Fab light chain polypeptide of the first Fab molecule shares a carboxy-terminal peptide bond with the Fab heavy chain variable region of the second Fab molecule which in turn shares a carboxy-terminal peptide bond with the Fab light chain constant region of the second Fab molecule (VL₍₁₎-CL₍₁₎-VH₍₂₎-CL₍₂₎), as appropriate. The T cell activating bispecific antigen binding molecule according to these aspects may further comprise (i) an Fc domain subunit polypeptide (CH2-CH3(-CH4)), or (ii) a polypeptide wherein the Fab heavy chain of a third Fab molecule shares a carboxy-terminal peptide bond with an Fc domain subunit (VH₍₃₎-CH1₍₃₎-CH2-CH3(-CH4)) and the Fab light chain polypeptide of a third Fab molecule (VL₍₃₎-CL₍₃₎). In certain aspects the polypeptides are covalently linked, e.g., by a disulfide bond.

In some aspects, the T cell activating bispecific antigen binding molecule comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule, which in turn shares a carboxy-terminal peptide bond with an Fc domain subunit (VH₍₂₎-CL₍₂₎-VH₍₁₎-CH1₍₁₎-CH2-CH3(-CH4)). In other aspects, the T cell activating bispecific antigen binding molecule comprises a polypeptide wherein the Fab heavy chain of the first Fab molecule shares a carboxy-terminal peptide bond with the Fab heavy chain variable region of the second Fab molecule which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with an Fc domain subunit (VH₍₁₎-CH1₍₁₎-VH₍₂₎-CL₍₂₎-CH2-CH3(-CH4)).

In some of these aspects the T cell activating bispecific antigen binding molecule further comprises a crossover Fab light chain polypeptide of the second Fab molecule, wherein the Fab light chain variable region of the second Fab molecule shares a carboxy-terminal peptide bond with the Fab heavy chain constant region of the second Fab molecule (VL₍₂₎-CH1₍₂₎), and the Fab light chain polypeptide of the first Fab molecule (VL₍₁₎-CL₍₁₎). In others of these aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule which in turn shares a carboxy-terminal peptide bond with the Fab light chain polypeptide of the first Fab molecule (VL₍₂₎-CH1₍₂₎-VL₍₁₎-CL₍₁₎), or a polypeptide wherein the Fab light chain polypeptide of the first Fab molecule shares a carboxy-terminal peptide bond with the Fab heavy chain variable region of the second Fab molecule which in turn shares a carboxy-terminal peptide bond with the Fab light chain constant region of the second Fab molecule (VL₍₁₎-CL₍₁₎-VH₍₂₎-CL₍₂₎), as appropriate. The T cell activating bispecific antigen binding molecule according to these aspects may further comprise (i) an Fc domain subunit polypeptide (CH2-CH3(-CH4)), or (ii) a polypeptide wherein the Fab heavy chain of a third Fab molecule shares a carboxy-terminal peptide bond with an Fc domain subunit (VH₍₃₎-CH1₍₃₎-CH2-CH3(-CH4)) and the Fab light chain polypeptide of a third Fab molecule (VL₍₃₎-CL₍₃₎). In certain aspects the polypeptides are covalently linked, e.g., by a disulfide bond.

In some aspects, the first Fab molecule is fused at the C-terminus of the Fab heavy chain to the N-terminus of the Fab heavy chain of the second Fab molecule. In certain such aspects, the T cell activating bispecific antigen binding molecule does not comprise an Fc domain. In certain aspects, the T cell activating bispecific antigen binding molecule essentially consists of the first and the second Fab molecule, and optionally one or more peptide linkers, wherein the first Fab molecule is fused at the C-terminus of the Fab heavy chain to the N-terminus of the Fab heavy chain of the second Fab molecule. Such a configuration is schematically depicted in Figures 1O and 1S.

In other aspects, the second Fab molecule is fused at the C-terminus of the Fab heavy chain to the N-terminus of the Fab heavy chain of the first Fab molecule. In certain such aspects, the T cell activating bispecific antigen binding molecule does not comprise an Fc domain. In certain aspects, the T cell activating bispecific antigen binding molecule essentially consists of the first and the second Fab molecule, and optionally one or more peptide linkers, wherein the second Fab molecule is fused at the C-terminus of the Fab heavy chain to the N-terminus of the Fab heavy chain of the first Fab molecule. Such a configuration is schematically depicted in Figures 1P and 1T.

In some aspects, the first Fab molecule is fused at the C-terminus of the Fab heavy chain to the N-terminus of the Fab heavy chain of the second Fab molecule, and the T cell activating bispecific antigen binding molecule further comprises a third Fab molecule, wherein said third Fab molecule is fused at the C-terminus of the Fab heavy chain to the N-terminus of the Fab heavy chain of the first Fab molecule. In particular such aspects, said third Fab molecule is a conventional Fab molecule. In other such aspects, said third Fab molecule is a crossover Fab molecule as described herein, i.e. a Fab molecule wherein the variable domains VH and VL or the constant domains CL and CH1 of the Fab heavy and light chains are exchanged / replaced by each other. In certain such aspects, the T cell activating bispecific antigen binding molecule essentially consists of the first, the second and the third Fab molecule, and optionally one or more peptide linkers, wherein the first Fab molecule is fused at the C-terminus of the Fab heavy chain to the N-terminus of the Fab heavy chain of the second Fab molecule, and the third Fab molecule is fused at the C-terminus of the Fab heavy chain to the N-terminus of the Fab heavy chain of the first Fab molecule. Such a configuration is schematically depicted in Figure 1Q and 1U (particular aspects, wherein the third Fab molecule is a conventional Fab molecule and preferably identical to the first Fab molecule).

In some aspects, the first Fab molecule is fused at the C-terminus of the Fab heavy chain to the N-terminus of the Fab heavy chain of the second Fab molecule, and the T cell activating bispecific antigen binding molecule further comprises a third Fab molecule, wherein said third Fab molecule is fused at the N-terminus of the Fab heavy chain to the C-terminus of the Fab heavy chain of the second Fab molecule. In particular such aspects, said third Fab molecule is a crossover Fab molecule as described herein, i.e. a Fab molecule wherein the variable domains VH and VL or the constant domains CH1 and CL of the Fab heavy and light chains are exchanged / replaced by each other. In other such aspects, said third Fab molecule is a conventional Fab molecule. In certain such aspects, the T cell activating bispecific antigen binding molecule essentially consists of the first, the second and the third Fab molecule, and optionally one or more peptide linkers, wherein the first Fab molecule is fused at the C-terminus of the Fab heavy chain to the N-terminus of the Fab heavy chain of the second Fab molecule, and the third Fab molecule is fused at the N-terminus of the Fab heavy chain to the C-terminus of the Fab heavy chain of the second Fab molecule. Such a configuration is schematically depicted in Figure 1W and 1Y (particular aspects, wherein the third Fab molecule is a crossover Fab molecule and preferably identical to the second Fab molecule).

In some aspects, the second Fab molecule is fused at the C-terminus of the Fab heavy chain to the N-terminus of the Fab heavy chain of the first Fab molecule, and the T cell activating bispecific antigen binding molecule further comprises a third Fab molecule, wherein said third Fab molecule is fused at the N-terminus of the Fab heavy chain to the C-terminus of the Fab heavy chain of the first Fab molecule. In particular such aspects, said third Fab molecule is a conventional Fab molecule. In other such aspects, said third Fab molecule is a crossover Fab molecule as described herein, i.e. a Fab molecule wherein the variable domains VH and VL or the constant domains CH1 and CL of the Fab heavy and light chains are exchanged / replaced by each other. In certain such aspects, the T cell activating bispecific antigen binding molecule essentially consists of the first, the second and the third Fab molecule, and optionally one or more peptide linkers, wherein the second Fab molecule is fused at the C-terminus of the Fab heavy chain to the N-terminus of the Fab heavy chain of the first Fab molecule, and the third Fab molecule is fused at the N-terminus of the Fab heavy chain to the C-terminus of the Fab heavy chain of the first Fab molecule. Such a configuration is schematically depicted in Figure 1R and 1V (particular aspects, wherein the third Fab molecule is a conventional Fab molecule and preferably identical to the first Fab molecule).

In some aspects, the second Fab molecule is fused at the C-terminus of the Fab heavy chain to the N-terminus of the Fab heavy chain of the first Fab molecule, and the T cell activating bispecific antigen binding molecule further comprises a third Fab molecule, wherein said third Fab molecule is fused at the C-terminus of the Fab heavy chain to the N-terminus of the Fab heavy chain of the second Fab molecule. In particular such aspects, said third Fab molecule is a crossover Fab molecule as described herein, i.e. a Fab molecule wherein the variable domains VH and VL or the constant domains CH1 and CL of the Fab heavy and light chains are exchanged / replaced by each other. In other such aspects, said third Fab molecule is a conventional Fab molecule. In certain such aspects, the T cell activating bispecific antigen binding molecule essentially consists of the first, the second and the third Fab molecule, and optionally one or more peptide linkers, wherein the second Fab molecule is fused at the C-terminus of the Fab heavy chain to the N-terminus of the Fab heavy chain of the first Fab molecule, and the third Fab molecule is fused at the C-terminus of the Fab heavy chain to the N-terminus of the Fab heavy chain of the second Fab molecule. Such a configuration is schematically depicted in Figure 1X and 1Z (particular aspects, wherein the third Fab molecule is a crossover Fab molecule and preferably identical to the first Fab molecule).

In certain aspects the T cell activating bispecific antigen binding molecule according to the present disclosure comprises a polypeptide wherein the Fab heavy chain of the first Fab molecule shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region) (VH₍₁₎-CH1₍₁₎-VL₍₂₎-CH1₍₂₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎).

In certain aspects the T cell activating bispecific antigen binding molecule according to the present disclosure comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule (VL₍₂₎-CH1₍₂₎-VH₍₁₎-CH1₍₁₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎).

In certain aspects the T cell activating bispecific antigen binding molecule according to the present disclosure comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule (VH₍₂₎-CL₍₂₎-VH₍₁₎-CH1₍₁₎). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎).

In certain aspects the T cell activating bispecific antigen binding molecule according to the present disclosure comprises a polypeptide wherein the Fab heavy chain of a third Fab molecule shares a carboxy-terminal peptide bond with the Fab heavy chain of the first Fab molecule, which in turn shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region) (VH₍₃₎-CH1₍₃₎-VH₍₁₎-CH1₍₁₎-VL₍₂₎-CH1₍₂₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some aspects the T cell activating bispecific antigen binding molecule further comprises the Fab light chain polypeptide of a third Fab molecule (VL₍₃₎-CL₍₃₎).

In certain aspects the T cell activating bispecific antigen binding molecule according to the present disclosure comprises a polypeptide wherein the Fab heavy chain of a third Fab molecule shares a carboxy-terminal peptide bond with the Fab heavy chain of the first Fab molecule, which in turn shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region) (VH₍₃₎-CH1₍₃₎-VH₍₁₎-CH1₍₁₎-VH₍₂₎-CL₍₂₎). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some aspects the T cell activating bispecific antigen binding molecule further comprises the Fab light chain polypeptide of a third Fab molecule (VL₍₃₎-CL₍₃₎).

In certain aspects the T cell activating bispecific antigen binding molecule according to the present disclosure comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule, which in turn shares a carboxy-terminal peptide bond with the Fab heavy chain of a third Fab molecule (VL₍₂₎-CH1₍₂₎-VH₍₁₎-CH1₍₁₎-VH₍₃₎-CH1₍₃₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some aspects the T cell activating bispecific antigen binding molecule further comprises the Fab light chain polypeptide of a third Fab molecule (VL₍₃₎-CL₍₃₎).

In certain aspects the T cell activating bispecific antigen binding molecule according to the present disclosure comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule, which in turn shares a carboxy-terminal peptide bond with the Fab heavy chain of a third Fab molecule (VH₍₂₎-CL₍₂₎-VH₍₁₎-CH1₍₁₎-VH₍₃₎-CH1₍₃₎). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some aspects the T cell activating bispecific antigen binding molecule further comprises the Fab light chain polypeptide of a third Fab molecule (VL₍₃₎-CL₍₃₎).

In certain aspects the T cell activating bispecific antigen binding molecule according to the present disclosure comprises a polypeptide wherein the Fab heavy chain of the first Fab molecule shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab light chain variable region of a third Fab molecule, which in turn shares a carboxy-terminal peptide bond with the Fab heavy chain constant region of a third Fab molecule (i.e. the third Fab molecule comprises a crossover Fab heavy chain, wherein the heavy chain variable region is replaced by a light chain variable region) (VH₍₁₎-CH1₍₁₎-VL₍₂₎-CH1₍₂₎-VL₍₃₎-CH1₍₃₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some aspects the T cell activating bispecific antigen binding molecule further comprises a polypeptide wherein the Fab heavy chain variable region of a third Fab molecule shares a carboxy-terminal peptide bond with the Fab light chain constant region of a third Fab molecule (VH₍₃₎-CL₍₃₎).

In certain aspects the T cell activating bispecific antigen binding molecule according to the present disclosure comprises a polypeptide wherein the Fab heavy chain of the first Fab molecule shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain variable region of a third Fab molecule, which in turn shares a carboxy-terminal peptide bond with the Fab light chain constant region of a third Fab molecule (i.e. the third Fab molecule comprises a crossover Fab heavy chain, wherein the heavy chain constant region is replaced by a light chain constant region) (VH₍₁₎-CH1₍₁₎-VH₍₂₎-CL₍₂₎-VH₍₃₎-CL₍₃₎). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some aspects the T cell activating bispecific antigen binding molecule further comprises a polypeptide wherein the Fab light chain variable region of a third Fab molecule shares a carboxy-terminal peptide bond with the Fab heavy chain constant region of a third Fab molecule (VL₍₃₎-CH₍₃₎).

In certain aspects the T cell activating bispecific antigen binding molecule according to the present disclosure comprises a polypeptide wherein the Fab light chain variable region of a third Fab molecule shares a carboxy-terminal peptide bond with the Fab heavy chain constant region of a third Fab molecule (i.e. the third Fab molecule comprises a crossover Fab heavy chain, wherein the heavy chain variable region is replaced by a light chain variable region), which in turn shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule (VL₍₃₍-CH1₍₃₎-VL₍₂₎-CH1₍₂₎-VH₍₁₎-CH1₍₁₎). In some aspects the T cell activating bispecific antigen binding molecule further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some aspects the T cell activating bispecific antigen binding molecule further comprises a polypeptide wherein the Fab heavy chain variable region of a third Fab molecule shares a carboxy-terminal peptide bond with the Fab light chain constant region of a third Fab molecule (VH₍₃₎-CL₍₃₎).

In certain aspects the T cell activating bispecific antigen binding molecule according to the present disclosure comprises a polypeptide wherein the Fab heavy chain variable region of a third Fab molecule shares a carboxy-terminal peptide bond with the Fab light chain constant region of a third Fab molecule (i.e. the third Fab molecule comprises a crossover Fab heavy chain, wherein the heavy chain constant region is replaced by a light chain constant region), which in turn shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule (VH₍₃₎-CL₍₃₎-VH₍₂₎-CL₍₂₎-VH₍₁₎-CH1₍₁₎). In some aspects the T cell activating bispecific antigen binding molecule further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some aspects the T cell activating bispecific antigen binding molecule further comprises a polypeptide wherein the Fab light chain variable region of a third Fab molecule shares a carboxy-terminal peptide bond with the Fab heavy chain constant region of a third Fab molecule (VL₍₃₎-CH₍₃₎).

According to any of the above aspects, components of the T cell activating bispecific antigen binding molecule (e.g. Fab molecules, Fc domain) may be fused directly or through various linkers, particularly peptide linkers comprising one or more amino acids, typically about 2-20 amino acids, that are described herein or are known in the art. Suitable, non-immunogenic peptide linkers include, for example, (G₄S)ₙ, (SG₄)ₙ, (G₄S)ₙ or G₄(SG₄)ₙ peptide linkers, wherein n is generally an integer from 1 to 10, typically from 2 to 4.

### Fc domain

The Fc domain of the T cell activating bispecific antigen binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one aspect the T cell activating bispecific antigen binding molecule disclosed herein comprises not more than one Fc domain.

In one aspect, the Fc domain of the T cell activating bispecific antigen binding molecule is an IgG Fc domain. In a particular aspect the Fc domain is an IgG₁ Fc domain. In another aspect the Fc domain is an IgG₄ Fc domain. In a more specific aspect, the Fc domain is an IgG₄ Fc domain comprising an amino acid substitution at position S228 (Kabat numbering), particularly the amino acid substitution S228P. This amino acid substitution reduces in vivo Fab arm exchange of IgG₄ antibodies (see Stubenrauch et al., Drug Metabolism and Disposition 38, 84-91 (2010)). In a further particular aspect the Fc domain is human. An exemplary sequence of a human IgG₁ Fc region is given in SEQ ID NO: 13.

### Fc domain modifications promoting heterodimerization

T cell activating bispecific antigen binding molecules according to the present disclosure comprise different Fab molecules, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of T cell activating bispecific antigen binding molecules in recombinant production, it will thus be advantageous to introduce in the Fc domain of the T cell activating bispecific antigen binding molecule a modification promoting the association of the desired polypeptides.

Accordingly, in particular aspects the Fc domain of the T cell activating bispecific antigen binding molecule according to the present disclosure comprises a modification promoting the association of the first and the second subunit of the Fc domain. The site of most extensive protein-protein interaction between the two subunits of a human IgG Fc domain is in the CH3 domain of the Fc domain. Thus, in one aspect said modification is in the CH3 domain of the Fc domain.

There exist several approaches for modifications in the CH3 domain of the Fc domain in order to enforce heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291.

Typically, in all such approaches the CH3 domain of the first subunit of the Fc domain and the CH3 domain of the second subunit of the Fc domain are both engineered in a complementary manner so that each CH3 domain (or the heavy chain comprising it) can no longer homodimerize with itself but is forced to heterodimerize with the complementarily engineered other CH3 domain (so that the first and second CH3 domain heterodimerize and no homdimers between the two first or the two second CH3 domains are formed). These different approaches for improved heavy chain heterodimerization are contemplated as different alternatives in combination with the heavy-light chain modifications (VH and VL exchange/replacement in one binding arm and the introduction of substitutions of charged amino acids with opposite charges in the CH1/CL interface) in the T cell activating bispecific antigen binding molecule according to the present disclosure which reduce light chain mispairing and Bence Jones-type side products.

In a specific aspect said modification promoting the association of the first and the second subunit of the Fc domain is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular aspect, in the CH3 domain of the first subunit of the Fc domain of the T cell activating bispecific antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

Preferably said amino acid residue having a larger side chain volume is selected from the group consisting of arginine (R), phenylalanine (F), tyrosine (Y), and tryptophan (W).

Preferably said amino acid residue having a smaller side chain volume is selected from the group consisting of alanine (A), serine (S), threonine (T), and valine (V).

The protuberance and cavity can be made by altering the nucleic acid encoding the polypeptides, e.g. by site-specific mutagenesis, or by peptide synthesis.

In a specific aspect, in the CH3 domain of the first subunit of the Fc domain (the "knobs" subunit) the threonine residue at position 366 is replaced with a tryptophan residue (T366W), and in the CH3 domain of the second subunit of the Fc domain (the "hole" subunit) the tyrosine residue at position 407 is replaced with a valine residue (Y407V). In one aspect, in the second subunit of the Fc domain additionally the threonine residue at position 366 is replaced with a serine residue (T366S) and the leucine residue at position 368 is replaced with an alanine residue (L368A) (numberings according to Kabat EU index).

In yet a further aspect, in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C) (numberings according to Kabat EU index). Introduction of these two cysteine residues results in formation of a disulfide bridge between the two subunits of the Fc domain, further stabilizing the dimer (Carter, J Immunol Methods 248, 7-15 (2001)).

In a particular aspect, the first subunit of the Fc domain comprises amino acid substitutions S354C and T366W, and the second subunit of the Fc domain comprises amino acid substitutions Y349C, T366S, L368A and Y407V (numbering according to Kabat EU index).

In a particular aspect the Fab molecule which specifically binds an activating T cell antigen is fused (optionally via a Fab molecule which specifically binds to a target cell antigen) to the first subunit of the Fc domain (comprising the "knob" modification). Without wishing to be bound by theory, fusion of the Fab molecule which specifically binds an activating T cell antigen to the knob-containing subunit of the Fc domain will (further) minimize the generation of antigen binding molecules comprising two Fab molecules which bind to an activating T cell antigen (steric clash of two knob-containing polypeptides).

Other techniques of CH3-modification for enforcing the heterodimerization are contemplated as alternatives and are described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one aspect the heterodimerization approach described in EP 1870459 A1, is used alternatively. This approach is based on the introduction of charged amino acids with opposite charges at specific amino acid positions in the CH3/CH3 domain interface between the two subunits of the Fc domain. One preferred aspect for the T cell activating bispecific antigen binding molecule disclosed herein are amino acid mutations R409D; K370E in one of the two CH3 domains (of the Fc domain) and amino acid mutations D399K; E357K in the other one of the CH3 domains of the Fc domain (numbering according to Kabat EU index).

In another aspect the T cell activating bispecific antigen binding molecule disclosed herein comprises amino acid mutation T366W in the CH3 domain of the first subunit of the Fc domain and amino acid mutations T366S, L368A, Y407V in the CH3 domain of the second subunit of the Fc domain, and additionally amino acid mutations R409D; K370E in the CH3 domain of the first subunit of the Fc domain and amino acid mutations D399K; E357K in the CH3 domain of the second subunit of the Fc domain (numberings according to Kabat EU index).

In another aspect, the T cell activating bispecific antigen binding molecule disclosed herein comprises amino acid mutations S354C, T366W in the CH3 domain of the first subunit of the Fc domain and amino acid mutations Y349C, T366S, L368A, Y407V in the CH3 domain of the second subunit of the Fc domain, or said T cell activating bispecific antigen binding molecule comprises amino acid mutations Y349C, T366W in the CH3 domain of the first subunit of the Fc domain and amino acid mutations S354C, T366S, L368A, Y407V in the CH3 domains of the second subunit of the Fc domain and additionally amino acid mutations R409D; K370E in the CH3 domain of the first subunit of the Fc domain and amino acid mutations D399K; E357K in the CH3 domain of the second subunit of the Fc domain (all numberings according to Kabat EU index).

In one aspect the heterodimerization approach described in WO 2013/157953 is used alternatively. In one aspect a first CH3 domain comprises amino acid mutation T366K and a second CH3 domain comprises amino acid mutation L351D (numberings according to Kabat EU index). In a further aspect the first CH3 domain comprises further amino acid mutation L351K. In a further aspect the second CH3 domain comprises further an amino acid mutation selected from Y349E, Y349D and L368E (preferably L368E) (numberings according to Kabat EU index). In one aspect the heterodimerization approach described in WO 2012/058768 is used alternatively. In one aspect a first CH3 domain comprises amino acid mutations L351Y, Y407A and a second CH3 domain comprises amino acid mutations T366A, K409F. In a further aspect the second CH3 domain comprises a further amino acid mutation at position T411, D399, S400, F405, N390, or K392, e.g. selected from a) T411N, T411R, T411Q, T411K, T411D, T411E or T411W, b) D399R, D399W, D399Y or D399K, c) S400E, S400D, S400R, or S400K, d) F405I, F405M, F405T, F405S, F405V or F405W, e) N390R, N390K or N390D, f) K392V, K392M, K392R, K392L, K392F or K392E (numberings according to Kabat EU index). In a further aspect a first CH3 domain comprises amino acid mutations L351Y, Y407A and a second CH3 domain comprises amino acid mutations T366V, K409F. In a further aspect a first CH3 domain comprises amino acid mutation Y407A and a second CH3 domain comprises amino acid mutations T366A, K409F. In a further aspect the second CH3 domain further comprises amino acid mutations K392E, T411E, D399R and S400R (numberings according to Kabat EU index). In one aspect the heterodimerization approach described in WO 2011/143545 is used alternatively, e.g. with the amino acid modification at a position selected from the group consisting of 368 and 409 (numbering according to Kabat EU index).

In one aspect the heterodimerization approach described in WO 2011/090762, which also uses the knobs-into-holes technology described above, is used alternatively. In one aspect a first CH3 domain comprises amino acid mutation T366W and a second CH3 domain comprises amino acid mutation Y407A. In one aspect a first CH3 domain comprises amino acid mutation T366Y and a second CH3 domain comprises amino acid mutation Y407T (numberings according to Kabat EU index).

In one aspect the T cell activating bispecific antigen binding molecule or its Fc domain is of IgG₂ subclass and the heterodimerization approach described in WO 2010/129304 is used alternatively.

In an alternative aspect a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable. In one such aspect a first CH3 domain comprises amino acid substitution of K392 or N392 with a negatively charged amino acid (e.g. glutamic acid (E), or aspartic acid (D), preferably K392D or N392D) and a second CH3 domain comprises amino acid substitution of D399, E356, D356, or E357 with a positively charged amino acid (e.g. lysine (K) or arginine (R), preferably D399K, E356K, D356K, or E357K, and more preferably D399K and E356K). In a further aspect the first CH3 domain further comprises amino acid substitution of K409 or R409 with a negatively charged amino acid (e.g. glutamic acid (E), or aspartic acid (D), preferably K409D or R409D). In a further aspect the first CH3 domain further or alternatively comprises amino acid substitution of K439 and/or K370 with a negatively charged amino acid (e.g. glutamic acid (E), or aspartic acid (D)) (all numberings according to Kabat EU index).

In yet a further aspect the heterodimerization approach described in WO 2007/147901 is used alternatively. In one aspect a first CH3 domain comprises amino acid mutations K253E, D282K, and K322D and a second CH3 domain comprises amino acid mutations D239K, E240K, and K292D (numberings according to Kabat EU index).

In still another aspect the heterodimerization approach described in WO 2007/110205 can be used alternatively.

In one aspect, the first subunit of the Fc domain comprises amino acid substitutions K392D and K409D, and the second subunit of the Fc domain comprises amino acid substitutions D356K and D399K (numbering according to Kabat EU index).

### Fc domain modifications reducing Fc receptor binding and/or effector function

The Fc domain confers to the T cell activating bispecific antigen binding molecule favorable pharmacokinetic properties, including a long serum half-life which contributes to good accumulation in the target tissue and a favorable tissue-blood distribution ratio. At the same time it may, however, lead to undesirable targeting of the T cell activating bispecific antigen binding molecule to cells expressing Fc receptors rather than to the preferred antigen-bearing cells. Moreover, the co-activation of Fc receptor signaling pathways may lead to cytokine release which, in combination with the T cell activating properties and the long half-life of the antigen binding molecule, results in excessive activation of cytokine receptors and severe side effects upon systemic administration. Activation of (Fc receptor-bearing) immune cells other than T cells may even reduce efficacy of the T cell activating bispecific antigen binding molecule due to the potential destruction of T cells e.g. by NK cells.

Accordingly, in particular aspects, the Fc domain of the T cell activating bispecific antigen binding molecules according to the present disclosure exhibits reduced binding affinity to an Fc receptor and/or reduced effector function, as compared to a native IgG₁ Fc domain. In one such aspect the Fc domain (or the T cell activating bispecific antigen binding molecule comprising said Fc domain) exhibits less than 50%, preferably less than 20%, more preferably less than 10% and most preferably less than 5% of the binding affinity to an Fc receptor, as compared to a native IgG₁ Fc domain (or a T cell activating bispecific antigen binding molecule comprising a native IgG₁ Fc domain), and/or less than 50%, preferably less than 20%, more preferably less than 10% and most preferably less than 5% of the effector function, as compared to a native IgG₁ Fc domain domain (or a T cell activating bispecific antigen binding molecule comprising a native IgG₁ Fc domain). In one aspect, the Fc domain domain (or the T cell activating bispecific antigen binding molecule comprising said Fc domain) does not substantially bind to an Fc receptor and/or induce effector function. In a particular aspect the Fc receptor is an Fcγ receptor. In one aspect the Fc receptor is a human Fc receptor. In one aspect the Fc receptor is an activating Fc receptor. In a specific aspect the Fc receptor is an activating human Fcγ receptor, more specifically human FcγRIIIa, FcγRI or FcγRIIa, most specifically human FcγRIIIa. In one aspect the effector function is one or more selected from the group of CDC, ADCC, ADCP, and cytokine secretion. In a particular aspect the effector function is ADCC. In one aspect the Fc domain domain exhibits substantially similar binding affinity to neonatal Fc receptor (FcRn), as compared to a native IgG₁ Fc domain domain. Substantially similar binding to FcRn is achieved when the Fc domain (or the T cell activating bispecific antigen binding molecule comprising said Fc domain) exhibits greater than about 70%, particularly greater than about 80%, more particularly greater than about 90% of the binding affinity of a native IgG₁ Fc domain (or the T cell activating bispecific antigen binding molecule comprising a native IgG₁ Fc domain) to FcRn. In certain aspects the Fc domain is engineered to have reduced binding affinity to an Fc receptor and/or reduced effector function, as compared to a non-engineered Fc domain. In particular aspects, the Fc domain of the T cell activating bispecific antigen binding molecule comprises one or more amino acid mutation that reduces the binding affinity of the Fc domain to an Fc receptor and/or effector function. Typically, the same one or more amino acid mutation is present in each of the two subunits of the Fc domain. In one aspect the amino acid mutation reduces the binding affinity of the Fc domain to an Fc receptor. In one aspect the amino acid mutation reduces the binding affinity of the Fc domain to an Fc receptor by at least 2-fold, at least 5-fold, or at least 10-fold. In aspects where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one aspect the T cell activating bispecific antigen binding molecule comprising an engineered Fc domain exhibits less than 20%, particularly less than 10%, more particularly less than 5% of the binding affinity to an Fc receptor as compared to a T cell activating bispecific antigen binding molecule comprising a non-engineered Fc domain. In a particular aspect the Fc receptor is an Fcγ receptor. In some aspects the Fc receptor is a human Fc receptor. In some aspects the Fc receptor is an activating Fc receptor. In a specific aspect the Fc receptor is an activating human Fcγ receptor, more specifically human FcγRIIIa, FcγRI or FcγRIIa, most specifically human FcγRIIIa. Preferably, binding to each of these receptors is reduced. In some aspects binding affinity to a complement component, specifically binding affinity to C1q, is also reduced. In one aspect binding affinity to neonatal Fc receptor (FcRn) is not reduced. Substantially similar binding to FcRn, i.e. preservation of the binding affinity of the Fc domain to said receptor, is achieved when the Fc domain (or the T cell activating bispecific antigen binding molecule comprising said Fc domain) exhibits greater than about 70% of the binding affinity of a non-engineered form of the Fc domain (or the T cell activating bispecific antigen binding molecule comprising said non-engineered form of the Fc domain) to FcRn. The Fc domain, or T cell activating bispecific antigen binding molecules disclosed herein comprising said Fc domain, may exhibit greater than about 80% and even greater than about 90% of such affinity. In certain aspects the Fc domain of the T cell activating bispecific antigen binding molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one aspect the reduced effector function is one or more selected from the group of reduced CDC, reduced ADCC, reduced ADCP, and reduced cytokine secretion. In a particular aspect the reduced effector function is reduced ADCC. In one aspect the reduced ADCC is less than 20% of the ADCC induced by a non-engineered Fc domain (or a T cell activating bispecific antigen binding molecule comprising a non-engineered Fc domain).

In one aspect the amino acid mutation that reduces the binding affinity of the Fc domain to an Fc receptor and/or effector function is an amino acid substitution. In one aspect the Fc domain comprises an amino acid substitution at a position selected from the group of E233, L234, L235, N297, P331 and P329 (numberings according to Kabat EU index). In a more specific aspect the Fc domain comprises an amino acid substitution at a position selected from the group of L234, L235 and P329 (numberings according to Kabat EU index). In some aspects the Fc domain comprises the amino acid substitutions L234A and L235A (numberings according to Kabat EU index). In one such aspect, the Fc domain is an IgG₁ Fc domain, particularly a human IgG₁ Fc domain. In one aspect the Fc domain comprises an amino acid substitution at position P329. In a more specific aspect the amino acid substitution is P329A or P329G, particularly P329G (numberings according to Kabat EU index). In one aspect the Fc domain comprises an amino acid substitution at position P329 and a further amino acid substitution at a position selected from E233, L234, L235, N297 and P331 (numberings according to Kabat EU index). In a more specific aspect the further amino acid substitution is E233P, L234A, L235A, L235E, N297A, N297D or P331S. In particular aspects the Fc domain comprises amino acid substitutions at positions P329, L234 and L235(numberings according to Kabat EU index). In more particular aspects the Fc domain comprises the amino acid mutations L234A, L235A and P329G ("P329G LALA"). In one such aspect, the Fc domain is an IgG₁ Fc domain, particularly a human IgG₁ Fc domain. The "P329G LALA" combination of amino acid substitutions almost completely abolishes Fcγ receptor (as well as complement) binding of a human IgG₁ Fc domain, as described in PCT publication no. WO 2012/130831. WO 2012/130831 also describes methods of preparing such mutant Fc domains and methods for determining its properties such as Fc receptor binding or effector functions.

IgG₄ antibodies exhibit reduced binding affinity to Fc receptors and reduced effector functions as compared to IgG₁ antibodies. Hence, in some aspects the Fc domain of the T cell activating bispecific antigen binding molecules disclosed herein is an IgG₄ Fc domain, particularly a human IgG₄ Fc domain. In one aspect the IgG₄ Fc domain comprises amino acid substitutions at position S228, specifically the amino acid substitution S228P (numberings according to Kabat EU index). To further reduce its binding affinity to an Fc receptor and/or its effector function, in one aspect the IgG₄ Fc domain comprises an amino acid substitution at position L235, specifically the amino acid substitution L235E (numberings according to Kabat EU index). In another aspect, the IgG₄ Fc domain comprises an amino acid substitution at position P329, specifically the amino acid substitution P329G (numberings according to Kabat EU index). In a particular aspect, the IgG₄ Fc domain comprises amino acid substitutions at positions S228, L235 and P329, specifically amino acid substitutions S228P, L235E and P329G (numberings according to Kabat EU index). Such IgG₄ Fc domain mutants and their Fcγ receptor binding properties are described in PCT publication no. WO 2012/130831.

In a particular aspect the Fc domain exhibiting reduced binding affinity to an Fc receptor and/or reduced effector function, as compared to a native IgG₁ Fc domain, is a human IgG₁ Fc domain comprising the amino acid substitutions L234A, L235A and optionally P329G, or a human IgG₄ Fc domain comprising the amino acid substitutions S228P, L235E and optionally P329G (numberings according to Kabat EU index).

In certain aspects N-glycosylation of the Fc domain has been eliminated. In one such aspect the Fc domain comprises an amino acid mutation at position N297, particularly an amino acid substitution replacing asparagine by alanine (N297A) or aspartic acid (N297D) (numberings according to Kabat EU index).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056) (numberings according to Kabat EU index). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or a T cell activating bispecific antigen binding molecule comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI^{™} non-radioactive cytotoxicity assay for flow cytometry (CellTechnology, Inc. Mountain View, CA); and CytoTox 96^{®} non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some aspects, binding of the Fc domain to a complement component, specifically to C1q, is reduced. Accordingly, in some aspects wherein the Fc domain is engineered to have reduced effector function, said reduced effector function includes reduced CDC. C1q binding assays may be carried out to determine whether the T cell activating bispecific antigen binding molecule is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

### Antigen Binding Moieties

The antigen binding molecule disclosed herein is bispecific, i.e. it comprises at least two antigen binding moieties capable of specific binding to two distinct antigenic determinants. According to particular aspects, the antigen binding moieties are Fab molecules (i.e. antigen binding domains composed of a heavy and a light chain, each comprising a variable and a constant domain). In one aspect said Fab molecules are human. In another aspect said Fab molecules are humanized. In yet another aspect said Fab molecules comprise human heavy and light chain constant domains.

Preferably, at least one of the antigen binding moieties is a crossover Fab molecule. Such modification reduces mispairing of heavy and light chains from different Fab molecules, thereby improving the yield and purity of the T cell activating bispecific antigen binding molecule disclosed herein in recombinant production. In a particular crossover Fab molecule useful for the T cell activating bispecific antigen binding molecule disclosed herein, the variable domains of the Fab light chain and the Fab heavy chain (VL and VH, respectively) are exchanged. Even with this domain exchange, however, the preparation of the T cell activating bispecific antigen binding molecule may comprise certain side products due to a so-called Bence Jones-type interaction between mispaired heavy and light chains (see Schaefer et al, PNAS, 108 (2011) 11187-11191). To further reduce mispairing of heavy and light chains from different Fab molecules and thus increase the purity and yield of the desired T cell activating bispecific antigen binding molecule, according to the present disclosure charged amino acids with opposite charges may be introduced at specific amino acid positions in the CH1 and CL domains of either the Fab molecule(s) specifically binding to a target cell antigen, or the Fab molecule specifically binding to an activating T cell antigen. Charge modifications are made either in the conventional Fab molecule(s) comprised in the T cell activating bispecific antigen binding molecule (such as shown e.g. in Figures 1 A-C, G-J), or in the VH/VL crossover Fab molecule(s) comprised in the T cell activating bispecific antigen binding molecule (such as shown e.g. in Figure 1 D-F, K-N) (but not in both). In particular aspects, the charge modifications are made in the conventional Fab molecule(s) comprised in the T cell activating bispecific antigen binding molecule (which in particular aspects specifically bind(s) to the target cell antigen).

In a particular aspect according to the present disclosure, the T cell activating bispecific antigen binding molecule is capable of simultaneous binding to a target cell antigen, particularly a tumor cell antigen, and an activating T cell antigen, particularly CD3. In one aspect, the T cell activating bispecific antigen binding molecule is capable of crosslinking a T cell and a target cell by simultaneous binding to a target cell antigen and an activating T cell antigen. In an even more particular aspect, such simultaneous binding results in lysis of the target cell, particularly a tumor cell. In one aspect, such simultaneous binding results in activation of the T cell. In other aspects, such simultaneous binding results in a cellular response of a T lymphocyte, particularly a cytotoxic T lymphocyte, selected from the group of: proliferation, differentiation, cytokine secretion, cytotoxic effector molecule release, cytotoxic activity, and expression of activation markers. In one aspect, binding of the T cell activating bispecific antigen binding molecule to the activating T cell antigen, particularly CD3, without simultaneous binding to the target cell antigen does not result in T cell activation.

In one aspect, the T cell activating bispecific antigen binding molecule is capable of re-directing cytotoxic activity of a T cell to a target cell. In a particular aspect, said re-direction is independent of MHC-mediated peptide antigen presentation by the target cell and and/or specificity of the T cell.

Particularly, a T cell according to any of the aspects described herein is a cytotoxic T cell. In some aspects the T cell is a CD4⁺ or a CD8⁺ T cell, particularly a CD8⁺ T cell.

### Activating T cell antigen binding moiety

The T cell activating bispecific antigen binding molecule of the present disclosure comprises at least one antigen binding moiety, particularly a Fab molecule, which specifically binds to an activating T cell antigen (also referred to herein as an "activating T cell antigen binding moiety, or activating T cell antigen binding Fab molecule"). In a particular aspect, the T cell activating bispecific antigen binding molecule comprises not more than one antigen binding moiety capable of specific binding to an activating T cell antigen. In one aspect the T cell activating bispecific antigen binding molecule provides monovalent binding to the activating T cell antigen.

In particular aspects, the antigen binding moiety which specifically binds an activating T cell antigen is a crossover Fab molecule as described herein, i.e. a Fab molecule wherein the variable domains VH and VL or the constant domains CH1 and CL of the Fab heavy and light chains are exchanged / replaced by each other. In such aspects, the antigen binding moiety(ies) which specifically binds a target cell antigen is preferably a conventional Fab molecule. In aspects where there is more than one antigen binding moiety, particularly Fab molecule, which specifically binds to a target cell antigen comprised in the T cell activating bispecific antigen binding molecule, the antigen binding moiety which specifically binds to an activating T cell antigen preferably is a crossover Fab molecule and the antigen binding moieties which specifically bind to a target cell antigen are conventional Fab molecules.

In alternative aspects, the antigen binding moiety which specifically binds an activating T cell antigen is a conventional Fab molecule. In such aspects, the antigen binding moiety(ies) which specifically binds a target cell antigen is a crossover Fab molecule as described herein, i.e. a Fab molecule wherein the variable domains VH and VL or the constant domains CH1 and CL of the Fab heavy and light chains are exchanged / replaced by each other.

In a particular aspect the activating T cell antigen is CD3, particularly human CD3 (SEQ ID NO: 1) or cynomolgus CD3 (SEQ ID NO: 2), most particularly human CD3. In a particular aspect the activating T cell antigen binding moiety is cross-reactive for (i.e. specifically binds to) human and cynomolgus CD3. In some aspects, the activating T cell antigen is the epsilon subunit of CD3 (CD3 epsilon).

In some aspects, the activating T cell antigen binding moiety specifically binds to CD3, particularly CD3 epsilon, and comprises at least one heavy chain complementarity determining region (CDR) selected from the group consisting of SEQ ID NO: 4, SEQ ID NO: 5 and SEQ ID NO: 6 and at least one light chain CDR selected from the group of SEQ ID NO: 8, SEQ ID NO: 9, SEQ ID NO: 10.

In one aspect the CD3 binding antigen binding moiety, particularly Fab molecule, comprises a heavy chain variable region comprising the heavy chain CDR1 of SEQ ID NO: 4, the heavy chain CDR2 of SEQ ID NO: 5, the heavy chain CDR3 of SEQ ID NO: 6, and a light chain variable region comprising the light chain CDR1 of SEQ ID NO: 8, the light chain CDR2 of SEQ ID NO: 9, and the light chain CDR3 of SEQ ID NO: 10.

In another aspect the CD3 binding antigen binding moiety, particularly Fab molecule, comprises a heavy chain variable region comprising the heavy chain CDR1 of SEQ ID NO: 4, the heavy chain CDR2 of SEQ ID NO: 37, the heavy chain CDR3 of SEQ ID NO: 6, and a light chain variable region comprising the light chain CDR1 of SEQ ID NO: 38, the light chain CDR2 of SEQ ID NO: 9, and the light chain CDR3 of SEQ ID NO: 10.

In one aspect the CD3 binding antigen binding moiety, particularly Fab molecule, comprises a heavy chain variable region sequence that is at least about 95%, 96%, 97%, 98%, 99% or 100% identical to SEQ ID NO: 3 and a light chain variable region sequence that is at least about 95%, 96%, 97%, 98%, 99% or 100% identical to SEQ ID NO: 7.

In one aspect the CD3 binding antigen binding moiety, particularly Fab molecule, comprises a heavy chain variable region comprising the amino acid sequence of SEQ ID NO: 3 and a light chain variable region comprising the amino acid sequence of SEQ ID NO: 7.

In one aspect the CD3 binding antigen binding moiety, particularly Fab molecule, comprises the heavy chain variable region sequence of SEQ ID NO: 3 and the light chain variable region sequence of SEQ ID NO: 7.

In one aspect the CD3 binding antigen binding moiety, particularly Fab molecule, comprises a heavy chain variable region sequence that is at least about 95%, 96%, 97%, 98%, 99% or 100% identical to SEQ ID NO: 39 and a light chain variable region sequence that is at least about 95%, 96%, 97%, 98%, 99% or 100% identical to SEQ ID NO: 40.

In one aspect the CD3 binding antigen binding moiety, particularly Fab molecule, comprises a heavy chain variable region comprising the amino acid sequence of SEQ ID NO: 39 and a light chain variable region comprising the amino acid sequence of SEQ ID NO: 40.

In one aspect the CD3 binding antigen binding moiety, particularly Fab molecule, comprises the heavy chain variable region sequence of SEQ ID NO: 39 and the light chain variable region sequence of SEQ ID NO: 40.

### Target cell antigen binding moiety

The T cell activating bispecific antigen binding molecule of the present disclosure comprises at least one antigen binding moiety, particularly a Fab molecule, which specifically binds to CD19 (target cell antigen). In certain aspects, the T cell activating bispecific antigen binding molecule comprises two antigen binding moieties, particularly Fab molecules, which specifically bind to CD19. In a particular such aspect, each of these antigen binding moieties specifically binds to the same antigenic determinant. In an even more particular aspect, all of these antigen binding moieties are identical, i.e. they comprise the same amino acid sequences including the same amino acid substitutions in the CH1 and CL domain as described herein (if any). In one aspect, the T cell activating bispecific antigen binding molecule comprises an immunoglobulin molecule which specifically binds to CD19. In one aspect the T cell activating bispecific antigen binding molecule comprises not more than two antigen binding moieties, particularly Fab molecules, which specifically bind to CD19.

In particular aspects, the antigen binding moiety(ies) which specficially bind to CD19 is/are a conventional Fab molecule. In such aspects, the antigen binding moiety(ies) which specifically binds an activating T cell antigen is a crossover Fab molecule as described herein, i.e. a Fab molecule wherein the variable domains VH and VL or the constant domains CH1 and CL of the Fab heavy and light chains are exchanged / replaced by each other.

In alternative aspects, the antigen binding moiety(ies)which specficially bind to CD19 is/are a crossover Fab molecule as described herein, i.e. a Fab molecule wherein the variable domains VH and VL or the constant domains CH1 and CL of the Fab heavy and light chains are exchanged / replaced by each other. In such aspects, the antigen binding moiety(ies)which specifically binds an activating T cell antigen is a conventional Fab molecule.

The CD19 binding moiety is able to direct the T cell activating bispecific antigen binding molecule to a target site, for example to a specific type of tumor cell that expresses CD19.

In a particular aspect, the antigen binding moiety, particularly Fab molecule, which specifically binds to CD19 comprises a heavy chain variable region comprising the heavy chain complementarity determining region (CDR) 1 of SEQ ID NO: 14, the heavy chain CDR 2 of SEQ ID NO: 15, and the heavy chain CDR 3 of SEQ ID NO: 16, and a light chain variable region comprising the light chain CDR 1 of SEQ ID NO: 17, the light chain CDR 2 of SEQ ID NO: 18 and the light chain CDR 3 of SEQ ID NO: 19. In a further aspect, the antigen binding moiety, particularly Fab molecule, which specifically binds to CD19 comprises a heavy chain variable region that is at least 95%, 96%, 97%, 98%, or 99% identical to the sequence of SEQ ID NO: 20, and a light chain variable region that is at least 95%, 96%, 97%, 98%, or 99% identical to the sequence of SEQ ID NO: 21. In still a further aspect, the antigen binding moiety, particularly Fab molecule, which specifically binds to CD19 comprises the heavy chain variable region sequence of SEQ ID NO: 20, and the light chain variable region sequence of SEQ ID NO: 21. In a particular aspect, the T cell activating bispecific antigen binding molecule comprises a polypeptide that is at least 95%, 96%, 97%, 98%, or 99% identical to the sequence of SEQ ID NO: 24, a polypeptide that is at least 95%, 96%, 97%, 98%, or 99% identical to the sequence of SEQ ID NO: 30, a polypeptide that is at least 95%, 96%, 97%, 98%, or 99% identical to the sequence of SEQ ID NO: 31, and a polypeptide that is at least 95%, 96%, 97%, 98%, or 99% identical to the sequence of SEQ ID NO: 32. In a further particular aspect, the T cell activating bispecific antigen binding molecule comprises a polypeptide sequence of SEQ ID NO: 24, a polypeptide sequence of SEQ ID NO: 30, a polypeptide sequence of SEQ ID NO: 31 and a polypeptide sequence of SEQ ID NO: 32.

In one aspect, the antigen binding moiety, particularly Fab molecule, which specifically binds to CD19 comprises a heavy chain variable region comprising the heavy chain complementarity determining region (CDR) 1 of SEQ ID NO: 50, the heavy chain CDR 2 of SEQ ID NO: 51, and the heavy chain CDR 3 of SEQ ID NO: 52, and a light chain variable region comprising the light chain CDR 1 of SEQ ID NO: 53, the light chain CDR 2 of SEQ ID NO: 54 and the light chain CDR 3 of SEQ ID NO: 55. In a further aspect, the antigen binding moiety, particularly Fab molecule, which specifically binds to CD19 comprises a heavy chain variable region that is at least 95%, 96%, 97%, 98%, or 99% identical to the sequence of SEQ ID NO: 56, and a light chain variable region that is at least 95%, 96%, 97%, 98%, or 99% identical to the sequence of SEQ ID NO: 57. In still a further aspect, the antigen binding moiety, particularly Fab molecule, which specifically binds to CD19 comprises the heavy chain variable region sequence of SEQ ID NO: 56, and the light chain variable region sequence of SEQ ID NO: 57.

In another aspect, the antigen binding moiety, particularly Fab molecule, which specifically binds to CD19 comprises
(i) a heavy chain variable region comprising the heavy chain complementarity determining region (CDR) 1 of SEQ ID NO: 58, the heavy chain CDR 2 of SEQ ID NO: 59, and the heavy chain CDR 3 of SEQ ID NO: 60, and a light chain variable region comprising the light chain CDR 1 of SEQ ID NO: 61, the light chain CDR 2 of SEQ ID NO: 62 and the light chain CDR 3 of SEQ ID NO: 63;
(ii) a heavy chain variable region comprising the heavy chain complementarity determining region (CDR) 1 of SEQ ID NO: 66, the heavy chain CDR 2 of SEQ ID NO: 67, and the heavy chain CDR 3 of SEQ ID NO: 68, and a light chain variable region comprising the light chain CDR 1 of SEQ ID NO: 69, the light chain CDR 2 of SEQ ID NO: 70 and the light chain CDR 3 of SEQ ID NO: 71;
(iii) a heavy chain variable region comprising the heavy chain complementarity determining region (CDR) 1 of SEQ ID NO: 74, the heavy chain CDR 2 of SEQ ID NO: 75, and the heavy chain CDR 3 of SEQ ID NO: 76, and a light chain variable region comprising the light chain CDR 1 of SEQ ID NO: 77, the light chain CDR 2 of SEQ ID NO: 78 and the light chain CDR 3 of SEQ ID NO: 79;
(iv) a heavy chain variable region comprising the heavy chain complementarity determining region (CDR) 1 of SEQ ID NO: 82, the heavy chain CDR 2 of SEQ ID NO: 83, and the heavy chain CDR 3 of SEQ ID NO: 84, and a light chain variable region comprising the light chain CDR 1 of SEQ ID NO: 85, the light chain CDR 2 of SEQ ID NO: 86 and the light chain CDR 3 of SEQ ID NO: 87;
(v) a heavy chain variable region comprising the heavy chain complementarity determining region (CDR) 1 of SEQ ID NO: 90, the heavy chain CDR 2 of SEQ ID NO: 91, and the heavy chain CDR 3 of SEQ ID NO: 92, and a light chain variable region comprising the light chain CDR 1 of SEQ ID NO: 93, the light chain CDR 2 of SEQ ID NO: 94 and the light chain CDR 3 of SEQ ID NO: 95; or
(vi) a heavy chain variable region comprising the heavy chain complementarity determining region (CDR) 1 of SEQ ID NO: 98, the heavy chain CDR 2 of SEQ ID NO: 99, and the heavy chain CDR 3 of SEQ ID NO: 100, and a light chain variable region comprising the light chain CDR 1 of SEQ ID NO: 101, the light chain CDR 2 of SEQ ID NO: 102 and the light chain CDR 3 of SEQ ID NO: 103.

In a further aspect, the antigen binding moiety, particularly Fab molecule, which specifically binds to CD19 comprises
(i) a heavy chain variable region that is at least 95%, 96%, 97%, 98%, or 99% identical to the sequence of SEQ ID NO: 64, and a light chain variable region that is at least 95%, 96%, 97%, 98%, or 99% identical to the sequence of SEQ ID NO: 65;
(ii) a heavy chain variable region that is at least 95%, 96%, 97%, 98%, or 99% identical to the sequence of SEQ ID NO: 72, and a light chain variable region that is at least 95%, 96%, 97%, 98%, or 99% identical to the sequence of SEQ ID NO: 73;
(iii) a heavy chain variable region that is at least 95%, 96%, 97%, 98%, or 99% identical to the sequence of SEQ ID NO: 80, and a light chain variable region that is at least 95%, 96%, 97%, 98%, or 99% identical to the sequence of SEQ ID NO: 81;
(iv) a heavy chain variable region that is at least 95%, 96%, 97%, 98%, or 99% identical to the sequence of SEQ ID NO: 88, and a light chain variable region that is at least 95%, 96%, 97%, 98%, or 99% identical to the sequence of SEQ ID NO: 89;
(v) a heavy chain variable region that is at least 95%, 96%, 97%, 98%, or 99% identical to the sequence of SEQ ID NO: 96, and a light chain variable region that is at least 95%, 96%, 97%, 98%, or 99% identical to the sequence of SEQ ID NO: 97; or
(vi) a heavy chain variable region that is at least 95%, 96%, 97%, 98%, or 99% identical to the sequence of SEQ ID NO: 104, and a light chain variable region that is at least 95%, 96%, 97%, 98%, or 99% identical to the sequence of SEQ ID NO: 105.

In still a further aspect, the antigen binding moiety, particularly Fab molecule, which specifically binds to CD19 comprises
(i) the heavy chain variable region sequence of SEQ ID NO: 64, and the light chain variable region sequence of SEQ ID NO: 65;
(ii) the heavy chain variable region sequence of SEQ ID NO: 72, and the light chain variable region sequence of SEQ ID NO: 73;
(iii) the heavy chain variable region sequence of SEQ ID NO: 80, and the light chain variable region sequence of SEQ ID NO: 81;
(iv) the heavy chain variable region sequence of SEQ ID NO: 88, and the light chain variable region sequence of SEQ ID NO: 89;
(v) the heavy chain variable region sequence of SEQ ID NO: 96, and the light chain variable region sequence of SEQ ID NO: 97; or
(vi) the heavy chain variable region sequence of SEQ ID NO: 104, and the light chain variable region sequence of SEQ ID NO: 105.

In a particular aspect, the T cell activating bispecific antigen binding molecule comprises a polypeptide that is at least 95%, 96%, 97%, 98%, or 99% identical to the sequence of SEQ ID NO: 24, a polypeptide that is at least 95%, 96%, 97%, 98%, or 99% identical to the sequence of SEQ ID NO: 114, a polypeptide that is at least 95%, 96%, 97%, 98%, or 99% identical to the sequence of SEQ ID NO: 115, and a polypeptide that is at least 95%, 96%, 97%, 98%, or 99% identical to the sequence of SEQ ID NO: 116. In a further particular aspect, the T cell activating bispecific antigen binding molecule comprises a polypeptide sequence of SEQ ID NO: 24, a polypeptide sequence of SEQ ID NO: 114, a polypeptide sequence of SEQ ID NO: 115 and a polypeptide sequence of SEQ ID NO: 116.

### Polynucleotides

The disclosure further provides isolated polynucleotides encoding a T cell activating bispecific antigen binding molecule as described herein or a fragment thereof. In some aspects, said fragment is an antigen binding fragment.

The polynucleotides encoding T cell activating bispecific antigen binding molecules as disclosed herein may be expressed as a single polynucleotide that encodes the entire T cell activating bispecific antigen binding molecule or as multiple (e.g., two or more) polynucleotides that are co-expressed. Polypeptides encoded by polynucleotides that are co-expressed may associate through, e.g., disulfide bonds or other means to form a functional T cell activating bispecific antigen binding molecule. For example, the light chain portion of a Fab molecule may be encoded by a separate polynucleotide from the portion of the T cell activating bispecific antigen binding molecule comprising the heavy chain portion of the Fab molecule, an Fc domain subunit and optionally (part of) another Fab molecule. When co-expressed, the heavy chain polypeptides will associate with the light chain polypeptides to form the Fab molecule. In another example, the portion of the T cell activating bispecific antigen binding molecule comprising one of the two Fc domain subunits and optionally (part of) one or more Fab molecules could be encoded by a separate polynucleotide from the portion of the T cell activating bispecific antigen binding molecule comprising the the other of the two Fc domain subunits and optionally (part of) a Fab molecule. When co-expressed, the Fc domain subunits will associate to form the Fc domain.

In some aspects, the isolated polynucleotide encodes the entire T cell activating bispecific antigen binding molecule as described herein. In other aspects, the isolated polynucleotide encodes a polypeptides comprised in the T cell activating bispecific antigen binding molecule as described herein.

In certain aspects the polynucleotide or nucleic acid is DNA. In other aspects, a polynucleotide is RNA, for example, in the form of messenger RNA (mRNA). RNA may be single stranded or double stranded.

### Recombinant Methods

T cell activating bispecific antigen binding molecules disclosed herein may be obtained, for example, by solid-state peptide synthesis (e.g. Merrifield solid phase synthesis) or recombinant production. For recombinant production one or more polynucleotide encoding the T cell activating bispecific antigen binding molecule (fragment), e.g., as described above, is isolated and inserted into one or more vectors for further cloning and/or expression in a host cell. Such polynucleotide may be readily isolated and sequenced using conventional procedures. **In** one aspect a vector, preferably an expression vector, comprising one or more of the polynucleotides disclosed herein is provided. Methods which are well known to those skilled in the art can be used to construct expression vectors containing the coding sequence of a T cell activating bispecific antigen binding molecule (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the T cell activating bispecific antigen binding molecule (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector as disclosed herein may encode one or more polypeptides, which are post- or co-translationally separated into the final proteins via proteolytic cleavage. **In** addition, a vector, polynucleotide, or nucleic acid as disclosed herein may encode heterologous coding regions, either fused or unfused to a polynucleotide encoding the T cell activating bispecific antigen binding molecule (fragment) disclosed herein, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions as disclosed herein may be associated with additional coding regions which encode secretory or signal peptides, which direct the secretion of a polypeptide encoded by a polynucleotide as disclosed herein. For example, if secretion of the T cell activating bispecific antigen binding molecule is desired, DNA encoding a signal sequence may be placed upstream of the nucleic acid encoding a T cell activating bispecific antigen binding molecule as disclosed herein or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain aspects, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the T cell activating bispecific antigen binding molecule may be included within or at the ends of the T cell activating bispecific antigen binding molecule (fragment) encoding polynucleotide.

In a further aspect, a host cell comprising one or more polynucleotides as disclosed herein is provided. In certain aspects a host cell comprising one or more vectors as disclosed herein is provided. The polynucleotides and vectors may incorporate any of the features, singly or in combination, described herein in relation to polynucleotides and vectors, respectively. In one such aspect a host cell comprises (e.g. has been transformed or transfected with) a vector comprising a polynucleotide that encodes (part of) a T cell activating bispecific antigen binding molecule as disclosed herein. As used herein, the term "host cell" refers to any kind of cellular system which can be engineered to generate the T cell activating bispecific antigen binding molecules disclosed herein or fragments thereof. Host cells suitable for replicating and for supporting expression of T cell activating bispecific antigen binding molecules are well known in the art. Such cells may be transfected or transduced as appropriate with the particular expression vector and large quantities of vector containing cells can be grown for seeding large scale fermenters to obtain sufficient quantities of the T cell activating bispecific antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES^{™} technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one aspect, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one aspect, a method of producing a T cell activating bispecific antigen binding molecule according to the present disclosure is provided, wherein the method comprises culturing a host cell comprising a polynucleotide encoding the T cell activating bispecific antigen binding molecule, as provided herein, under conditions suitable for expression of the T cell activating bispecific antigen binding molecule, and recovering the T cell activating bispecific antigen binding molecule from the host cell (or host cell culture medium).

The components of the T cell activating bispecific antigen binding molecule are genetically fused to each other. T cell activating bispecific antigen binding molecule can be designed such that its components are fused directly to each other or indirectly through a linker sequence. The composition and length of the linker may be determined in accordance with methods well known in the art and may be tested for efficacy. Examples of linker sequences between different components of T cell activating bispecific antigen binding molecules are found in the sequences provided herein. Additional sequences may also be included to incorporate a cleavage site to separate the individual components of the fusion if desired, for example an endopeptidase recognition sequence.

In certain aspects the one or more antigen binding moieties of the T cell activating bispecific antigen binding molecules comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the T cell activating bispecific antigen binding molecules disclosed herein. Nonlimiting antibodies, antibody fragments, antigen binding domains or variable regions useful in the present disclosure can be of murine, primate, or human origin. If the T cell activating bispecific antigen binding molecule is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or α-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the antigen binding moieties useful in the present disclosure are engineered to have enhanced binding affinity according to, for example, the methods disclosed in U.S. Pat. Appl. Publ. No. 2004/0132066. The ability of the T cell activating bispecific antigen binding molecule disclosed herein to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g. an antibody that competes with the V9 antibody for binding to CD3. In certain aspects, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. CD3) is incubated in a solution comprising a first labeled antibody that binds to the antigen (e.g. V9 antibody, described in US 6,054,297) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

T cell activating bispecific antigen binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the T cell activating bispecific antigen binding molecule binds. For example, for affinity chromatography purification of T cell activating bispecific antigen binding molecules as disclosed herein, a matrix with protein A or protein G may be used. Sequential Protein A or G affinity chromatography and size exclusion chromatography can be used to isolate a T cell activating bispecific antigen binding molecule essentially as described in the Examples. The purity of the T cell activating bispecific antigen binding molecule can be determined by any of a variety of well known analytical methods including gel electrophoresis, high pressure liquid chromatography, and the like. For example, the heavy chain fusion proteins expressed as described in the Examples were shown to be intact and properly assembled as demonstrated by reducing SDS-PAGE (see e.g. Figure 3). Three bands were resolved at approximately Mr 25,000, Mr 50,000 and Mr 75,000, corresponding to the predicted molecular weights of the T cell activating bispecific antigen binding molecule light chain, heavy chain and heavy chain/light chain fusion protein.

### Assays

T cell activating bispecific antigen binding molecules provided herein may be identified, screened for, or characterized for their physical/chemical properties and/or biological activities by various assays known in the art.

### Affinity assays

The affinity of the T cell activating bispecific antigen binding molecule for an Fc receptor or a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of T cell activating bispecific antigen binding molecules for different receptors or target antigens may be evaluated using cell lines expressing the particular receptor or target antigen, for example by flow cytometry (FACS). A specific illustrative and exemplary aspect for measuring binding affinity is described in the following and in the Examples below.

According to one aspect, K_{D} is measured by surface plasmon resonance using a BIACORE^{®} T100 machine (GE Healthcare) at 25 °C.

To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions. Anti Penta-His antibody is diluted with 10 mM sodium acetate, pH 5.0, to 40 µg/ml before injection at a flow rate of 5 µl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 µl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 µl/min. The dissociation is monitored for 180 s.

Bulk refractive index differences are corrected for by subtracting the response obtained on reference flow cell. The steady state response was used to derive the dissociation constant K_{D} by non-linear curve fitting of the Langmuir binding isotherm. Association rates (kₒₙ) and dissociation rates (k_{off}) are calculated using a simple one-to-one Langmuir binding model (BIACORE^{®} T100 Evaluation Software version 1.1.1) by simultaneously fitting the association and dissociation sensorgrams. The equilibrium dissociation constant (K_{D}) is calculated as the ratio k_{off}/kₒₙ. See, e.g., Chen et al., J Mol Biol 293, 865-881 (1999).

### Activity assays

Biological activity of the T cell activating bispecific antigen binding molecules disclosed herein can be measured by various assays as described in the Examples. Biological activities may for example include the induction of proliferation of T cells, the induction of signaling in T cells, the induction of expression of activation markers in T cells, the induction of cytokine secretion by T cells, the induction of lysis of target cells such as tumor cells, and the induction of tumor regression and/or the improvement of survival.

### Compositions, Formulations, and Routes of Administration

In a further aspect, the present disclosure provides pharmaceutical compositions comprising any of the T cell activating bispecific antigen binding molecules provided herein, e.g., for use in any of the below therapeutic methods. In one aspect, a pharmaceutical composition comprises any of the T cell activating bispecific antigen binding molecules provided herein and a pharmaceutically acceptable carrier. In another aspect, a pharmaceutical composition comprises any of the T cell activating bispecific antigen binding molecules provided herein and at least one additional therapeutic agent, e.g., as described below.

Further provided is a method of producing a T cell activating bispecific antigen binding molecule as disclosed herein in a form suitable for administration in vivo, the method comprising (a) obtaining a T cell activating bispecific antigen binding molecule according to the present disclosure, and (b) formulating the T cell activating bispecific antigen binding molecule with at least one pharmaceutically acceptable carrier, whereby a preparation of T cell activating bispecific antigen binding molecule is formulated for administration in vivo.

Pharmaceutical compositions of the present disclosure comprise a therapeutically effective amount of one or more T cell activating bispecific antigen binding molecule dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one T cell activating bispecific antigen binding molecule and optionally an additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (*e.g*. antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The composition may comprise different types of carriers depending on whether it is to be administered in solid, liquid or aerosol form, and whether it need to be sterile for such routes of administration as injection. T cell activating bispecific antigen binding molecules of the present disclosure (and any additional therapeutic agent) can be administered intravenously, intradermally, intraarterially, intraperitoneally, intralesionally, intracranially, intraarticularly, intraprostatically, intrasplenically, intrarenally, intrapleurally, intratracheally, intranasally, intravitreally, intravaginally, intrarectally, intratumorally, intramuscularly, intraperitoneally, subcutaneously, subconjunctivally, intravesicularlly, mucosally, intrapericardially, intraumbilically, intraocularally, orally, topically, locally, by inhalation (e.g. aerosol inhalation), injection, infusion, continuous infusion, localized perfusion bathing target cells directly, via a catheter, via a lavage, in cremes, in lipid compositions (e.g. liposomes), or by other method or any combination of the forgoing as would be known to one of ordinary skill in the art (see, for example, Remington's Pharmaceutical Sciences, 18th Ed. Mack Printing Company, 1990). Parenteral administration, in particular intravenous injection, is most commonly used for administering polypeptide molecules such as the T cell activating bispecific antigen binding molecules disclosed herein.

Parenteral compositions include those designed for administration by injection, e.g. subcutaneous, intradermal, intralesional, intravenous, intraarterial intramuscular, intrathecal or intraperitoneal injection. For injection, the T cell activating bispecific antigen binding molecules disclosed herein may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the T cell activating bispecific antigen binding molecules may be in powder form for constitution with a suitable vehicle, e.g., sterile pyrogen-free water, before use. Sterile injectable solutions are prepared by incorporating the T cell activating bispecific antigen binding molecules disclosed herein in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular aspects, prolonged absorption of an injectable composition can be brought about by the use in the compositions of agents delaying absorption, such as, for example, aluminum monostearate, gelatin or combinations thereof.

In addition to the compositions described previously, the T cell activating bispecific antigen binding molecules may also be formulated as a depot preparation. Such long acting formulations may be administered by implantation (for example subcutaneously or intramuscularly) or by intramuscular injection. Thus, for example, the T cell activating bispecific antigen binding molecules may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the T cell activating bispecific antigen binding molecules disclosed herein may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The T cell activating bispecific antigen binding molecules may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

### Therapeutic Methods and Compositions

Any of the T cell activating bispecific antigen binding molecules provided herein may be used in therapeutic methods. T cell activating bispecific antigen binding molecules as disclosed herein can be used as immunotherapeutic agents, for example in the treatment of cancers.

For use in therapeutic methods, T cell activating bispecific antigen binding molecules as disclosed herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, T cell activating bispecific antigen binding molecules as disclosed herein for use as a medicament are provided. In further aspects, T cell activating bispecific antigen binding molecules as disclosed herein for use in treating a disease are provided. In certain aspects, T cell activating bispecific antigen binding molecules as disclosed herein for use in a method of treatment are provided. In one aspect, the disclosure provides a T cell activating bispecific antigen binding molecule as described herein for use in the treatment of a disease in an individual in need thereof. In certain aspects, the disclosure provides a T cell activating bispecific antigen binding molecule for use in a method of treating an individual having a disease comprising administering to the individual a therapeutically effective amount of the T cell activating bispecific antigen binding molecule. In certain aspects the disease to be treated is a proliferative disorder. In a particular aspect the disease is cancer, particularly a B-cell cancer. In certain aspects the method further comprises administering to the individual a therapeutically effective amount of at least one additional therapeutic agent, e.g., an anti-cancer agent if the disease to be treated is cancer. In further aspects, the disclosure provides a T cell activating bispecific antigen binding molecule as described herein for use in inducing lysis of a target cell, particularly a B cell. In certain aspects, the disclosure provides a T cell activating bispecific antigen binding molecule for use in a method of inducing lysis of a target cell, particularly a B cell, in an individual comprising administering to the individual an effective amount of the T cell activating bispecific antigen binding molecule to induce lysis of a target cell. An "individual" according to any of the above aspects is a mammal, preferably a human.

In a further aspect, the disclosure provides for the use of a T cell activating bispecific antigen binding molecule as disclosed herein in the manufacture or preparation of a medicament. In one aspect the medicament is for the treatment of a disease in an individual in need thereof. In a further aspect, the medicament is for use in a method of treating a disease comprising administering to an individual having the disease a therapeutically effective amount of the medicament. In certain aspects the disease to be treated is a proliferative disorder. In a particular aspect the disease is cancer, particularly a B-cell cancer. In one aspect, the method further comprises administering to the individual a therapeutically effective amount of at least one additional therapeutic agent, e.g., an anti-cancer agent if the disease to be treated is cancer. In a further aspect, the medicament is for inducing lysis of a target cell, particularly a B cell. In still a further aspect, the medicament is for use in a method of inducing lysis of a target cell, particularly a B cell, in an individual comprising administering to the individual an effective amount of the medicament to induce lysis of a target cell. An "individual" according to any of the above aspects may be a mammal, preferably a human.

In a further aspect, the disclosure provides a method for treating a disease. In one aspect, the method comprises administering to an individual having such disease a therapeutically effective amount of a T cell activating bispecific antigen binding moleculeas disclosed herein. In one aspect a composition is administered to said invididual, comprising the T cell activating bispecific antigen binding molecule disclosed herein in a pharmaceutically acceptable form. In certain aspects the disease to be treated is a proliferative disorder. In a particular aspect the disease is cancer, particularly a B-cell cancer. In certain aspects the method further comprises administering to the individual a therapeutically effective amount of at least one additional therapeutic agent, e.g., an anti-cancer agent if the disease to be treated is cancer. An "individual" according to any of the above aspects may be a mammal, preferably a human.

In a further aspect, the disclosure provides a method for inducing lysis of a target cell, particularly a B cell. In one aspect the method comprises contacting a target cell with a T cell activating bispecific antigen binding molecule disclosed herein in the presence of a T cell, particularly a cytotoxic T cell. In a further aspect, a method for inducing lysis of a target cell, particularly a B cell, in an individual is provided. In one such aspect, the method comprises administering to the individual an effective amount of a T cell activating bispecific antigen binding molecule to induce lysis of a target cell. In one aspect, an "individual" is a human.

In certain aspects the disease to be treated is a proliferative disorder, particularly cancer. Nonlimiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a T cell activating bispecific antigen binding molecule of the present disclosure include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain aspects the cancer is a B-cell cancer. The B-cell cancer is in one aspect a B-cell lymphoma or a B-cell leukemia. In one aspect the B-cell cancer is non-Hodgkin lymphoma or acute lymphoblastic leukemia or chronic lymphocytic leukemia. A skilled artisan readily recognizes that in many cases the T cell activating bispecific antigen binding molecule may not provide a cure but may only provide partial benefit. In some aspects, a physiological change having some benefit is also considered therapeutically beneficial. Thus, in some aspects, an amount of T cell activating bispecific antigen binding molecule that provides a physiological change is considered an "effective amount" or a "therapeutically effective amount". The subject, patient, or individual in need of treatment is typically a mammal, more specifically a human.

In some aspects, an effective amount of a T cell activating bispecific antigen binding molecule as disclosed herein is administered to a cell. In other aspects, a therapeutically effective amount of a T cell activating bispecific antigen binding molecule as disclosed herein is administered to an individual for the treatment of disease.

For the prevention or treatment of disease, the appropriate dosage of a T cell activating bispecific antigen binding molecule as disclosed herein (when used alone or in combination with one or more other additional therapeutic agents) will depend on the type of disease to be treated, the route of administration, the body weight of the patient, the type of T cell activating bispecific antigen binding molecule, the severity and course of the disease, whether the T cell activating bispecific antigen binding molecule is administered for preventive or therapeutic purposes, previous or concurrent therapeutic interventions, the patient's clinical history and response to the T cell activating bispecific antigen binding molecule, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The T cell activating bispecific antigen binding molecule is suitably administered to the patient at one time or over a series of treatments. Depending on the type and severity of the disease, about 1 µg/kg to 15 mg/kg (e.g. 0.1 mg/kg - 10 mg/kg) of T cell activating bispecific antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the T cell activating bispecific antigen binding molecule would be in the range from about 0.005 mg/kg to about 10 mg/kg. In other nonlimiting examples, a dose may also comprise from about 1 microgram/kg body weight, about 5 microgram/kg body weight, about 10 microgram/kg body weight, about 50 microgram/kg body weight, about 100 microgram/kg body weight, about 200 microgram/kg body weight, about 350 microgram/kg body weight, about 500 microgram/kg body weight, about 1 milligram/kg body weight, about 5 milligram/kg body weight, about 10 milligram/kg body weight, about 50 milligram/kg body weight, about 100 milligram/kg body weight, about 200 milligram/kg body weight, about 350 milligram/kg body weight, about 500 milligram/kg body weight, to about 1000 mg/kg body weight or more per administration, and any range derivable therein. In nonlimiting examples of a derivable range from the numbers listed herein, a range of about 5 mg/kg body weight to about 100 mg/kg body weight, about 5 microgram/kg body weight to about 500 milligram/kg body weight, etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the T cell activating bispecific antigen binding molecule). An initial higher loading dose, followed by one or more lower doses may be administered. However, other dosage regimens may be useful. The progress of this therapy is easily monitored by conventional techniques and assays.

The T cell activating bispecific antigen binding molecules disclosed herein will generally be used in an amount effective to achieve the intended purpose. For use to treat or prevent a disease condition, the T cell activating bispecific antigen binding molecules disclosed herein, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from *in vivo* data, *e.g.,* animal models, using techniques that are well known in the art. One having ordinary skill in the art could readily optimize administration to humans based on animal data.

Dosage amount and interval may be adjusted individually to provide plasma levels of the T cell activating bispecific antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the T cell activating bispecific antigen binding molecules may not be related to plasma concentration. One having skill in the art will be able to optimize therapeutically effective local dosages without undue experimentation.

A therapeutically effective dose of the T cell activating bispecific antigen binding molecules described herein will generally provide therapeutic benefit without causing substantial toxicity. Toxicity and therapeutic efficacy of a T cell activating bispecific antigen binding molecule can be determined by standard pharmaceutical procedures in cell culture or experimental animals. Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀. T cell activating bispecific antigen binding molecules that exhibit large therapeutic indices are preferred. In one aspect, the T cell activating bispecific antigen binding molecule according to the present disclosure exhibits a high therapeutic index. The data obtained from cell culture assays and animal studies can be used in formulating a range of dosages suitable for use in humans. The dosage lies preferably within a range of circulating concentrations that include the ED₅₀ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1).

The attending physician for patients treated with T cell activating bispecific antigen binding molecules as disclosed herein would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other Agents and Treatments

The T cell activating bispecific antigen binding molecules disclosed herein may be administered in combination with one or more other agents in therapy. For instance, a T cell activating bispecific antigen binding molecule as disclosed herein may be co-administered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain aspects, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular aspect, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent. Such other agents are suitably present in combination in amounts that are effective for the purpose intended. The effective amount of such other agents depends on the amount of T cell activating bispecific antigen binding molecule used, the type of disorder or treatment, and other factors discussed above. The T cell activating bispecific antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the T cell activating bispecific antigen binding molecule disclosed herein can occur prior to, simultaneously, and/or following, administration of the additional therapeutic agent and/or adjuvant. T cell activating bispecific antigen binding molecules as disclosed herein can also be used in combination with radiation therapy.

### Articles of Manufacture

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a T cell activating bispecific antigen binding molecule as disclosed herein. The label or package insert indicates that the composition is used for treating the condition of choice. Moreover, the article of manufacture may comprise (a) a first container with a composition contained therein, wherein the composition comprises a T cell activating bispecific antigen binding molecule disclosed herein; and (b) a second container with a composition contained therein, wherein the composition comprises a further cytotoxic or otherwise therapeutic agent. The article of manufacture in this aspect of the disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Examples

The following are examples of methods and compositions of the invention. It is understood that various other aspects may be practiced, given the general description provided above.

### General methods

### Recombinant DNA Techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturers' instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242.

### DNA Sequencing

DNA sequences were determined by double strand sequencing.

### Gene Synthesis

Desired gene segments where required were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Preparation of humanized CD19 antibody

Humanized CD19 binder (var. 5) was prepared as described in PCT publication no. WO 2017/055541. As shown therein, this antibody has an improved stability, particularly deamidation stability, compared to other humanized variants, through introduction of an N (Asn) to Q (Gln) point mutation at position 64 of the heavy chain variable region (numbering according to Kabat) and an S (Ser) to P (Pro) point mutation at position 26e of the light chain variable region (numbering according to Kabat). In this improved humanized anti-human CD19 antibody the human/cynomolgus cross-reactivity of the parental murine antibody (8B8, WO 2011/147834) is retained.

### Preparation of anti-CD19 / anti-CD3 T cell bispecific (TCB) molecules with and without charge modifications

The following molecules were prepared in this example; schematic illustrations thereof are shown in Figure 2:
A. "2+1 IgG CrossFab, inverted" with charge modifications (VH/VL exchange in CD3 binder, charge modification in CD19 binder, parental murine CD19 binder (8B8)) (Figure 2A, SEQ ID NOs 22-25)
B. "2+1 IgG CrossFab, inverted" without charge modifications (CH1/CL exchange in CD3 binder, parental murine CD19 binder (8B8)) (Figure 2B, SEQ ID NOs 26-29)
C. "2+1 IgG CrossFab, inverted" with charge modifications (VH/VL exchange in CD3 binder, charge modification in CD19 binder, humanized CD19 binder (var. 5)) (Figure 2C, SEQ ID NOs 24, 30-32)
D. "2+1 IgG CrossFab, inverted" without charge modifications (CH1/CL exchange in CD3 binder, murine CD19 binder 10C1) (Figure 2D, SEQ ID NOs 28, 41-43)
E. "1+1 IgG CrossFab, inverted" without charge modifications (CH1/CL exchange in CD3 binder, parental murine CD19 binder (8B8)) (Figure 2E, SEQ ID NOs 27-29, 44)
F. "1+1 IgG CrossMab, with charge modifications (VH/VL exchange in CD3 binder, charge modification in CD19 binder, parental murine CD19 binder (8B8)) (Figure 2F, SEQ ID NOs 22, 24, 25, 45)
G. "2+1 IgG CrossFab, inverted" with charge modifications (VH/VL exchange in CD19 binder, charge modification in CD3 binder, parental murine CD19 binder (8B8)) (Figure 2G, SEQ ID NOs 46-49).
H. "2+1 IgG CrossFab, inverted" with charge modifications (VH/VL exchange in CD3 binder, charge modification in CD19 binder, humanized CD19 binder 2B11) (Figure 2H, SEQ ID NOs 24, 114-116)

The variable region of heavy and light chain DNA sequences were subcloned in frame with either the constant heavy chain or the constant light chain pre-inserted into the respective recipient mammalian expression vector. Protein expression is driven by an MPSV promoter and a synthetic polyA signal sequence is present at the 3' end of the CDS. In addition each vector contains an EBV OriP sequence.

The molecules were produced by co-transfecting HEK293-EBNA cells growing in suspension with the mammalian expression vectors using polyethylenimine (PEI). The cells are transfected with the corresponding expression vectors in a 1:2:1:1 ratio (A, C and H: "vector heavy chain (VH-CH1-VL-CH1-CH2-CH3)" : "vector light chain (VL-CL)" : "vector heavy chain (VH-CH1-CH2-CH3)" : "vector light chain (VH-CL)"; B and D: "vector heavy chain (VH-CH1-VH-CL-CH2-CH3)" : "vector light chain (VL-CL)" : "vector heavy chain (VH-CH1-CH2-CH3)" : "vector light chain (VL-CH1)"; G: "vector heavy chain (VL-CH1-VH-CH1-CH2-CH3)" : "vector light chain (VL-CL)" : "vector heavy chain (VL-CH1-CH2-CH3)" : "vector light chain (VH-CL)") or a 1:1:1:1 ratio (E: "vector heavy chain (VH-CH1-VH-CL-CH2-CH3)" : "vector light chain (VL-CL)" : "vector heavy chain (CH2-CH3)" : "vector light chain (VL-CH1)"; F: "vector heavy chain (VL-CH1-CH2-CH3)" : "vector light chain (VH-CL)" : "vector heavy chain (VH-CH1-CH2-CH3)" : "vector light chain (VL-CL)").

For transfection, HEK293 EBNA cells were cultivated in suspension serum free in Excell culture medium containing 6 mM L-glutamine and 250 mg/l G418. For the production in 600 ml tubespin flasks (max. working volume 400 ml) 600 million HEK293 EBNA cells were seeded 24 hours before transfection. For transfection, cells were centrifuged for 5 min at 210 x g, and supernatant was replaced by 20 ml pre-warmed CD CHO medium. Expression vectors are mixed in 20 ml CD CHO medium to a final amount of 400 µg DNA. After addition of 1080 µl PEI solution (2.7 µg/ml) the mixture was vortexed for 15 s and subsequently incubated for 10 min at room temperature. Afterwards cells were mixed with the DNA/PEI solution, transferred to a 600 ml tubespin flask and incubated for 3 hours at 37°C in an incubator with a 5% CO₂ atmosphere. After incubation, 360 ml Excell + 6 mM L-glutamine + 5 g/L Pepsoy + 1.0 mM VPA medium was added and cells were cultivated for 24 hours. One day after transfection 7% Feed 1 was added. After 7 days cultivation supernatant was collected for purification by centrifugation for 20 - 30 min at 3600 x g (Sigma 8K centrifuge), the solution was sterile filtered (0.22 µm filter) and sodium azide in a final concentration of 0.01% w/v was added. The solution was kept at 4°C. The concentration of the molecules in the culture medium was determined by ProteinA-HPLC. The basis of separation was binding of Fc-containing molecules on ProteinA at pH 8.0 and step elution from pH 2.5. There were two mobile phases. These were Tris (10 mM) - glycine (50 mM) - NaCl (100 mM) buffers, identical except that they were adjusted to different pHs (8 and 2.5). The column body was an Upchurch 2x20 mm pre-column with an internal volume of ~63 µl packed with POROS 20A. 100 µl of each sample was injected on equilibrated material with a flow rate of 0.5 ml/min. After 0.67 minutes the sample was eluted with a pH step to pH 2.5. Quantitation was done by determination of 280 nm absorbance and calculation using a standard curve with a concentration range of human IgG1 from 16 to 166 mg/l.

The secreted protein was purified from cell culture supernatants by affinity chromatography using Protein A affinity chromatography, followed by a size exclusion chromatographic step.

For affinity chromatography supernatant was loaded on a HiTrap ProteinA HP column (CV=5 mL, GE Healthcare) equilibrated with 25 ml 20 mM sodium phosphate, 20 mM sodium citrate, pH 7.5. Unbound protein was removed by washing with at least 10 column volumes 20 mM sodium phosphate, 20 mM sodium citrate, pH 7.5 and target protein was eluted in 6 column volumes 20 mM sodium citrate, 100 mM sodium chloride, 100 mM glycine, pH 3.0. Protein solution was neutralized by adding 1/10 of 0.5M sodium phosphate, pH8.0. Target protein was concentrated and filtrated prior loading on a HiLoad Superdex 200 column (GE Healthcare) equilibrated with 20 mM histidine, 140 mM sodium chloride, 0.01% Tween20, pH 6.0.

For in-process analytics after ProteinA chromatography the purity and molecular weight of the molecules in the single fractions were analyzed by SDS-PAGE in the absence of a reducing agent and staining with Coomassie (InstantBlue^{™}, Expedeon). The NuPAGE^{®} Pre-Cast gel system (4-12% Bis-Tris, Invitrogen) was used according to the manufacturer's instruction.

The protein concentration of purified protein samples was determined by measuring the optical density (OD) at 280 nm, using the molar extinction coefficient calculated on the basis of the amino acid sequence.

Final purity and molecular weight of molecules were analyzed by CE-SDS analyses in the presence and absence of a reducing agent. The Caliper LabChip GXII system (Caliper Lifescience) was used according to the manufacturer's instruction.

The aggregate content of molecules was analyzed using a TSKgel G3000 SW XL analytical size-exclusion column (Tosoh) in 25 mM K₂HPO₄, 125 mM NaCl, 200 mM L-arginine monohydrocloride, 0.02% (w/v) NaN₃, pH 6.7 running buffer at 25°C.

All molecules were produced and purified following the same method.

For molecules A-C and H the final recovery was highest for the three "2+1 IgG CrossFab, inverted" molecules with charge modifications (molecule A, C and H) and the final quality was very good with more than 99% monomer content (molecule C and H were tailing) and more than 96% purity on CE-SDS (Table 1 and 2, Figure 3A, 3C and 3H). Molecule B does not contain charge modifications in the CD19 Fabs. This has an impact on the final quality shown by CE-SDS and analytical SEC (Table 1 and 2, Figure 3B). The difference in quality is mostly visible after the first purification step on SDS-PAGE (Figure 4A, 4B, 4C, 4H). Molecule B contains more side products at 150 kDa and 70 kDa (half molecules and constructs probably missing light chains) than molecule A, C and H. For molecule A, C and H the major fraction within this preparation is the target molecule and the low molecular weight impurities can be removed by the final purification step (Table 1 and 2, Figure 3A, 3C, 3H).

Molecules E, F and G could be prepared in good quality (>98% monomer content) and >93% purity on CE-SDS (Table 1 and 2, Figure 3 E, F and G). Molecule D has a slightly less good quality with around 96% monomer content and 70% purity on CE-SDS (Table 1 and 2, Figure 3D).

**TABLE 1. Summary of production and purification of anti-CD19 / anti-CD3 TCB molecules with and without charge modifications.**

| Molecule | Titer [mg/l] | Recovery [%] | Yield [mg/l] | Analytical SEC (HMW/Monomer/LMW) [%] |
|---|---|---|---|---|
| A | 13.8 | 28 | 3.93 | 0/**100**/0 |
| B | 30.4 | 9 | 2.77 | 1/**97.3**/1.7 |
| C | 123 | 21 | 25.8 | 0.5/**99.5**/ tailing |
| D | 23 | 17 | 4 | 0/**95.6**/tailing |
| E | 33 | 10 | 3 | 1.4/**98.6**/0 |
| F | 39 | 18 | 7 | 0/**100**/0 |
| G | 25 | 25 | 6 | 0/**100**/0 |
| H | 64 | 38 | 24 | 0/**100**/ tailing |

**TABLE 2. CE-SDS analyses (non-reduced) of anti-CD19 / anti-CD3 TCB molecules with and without charge modifications.**

| Molecule | Peak # | Size [kDa] | Purity [%] |
|---|---|---|---|
| A | 1 | 220.9 | 100 |
| B | 1 | 94.6 | 2 |
| | 2 | 183.2 | 4.2 |
| | 3 | 205 | 5.2 |
| | 4 | 212.2 | 6.7 |
| | 5 | 221 | 81.9 |
| C | 1 | 172 | 3.2 |
| | 2 | 206 | 96.8 |
| D | 1 | 183 | 3 |
| | 2 | 207 | 27 |
| | 3 | 219 | 70 |
| E | 1 | 66 | 4 |
| | 2 | 152 | 2 |
| | 3 | 176 | 94 |
| F | 1 | 68 | 3 |
| | 2 | 177 | 97 |
| G | 1 | 171 | 3 |
| | 2 | 197 | 4 |
| | 3 | 216 | 93 |
| H | 1 | 167 | 2 |
| | 2 | 196 | 98 |

In the following five examples, molecule B is referred to as "CD19 TCB_8B8". A TCB of identical structure but based on the CD19 binder 10C1 (VH of SEQ ID NO: 33, VL of SEQ ID NO: 34) is also tested (molecule D, referred to in the following five examples as "CD19 TCB_10C1"). "DP47 TCB" refers to an untargeted TCB having an identical structure as molecule B but instead of a CD19 binder having the DP47 binder (VH of SEQ ID NO: 35, VL of SEQ ID NO: 36) that does not bind any known protein (used as negative control).

### Binding of CD19 IgGs and CD19 TCBs to human CD19- and CD3-expressing target cells

The binding of CD19 IgGs and CD19 TCBs to human CD19- and CD3-expressing target cells was tested using CD19-expressing Burkitt's lymphoma cells (Raji) and CD3-expressing immortalized T lymphocyte line (Jurkat). Briefly, cells were harvested, counted, checked for viability and resuspended at 2x10⁶ cells/ml in FACS buffer (100 µl PBS 0.1% BSA). 100 µl of cell suspension (containing 0.2x10⁶ cells) were incubated in round-bottom 96-well plate for 30 min at 4°C with increasing concentrations of the CD19 IgGs and CD19 TCBs (10 pM - 200 nM), washed twice with cold PBS 0.1% BSA, re-incubated for further 30 min at 4°C with the PE-conjugated AffiniPure F(ab')2 Fragment goat anti-human IgG Fcg Fragment Specific secondary antibody (Jackson Immuno Research Lab PE #109-116-170), washed twice with cold PBS 0.1% BSA and immediately analyzed by FACS using a FACS CantoII (Software FACS Diva) by gating live, DAPI-negative, cells.

The results are shown in Figure 5. Figure 5A shows binding of CD19 IgGs and CD19 TCBs to Raji cells. CD19 clones (8B8 and 10C1 as IgGs and TCBs) display comparable binding to CD19-expressing cells (EC50 values of binding to CD19-expressing cells are in the 3 - 8 nM range). Binding curves and the EC50 values related to binding were calculated using GraphPadPrism5 and are given in Table 3. Figure 5B shows binding of CD19 TCBs (based on 8B8 and 10C1 clones) to CD3-expressing Jurkat cells. Both clones display comparable binding to Jurkat cells. The EC50 values could not be calculated since the binding curve did not reach saturation.

**TABLE 3. EC50 values (nM) of binding of CD19 IgGs and CD19 TCBs to human CD19-expressing target Raji cells.**

| **Antibodies** | **EC50 [nM]** | **EC50 [ng/ml]** |
|---|---|---|
| CD19_IgG_8B8 | 3.1 | 455 |
| CD19_TCB_8B8 | 4.1 | 795 |
| CD19_IgG_10C1 | 5.2 | 763 |
| CD19_TCB_10C1 | 7.7 | 1493 |

### Lysis of CD19-expressing tumor cells and subsequent T cell activation mediated by CD19 TCB antibodies

The lysis of CD19-expressing tumor cells and subsequent T cell activation mediated by CD19 TCB antibodies was assessed on Nalm-6 (B cell precursor leukemia ALL, 70000 CD19 binding sites), Z-138 cells (mantle cell lymphoma, 10000 CD19 binding sites) and SUDHL-4 (B-NHL, 40000 CD19 binding sites). In the assay with Z-138 cells, blinatumomab was added as an additional CD19 TCB antibody for comparison. Human PBMCs were used as effectors and tumor lysis was detected at 18-21 h of incubation with the different bispecific antibodies. Briefly, target cells were harvested, washed, and plated at density of 30 000 cells/well using round-bottom 96-well plates. Peripheral blood mononuclear cells (PBMCs) were prepared by Histopaque density centrifugation of fresh blood obtained from healthy human donors. Fresh blood was diluted with sterile PBS and layered over Histopaque gradient (Sigma, #10771). After centrifugation (450 x g, 30 minutes, room temperature, no brake), the plasma above the PBMC-containing interphase was discarded and PBMCs transferred in a new falcon tube subsequently filled with 50 ml PBS. The mixture was centrifuged (350 x g, 10 minutes, room temperature), the supernatant discarded and the PBMC pellet washed twice with sterile PBS (centrifugation steps 300 x g, 10 minutes). The resulting PBMC population was counted automatically (ViCell) and re-suspended in RPMI1640 medium containing 2% FCS and 1% L-alanyl-L-glutamine (Biochrom, K0302) at 6 x 10⁶ cells/ml. For the tumor lysis assay, the antibodies were added at the indicated concentrations (range of 0.005 pM - 250 pM in triplicates). PBMCs were added to target cells at final effector-to-target (E:T) ratio of 10:1. Tumor cell lysis was assessed after 20-24 h of incubation at 37°C, 5% CO₂ by quantification of LDH released into cell supernatants by apoptotic/necrotic cells (LDH detection kit, Roche Applied Science, #11 644 793 001). Maximal lysis of the target cells (= 100%) was achieved by incubation of target cells with 1% Triton X-100. Minimal lysis (= 0%) refers to target cells co-incubated with effector cells without bispecific construct. For the assessment of T cell activation occurring upon tumor cell lysis, cells were centrifuged at 400 x g for 4 min and washed twice with PBS containing 0.1% BSA. Surface staining for CD8 (FITC anti-human CD8 Biolegend # 344704 or APCCy7, anti-human CD8 Biolegend # 301016), CD4 (BV421 anti-human CD4, Biolegend # 300532 or APC anti-human CD4, BD # 555349), CD25 (APC anti-human CD25 Biolegend # 356110 or PECy7 anti-human CD25 Biolegend # 302612) and CD69 (PE anti-human CD69, Biolegend # 310906 or BV421 anti-human CD69, Biolegend # 310930) was performed according to the suppliers' indications. Cells were washed twice with 150 µl/well PBS containing 0.1% BSA and fixed for 20 min at 4°C using 100 µl/well fixation buffer (BD # 554655). After centrifugation, the samples were resuspended in 100 µl/well PBS 0.1% BSA. Samples were analyzed at BD FACS CantoII. Results are shown in Figure 6. Figure 6 A-B show that CD19 TCB antibodies (based on 8B8 and 10C1 clone) induced a strong and target-specific killing of CD19+ target cells. CD19 TCB clones 8B8 and 10C1 were overall comparable in inducing lysis of CD19-expressing tumor cells with the clone 8B8 being slightly superior to clone 10C1, as outlined by the slightly lower EC50 values (calculated using GraphPadPrism5) related to tumor killing obtained with CD19 TCB 8B8 clone (Table 4). Figure 6 C-F and 6 G-J show that the two CD19 TCB antibodies were also comparable in inducing T cell activation after tumor killing (Figure 6 C-F, CD69 and CD25 expression on CD8 and CD4 T cells upon killing of Nalm-6 target cells; Figure 6 G-J, CD69 and CD25 expression on CD8 and CD4 T cells upon killing of SUDHL-4 target cells), with the clone 8B8 being slightly superior to clone 10C1, as outlined by the slightly lower EC50 values related to T cell activation (calculated using GraphPadPrism5) (Table 5). Figure 6K shows lysis of Z-138 cells mediated by the two CD19 TCB antibodies (based on 8B8 and 10C1 clone) and blinatumomab. CD19 TCB clone 8B8 was slightly superior to clone 10C1 but comparable to blinatumomab, as outlined by the EC50 values (calculated using GraphPadPrism5) related to tumor killing obtained with CD19 TCB 8B8 clone (Table 6). Figure 6 L-M and 6 N-O show that the two CD19 TCB antibodies were also comparable in inducing T cell activation after tumor killing (Figure 6 L-M, CD69 and CD25 expression on CD8 T cells, Figure 6 N-O, CD69 and CD25 expression on CD4 T cells), with the clone 8B8 being slightly superior to clone 10C1 but comparable to blinatumomab, as outlined by the EC50 values related to T cell activation (calculated using GraphPadPrism5) (Table 7).

**TABLE 4. EC50 values (pM) of tumor cell lysis mediated by CD19 TCB antibodies based on 8B8 and 10C1 clone) evaluated on CD19-expressing tumor target cells.**

| Target cell | Tumor lysis EC50 (pM) clone 8B8 | Tumor lysis EC50 (pM) clone 10C1 |
|---|---|---|
| Nalm-6 | 1.7 | 2.1 |
| SUDHL-4 | 0.8 | 3.4 |

**TABLE 5. EC50 values (pM) of T cell activation upon tumor cell lysis mediated by CD19 TCB (clones 8B8 and 10C1) using CD19-expressing tumor target cells (Nalm-6 and SUDHL-4).**

| Activation marker, Target cell | EC50 (pM) clone 8B8 | EC50 (pM) clone 10C1 |
|---|---|---|
| CD25/CD4, Nalm-6 | 3.3 | 11.8 |
| CD25/CD8, Nalm-6 | 0.7 | 3.0 |
| CD69/CD4, Nalm-6 | 0.9 | 5.6 |
| CD69/CD8, Nalm-6 | 0.8 | 3.8 |
| CD25/CD4, SUDHL-4 | 3.9 | 9.7 |
| CD25/CD8, SUDHL-4 | 2.1 | 7.2 |
| CD69/CD4, SUDHL-4 | 2.0 | 5.2 |
| CD69/CD8, SUDHL-4 | 2.7 | 10.5 |

**TABLE 6. EC50 values (pM) of tumor cell lysis mediated by CD19 TCB antibodies (based on 8B8 and 10C1 clone) and blinatumomab evaluated on CD19-expressing Z-138 tumor target cells.**

| Tumor cell lysis Z-138 cells (21 h) | EC50 (pM) | EC50 (ng/ml) |
|---|---|---|
| CD19 TCB_clone 8B8 | 4.1 | 0.8 |
| CD19 TCB_clone 10C1 | 11.1 | 2.2 |
| Blinatumomab | 4.8 | 0.26 |

**TABLE 7. EC50 values (pM) of T cell activation upon Z-138 tumor cell lysis mediated by CD19 TCB (clones 8B8 and 10C1) and blinatumomab.**

| Activation marker | EC50 (pM) clone 8B8 | EC50 (pM) clone 10C1 | EC50 (pM) blinatumomab |
|---|---|---|---|
| CD69/CD8 | 2.7 | 5.7 | 2.4 |
| CD25/CD8 | 2.4 | 5.9 | 4.5 |
| CD69/CD4 | 1.8 | 4.5 | 2.3 |
| CD25/CD4 | 2.4 | 6.7 | 3.7 |

### B cell depletion and subsequent T cell activation in human whole blood mediated by CD19 TCB antibodies (based on 8B8 and 10C1 clone)

Normal B cell depletion mediated by the two CD19 TCB antibodies (based on 8B8 and 10C1 clone) and subsequent T cell activation was also assessed using fresh heparinized human blood from healthy volunteers. Briefly, fresh blood was collected in heparin-containing syringes. Blood aliquots (190 µL/well) were placed in 96-deep well plates, supplemented with TCB dilutions (10 µL/well) and incubated for 20-24 h at 37°C in 5% CO₂ in a humidified cell incubator. After incubation, blood was mixed by pipetting up and down before 35 µL/well blood aliquots were transferred in 96-round-bottom plates and incubated with fluorescent anti-CD45 (Anti-human CD45 PerCPCy5.5, Biolegend # 300506), anti-CD4 (Anti-human CD4 FITC, Biolegend # 300506), anti-CD8 (Anti-human CD8 APCCy7, Biolegend # 301016), anti-CD22 (Anti-human CD22 APC, Biolegend # 302510), anti-CD25 (Anti-human CD25 PECy7, Biolegend # 302612), anti-CD14 (Anti-human CD14 PE, Biolegend # 325606) and anti-CD69 (Anti-human CD69 BV421, Biolegend # 310930) in total 55 µL volume for flow cytometry. After 15 min incubation at room temperature (in the dark) 180 µL/well of FACS lysis solution (BD Biosciences) was added to deplete erythrocytes and to fix cells prior to flow cytometry. Figure 7A shows that the CD19 TCB antibody based on 8B8 clone was slightly superior in mediating B cell depletion than the CD19 TCB antibody based on 10C1 clone. The same is highlighted by Figure 7B, which shows T cell activation (CD69 marker) after B cell depletion.

### Binding of CD19 TCB antibodies (based on 8B8 and 10C1 clone) to human and cynomolgus monkey B and T cells

The crossreactivity of CD19 TCB antibodies (based on 8B8 and 10C1 clone) was evaluated by assessing binding to human and cynomolgus monkey CD19-expressing B cells and CD3-expressing CD4 and CD8 T cells. Briefly, PBMCs from a healthy cynomolgus monkey as well as a healthy human donor were isolated from fresh blood. Fresh human blood was diluted with sterile PBS (2:1) and layered over Histopaque gradient (Sigma, #H8889). After centrifugation (450 x g, 30 minutes, room temperature, no brake), the plasma above the PBMC-containing interphase was discarded and PBMCs transferred in a new falcon tube subsequently filled with 50 ml of PBS. The mixture was centrifuged (350 x g, 10 minutes, room temperature). Erythrocytes were lysed using 5 ml ACK lysis buffer (0.155 M NH₄Cl + 10 mM KHCO₃ + 0.1 mM EDTA pH 7.3 in ddH₂O, incubation: 5 min, room temperature). 10 ml cell culture medium was added before centrifugation and the PBMC pellet washed twice with sterile PBS (centrifugation steps 300 x g, 10 minutes). Fresh cynomolgus monkey blood was diluted with sterile PBS (1:1) and layered over Histopaque gradient (Sigma, #10771). After centrifugation (520 x g, 30 minutes, room temperature, no brake), the broad band of PBMCs was transferred in a new falcon tube subsequently filled with 50 ml of PBS. The mixture was centrifuged (400 x g, 10 minutes, 4°C), the supernatant discarded and the PBMC pellet washed with sterile PBS (centrifugation 150 x g, 15 minutes). Erythrocytes were lysed using 1 ml ACK lysis buffer (0.155 M NH₄Cl + 10 mM KHCO₃ + 0.1 mM EDTA pH 7.3 in ddH₂O, incubation: 5 min, room temperature). 10 ml cell culture medium was added before centrifugation (300 x g, 10 min, RT). The resulting human and cynomolgus monkey PBMC populations were counted automatically (ViCell). The PBMCs were resuspended at 4x10⁶ cells/ml in FACS buffer (PBS + 0.1% BSA). 100 µl of cell suspension (containing 0.4x10⁶ cells) were seeded into round-bottom 96-well plates and centrifuged (420xg, 4 min). The supernatants were discarded and the cells incubated for 30 min at 4°C with increasing concentrations of the CD19 TCB (0.05 pM - 200 nM), washed twice with cold PBS 0.1% BSA, re-incubated for further 30 min at 4°C with PE-conjugated AffiniPure F(ab')2 Fragment goat anti-human IgG Fcg Fragment Specific secondary antibody (Jackson Immuno Research Lab PE #109-116-170) and anti-hu/cyno CD4 PerCPCy5.5 (BD # 552838), anti-hu/cyno CD8 APCCy7 (Biolegend # 328824), anti-hu/cyno CD20 PECy7 (BD # 560735), washed twice with cold PBS 0.1% BSA and fixed with BD FACS Lysing solution (BD # 349202) before the cells were analyzed by FACS using a FACS CantoII (Software FACS Diva). Binding curves were obtained using GraphPadPrism5.

Figure 8A and 8B show binding of CD19 TCB antibodies (based on 8B8 and 10C1 clone) to cynomolgus monkey and human B cells, Figure 8C and 8D show binding to cynomolgus monkey and human CD4 T cells, and Figure 8E and 8F show binding to cynomolgus monkey and human CD8 T cells.

### B cell depletion and subsequent T cell activation in human and cynomolgus monkey whole blood mediated by CD19 TCB antibodies (based on 8B8 clone) in comparison to blinatumomab

The functional activity and cross-reactivity of the CD19 TCB antibody (8B8 clone) was further assessed by detecting normal B cell depletion and subsequent T cell activation in freshly-isolated human and cynomolgus monkey whole blood from healthy volunteers. In addition, the activity of CD19 TCB (8B8 clone) was compared to blinatumomab in the same assays. Briefly, fresh human blood was collected in heparin-containing syringes and cynomolgus monkey blood was collected in Li-Heparin vacutainers. Blood aliquots (180 µL/well) were placed in 96-deep well plates, supplemented with TCB dilutions (20 µL/well) and incubated for 20 h at 37°C in 5% CO₂ in a humidified cell incubator. After incubation, blood was mixed by pipetting up and down before 35 µL blood aliquots were transferred in 96-U-bottom plates and incubated with fluorescent anti-hu CD45 APC (BD #561290) or anti-cyno CD45 APC (Biolegend #304037), anti-hu/cyno CD4 PerCPCy5.5 (BD # 552838), anti-hu/cyno CD8 APCCy7 (BD #557760), anti-hu/cyno CD20 PECy7 (BD #560735), anti-hu/cyno CD25 PE (BD #557138) and anti-hu/cyno CD69 BV421 (BD #562883) in total 55 µL volume for flow cytometry. After 15 min incubation at room temperature (in the dark) 200 µL/well of FACS lysis solution (BD #349202) was added to deplete erythrocytes and to fix cells prior to flow cytometry.

Figure 9 A-B and 9 C-D show the B cell depletion in cynomolgus monkey (left panel) and human (right panel) whole blood mediated by CD19 TCB antibody (8B8 clone) and blinatumomab. Figure 9 A-B show the results of the % of B cell depletion obtained by normalizing the B cell values to CD4 T cells, Figure 9 C-D show the results of the % of B cell depletion obtained by normalizing the B cell values to CD8 T cells. Figure 9 A-B and 9 C-D show that CD19 TCB antibody (8B8 clone) is human/cynomolgus monkey cross-reactive and displays comparable B cell depletion in human and cynomolgus monkey blood. In addition, CD19 TCB antibody (8B8 clone) displays comparable activity to blinatumomab in human whole blood. Blinatumomab is not cynomolgus monkey cross-reactive and thus it does not mediate B cell depletion in cynomolgus monkey. Figure 9 E-L show T cell activation occurring upon CD19 TCB (8B8 clone) and blinatumomab B cell depletion in cynomolgus monkey (Figure 9 A, C, E, G, I, K) and human (Figure 9 B, D, F, H, J, L) whole blood.

### Comparison of tumor cell lysis and subsequent T cell activation mediated by different CD19 TCB antibody formats

The comparison of tumor cell lysis and subsequent T cell activation mediated by different CD19 TCB antibody formats was assessed on Nalm-6 (B cell precursor leukemia ALL, 70000 CD19 binding sites) and Z-138 cells (mantle cell lymphoma, 10000 CD19 binding sites). The following CD19 TCB antibody formats (all based on the 8B8 clone) were tested: molecule B as shown in Figure 2B (named in this example "CD19 TCB_2+1_no charges_inverted"), molecule E as shown in Figure 2E (named in this example "CD19 TCB_1+1head to tail_no charges_inverted") and molecule F as shown in Figure 2F (named in this example "CD19 TCB_1+1_charges"). Corresponding untargeted TCB formats (i.e. TCBs containing the DP47 binder that does not target any known protein) were used as negative controls. Human isolated pan T cells were used as effectors and tumor lysis was detected at 21 h of incubation with the different bispecific antibody formats. Briefly, target cells were harvested, washed, and plated at density of 30 000 cells/well using round-bottom 96-well plates. Peripheral blood mononuclear cells (PBMCs) were prepared by Histopaque density centrifugation of fresh heparinized blood obtained from healthy human donors. Fresh blood was diluted with sterile PBS (2:1) and layered over Histopaque gradient (Sigma, #10771). After centrifugation (450 x g, 30 minutes, room temperature, no brake), the plasma above the PBMC-containing interphase was discarded and PBMCs transferred in a new falcon tube subsequently filled with 50 ml of PBS. The mixture was centrifuged (350 x g, 10 minutes, room temperature), the supernatant discarded and the PBMC pellet washed twice with sterile PBS (centrifugation steps 300 x g, 10 minutes). The resulting PBMC population was counted automatically (ViCell). T cells were isolated using the pan T cell isolation Kit II from Miltenyi Biotec (#130-091-156) following the instructions of the kit manual. For the tumor lysis assay, the antibodies were added at the indicated concentrations (range of 0.1 pM - 1 nM in triplicates) to the seeded target cells. Furthermore, anti-human CD107a PE (Biolegend #328608) was added into the cell culture. Pan T cells were added to target cells at final E:T ratio of 5:1. Tumor cell lysis was assessed after 21 h of incubation at 37°C, 5% CO₂ by quantification of LDH released into cell supernatants by apoptotic/necrotic cells (LDH detection kit, Roche Applied Science, #11 644 793 001). Maximal lysis of the target cells (= 100%) was achieved by incubation of target cells with 1% Triton X-100. Minimal lysis (= 0%) refers to target cells co-incubated with effector cells without bispecific construct. For the assessment of T cell activation occurring upon tumor cell lysis, cells were centrifuged at 350 x g for 5 min after collecting the supernatants for LDH release and washed twice with PBS containing 0.1% BSA. Surface staining for CD8 (APCCy7 anti-human CD8 Biolegend # 301016), CD4 (FITC anti-human CD4, Biolegend # 300506), CD69 (BV421 anti-human CD69, Biolegend # 310930), and CD25 (PECy7 anti-human CD25 Biolegend # 302612) was performed. Cells were washed twice with 150 µl/well PBS containing 0.1% BSA and fixed using 2% PFA in PBS 0.1% BSA. Samples were analyzed at BD FACS CantoII.

Figure 10A and 10B show that "CD19 TCB_2+1_no charges_inverted" displays a comparable (Z-138 cells, Figure 10B) or slightly superior lysis (Nalm-6, Figure 10A) of tumor cells than "CD19 TCB_1+1_head to tail_no charges_inverted". Both formats are significantly superior to the "CD19 TCB _1+_charges" on both tumor target cell lines. As expected, no lysis of tumor cells was observed with untargeted TCB controls. Figure 10 C-N show that the two CD19 TCB antibody formats ("CD19 TCB_2+1_no charges_inverted" and "CD19 TCB_1+1_head to tail_no charges_inverted") were also comparable in inducing T cell activation after tumor killing. More importantly, both formats were significantly better than the "CD19 TCB_1+1_charges.

**TABLE 8. EC50 values (pM) of Z-138 tumor cell lysis mediated by different CD19 TCB antibody formats.**

| Tumor cell lysis Z-138 cells (21 h) | EC50 (pM) | EC50 (ng/ml) |
|---|---|---|
| "CD19 TCB_2+1_no_charges_inverted" | 3.2 | 0.62 |
| "CD19 TCB_1+1_head totail_no charges_inverted" | 4.2 | 0.62 |
| "CD19 TCB_1+1_charges" | 53.7 | 7.85 |

**TABLE 9. EC50 values (pM) of T cell activation upon tumor cell lysis mediated by different CD19 TCB antibody formats upon lysis of Z-138 cells.**

| Activation marker, Z-138 cells | "CD19 TCB_2+1_no charges_inverted | "CD19 TCB_1+1_head to tail_no charges_inverted" | "CD19 TCB_1+1_charges" |
|---|---|---|---|
| CD107/CD8 | 1.32 | 2.76 | 4.02 |
| CD25/CD8 | 1.86 | 3.31 | 6.50 |
| CD69/CD8 | 0.83 | 1.71 | 2.59 |
| CD107/CD4 | 1.68 | 2.09 | 7.54 |
| CD25/CD4 | 3.22 | 3.55 | 10.02 |
| CD69/CD4 | 1.01 | 1.04 | 3.97 |

### Binding of different CD19 TCB antibody formats (based on 8B8 clone) to human CD19- and CD3-expressing target cells

The binding of different CD19 TCB antibody formats (based on 8B8 clone) was assessed on human CD19-expressing target cells (Nalm-6 and normal human B cells) and human CD3-expressing target cells (CD4, CD8 T cells and Jurkat cells). The following CD19 TCB antibody formats were tested: molecule B as shown in Figure 2B (named in this example "CD19 TCB_2+1_no charges_inverted") and molecule A as shown in Figure 2A (named in this example "CD19 TCB_2+1_charges CD19_inverted"). The untargeted TCB (i.e. TCB having the same format as molecule B but containing the DP47 binder that does not target any known protein) was used as negative control. Briefly, cells were harvested, counted, checked for viability and resuspended at 1.5x10⁶ cells/ml in FACS buffer (100 µl PBS 0.1% BSA). 100 µl of cell suspension (containing 0.15x10⁶ cells) were incubated in round-bottom 96-well plates for 30 min at 4°C with increasing concentrations of the CD19 TCBs (50 pM - 200 nM), washed twice with cold PBS 0.1% BSA, re-incubated for further 30 min at 4°C with the PE-conjugated AffiniPure F(ab')2 Fragment goat anti-human IgG Fcg Fragment Specific secondary antibody (Jackson Immuno Research Lab PE #109-116-170), washed twice with cold PBS 0.1% BSA and immediately analyzed by FACS using a FACS CantoII (Software FACS Diva).

Figure 11 shows the binding of different CD19 TCB antibody formats "CD19 TCB_2+1_no charges_inverted" and "CD19 TCB_2+1_charges CD19_inverted" to (A) Nalm-6 cells, (B) normal human B cells, (C) normal human CD4 T cells, (D) normal human CD8 T cells, and (E) Jurkat cells. Both CD19 TCB antibody formats displayed a comparable binding to both CD19 and CD3-expressing target cells. The EC50 values of binding could not be calculated since the binding curve did not reach saturation. The untargeted TCB was used as negative control (it does not bind to CD19-expressing target cells, but it does bind to CD3-expressing target cells since it contains the same binder included in the CD19 TCB antibody formats, Figure 11 A-E).

### Comparison of tumor cell lysis and subsequent T cell activation mediated by different CD19 TCB antibody formats

The comparison of tumor cell lysis and subsequent T cell activation mediated by different CD19 TCB antibody formats was assessed on Nalm-6 (B cell precursor leukemia ALL, 70000 CD19 binding sites) and Z-138 cells (mantle cell lymphoma, 10000 CD19 binding sites). The following CD19 TCB antibody formats (all based on the 8B8 clone) were tested: molecule B (named in this example "CD19 TCB_2+1_no charges_inverted"), molecule A (named in this example "CD19 TCB_2+1_charges CD19_inverted") and molecule G (named in this example "CD19 TCB_2+1_charges CD3_inverted"). The untargeted TCB (i.e. TCB having the same format as molecule B but containing the DP47 binder that does not target any known protein) was used as negative control. Human isolated pan T cells were used as effectors and tumor lysis was detected at 21 h of incubation with the different bispecific antibody formats. Briefly, target cells were harvested, washed, and plated at density of 50 000 cells/well using round-bottom 96-well plates. Peripheral blood mononuclear cells (PBMCs) were prepared by Histopaque density centrifugation of fresh heparinized blood obtained from healthy human donors. Fresh blood was diluted with sterile PBS and layered over Histopaque gradient (Sigma, #10771). After centrifugation (450 x g, 30 minutes, room temperature, no brake), the plasma above the PBMC-containing interphase was discarded and PBMCs transferred in a new falcon tube subsequently filled with 50 ml of PBS. The mixture was centrifuged (350 x g, 10 minutes, room temperature), the supernatant discarded and the PBMC pellet washed twice with sterile PBS (centrifugation steps 300 x g and 350 x g, 10 minutes). The resulting PBMC population was counted automatically (ViCell). T cells were isolated using the pan T cell isolation Kit from Miltenyi Biotec (#130-091-156) following the instructions of the kit manual. For the tumor lysis assay, the antibodies were added at the indicated concentrations (range of 0.1 pM - 1 nM in triplicates). Furthermore, anti-human CD107a PE (Biolegend #328608) was added into the cell culture. Pan T cells were added to target cells at final E:T ratio of 3:1. Tumor cell lysis was assessed after 21 h of incubation at 37°C, 5% CO₂ by quantification of LDH released into cell supernatants by apoptotic/necrotic cells (LDH detection kit, Roche Applied Science, #11 644 793 001). Maximal lysis of the target cells (= 100%) was achieved by incubation of target cells with 1% Triton X-100. Minimal lysis (= 0%) refers to target cells co-incubated with effector cells without bispecific construct. For the assessment of T cell activation occurring upon tumor cell lysis, PBMCs were centrifuged at 400 x g for 4 min and washed with PBS containing 0.1% BSA. Surface staining for CD8 (APCCy7 anti-human CD8 Biolegend #301016), CD4 (FITC anti-human CD4, Biolegend #300506), CD69 (BV421 anti-human CD69, Biolegend #310930) and CD25 (PECy7 anti-human CD25 Biolegend #302612) was performed according to the suppliers' indications. Cells were washed twice with 150 µl/well PBS containing 0.1% BSA and fixed using 2% PFA diluted in PBS 0.1% BSA. Samples were analyzed at BD FACS CantoII.

Figure 12A and 12B show that "CD19 TCB_2+1_no charges_inverted", "CD19 TCB_2+1_charges CD19_inverted" and "CD19 TCB_2+1_charges CD3_inverted" display a comparable lysis of both Z-138 tumor cells (B) and Nalm-6 tumor cells (A). As expected, no lysis of tumor cells was observed with untargeted TCB control. Figure 12 C-N show that "CD19 TCB_2+1_no charges_inverted", "CD19 TCB_2+1_charges CD_inverted" and "CD19 TCB_2+1_charges CD3_inverted" were also comparable in inducing T cell activation after tumor killing.

**TABLE 10. EC50 values (pM) of Z-138 tumor cell lysis mediated by different CD19 TCB antibody formats.**

| Tumor cell lysis Z-138 cells (21 h) | EC50 (pM) | EC50 (ng/ml) |
|---|---|---|
| "CD19 TCB_2+1_no charges_inverted" | 3.16 | 0.62 |
| "CD19 TCB_2+1_charges CD19_inverted" | ~4.70 | 0.92 |
| "CD19 TCB_2+1_charges CD3_inverted" | 2.93 | 0.57 |

**TABLE 11. EC50 values (pM) of T cell activation upon tumor cell lysis mediated by different CD19 TCB antibody formats upon lysis of Z-138 cells.**

| Activation marker, Z-138 cells | "CD19 TCB_1_no charges_inverted" | "CD19 TCB_2+1_charges CD19_inverted" | "CD19 TCB_2+1_charges CD3_inverted" |
|---|---|---|---|
| CD107/CD8 | 1.22 | 2.00 | 1.13 |
| CD25/CD8 | 1.70 | 2.87 | 1.53 |
| CD69/CD8 | 0.82 | 1.37 | 0.70 |
| CD107/CD4 | 1.37 | 2.34 | 1.14 |
| CD25/CD4 | 2.77 | ~4.58 | 1.85 |
| CD69/CD4 | ~0.81 | 1.41 | ~0.75 |

### Lysis of CD19-expressing tumor cells and subsequent T cell activation mediated by CD19 TCB antibody containing humanized CD19 binder

The lysis of CD19-expressing tumor cells and subsequent T cell activation mediated by CD19 TCB antibodies was assessed on Z-138 cells (mantle cell lymphoma, 10000 CD19 binding sites). In this assay, molecule A containing the parental murine CD19 binder 8B8 (named in this example "CD19 TCB_8B8_charges CD19_inverted") was compared to molecule C containing the humanized CD19 binder var. 5 (named in this example "CD19 TCB_hum 8B8_charges CD19_inverted") and molecule H containing the humanized CD19 binder 2B11 (named in this example "CD19 TCB_8B8-2B11_charges CD19_inverted"). Human PBMCs were used as effectors and tumor lysis was detected at 21 h of incubation with the different bispecific antibodies. Briefly, target cells were harvested, washed, and plated at density of 50 000 cells/well using round-bottom 96-well plates. Peripheral blood mononuclear cells (PBMCs) were prepared by Histopaque density centrifugation of fresh blood obtained from healthy human donors. Fresh blood was diluted with sterile PBS and layered over Histopaque gradient (Sigma, #10771). After centrifugation (450 x g, 30 minutes, room temperature, no brake), the plasma above the PBMC-containing interphase was discarded and PBMCs transferred in a new falcon tube subsequently filled with 50 ml of PBS. The mixture was centrifuged (350 x g, 10 minutes, room temperature), the supernatant discarded and the PBMC pellet washed twice with sterile PBS (centrifugation steps 300 x g, 10 minutes). The resulting PBMC population was counted automatically (ViCell) and re-suspended in RPMI1640 medium containing 10 % FCS and 1% L-alanyl-L-glutamine (Biochrom, K0302) at 5 x 10⁶ cells/ml. For the tumor lysis assay, the antibodies were added at the indicated concentrations (range of 0.1 pM - 1000 pM in triplicates). Furthermore, anti-human CD107a PE (Biolegend #328608) was added into the cell culture. PBMCs were added to target cells at final E:T ratio of 5:1. Tumor cell lysis was assessed after 20-24 h of incubation at 37°C, 5% CO₂ by quantification of LDH released into cell supernatants by apoptotic/necrotic cells (LDH detection kit, Roche Applied Science, #11 644 793 001). Maximal lysis of the target cells (= 100%) was achieved by incubation of target cells with 2.67% Triton X-100. Minimal lysis (= 0%) refers to target cells co-incubated with effector cells without bispecific construct. For the assessment of T cell activation occurring upon tumor cell lysis, cells were centrifuged at 400 x g for 4 min and washed twice with PBS containing 2% FCS, 5 mM EDTA and 0.25% sodium acide (FACS Buffer). Surface staining for CD8 (APCCy7, anti-human CD8 Biolegend # 301016), CD4 (PerCPCy5.5 anti-human CD4, BD # 552838), CD25 (PECy7 anti-human CD25 BD # 557741) and CD69 (BV421 anti-human CD69, Biolegend # 310930) was performed according to the suppliers' instructions. Cells were washed twice with 150 µl/well FACS Buffer and fixed for 20 min at 4°C using 120 µl/well fixation buffer (BD # 554655). Samples were analyzed at BD FACS CantoII.

Figure 13A shows that all three CD19 TCB antibodies induced equal killing of CD19+ target cells. There was no significant difference between the CD19 TCB containing the parental CD19 binder 8B8 and the CD19 TCB containing the humanized CD19 binders var. 5 or 2B11. Figure 13 B-D and E-G show that the three CD19 TCB antibodies were also comparable in inducing T cell activation after tumor killing.

## Claims

1. A T cell activating bispecific antigen binding molecule comprising
(a) a first antigen binding moiety which specifically binds to CD19, wherein the first antigen binding moiety is a conventional Fab molecule;
(b) a second antigen binding moiety which specifically binds to CD3, wherein the second antigen binding moiety is a crossover Fab molecule wherein the variable domains VL and VH of the Fab light chain and the Fab heavy chain are replaced by each other;
(c) a third antigen binding moiety which specifically binds to CD19, wherein the third antigen binding moiety is a conventional Fab molecule;
(d) an Fc domain composed of a first and a second subunit capable of stable association;
wherein in the first and the third antigen binding moiety in the constant domain CL the amino acid at position 124 is substituted independently by lysine (K), arginine (R) or histidine (H), numbering according to Kabat, and the amino acid at position 123 is substituted independently by lysine (K), arginine (R) or histidine (H), numbering according to Kabat, and in the constant domain CH1 the amino acid at position 147 is substituted independently by glutamic acid (E) or aspartic acid (D), numbering according to Kabat EU index, and the amino acid at position 213 is substituted independently by glutamic acid (E) or aspartic acid (D), numbering according to Kabat EU index; and
wherein the first and the third antigen binding moiety comprise a heavy chain variable region, particularly a humanized heavy chain variable region, comprising the heavy chain complementarity determining region (HCDR) 1 of SEQ ID NO: 50, the HCDR 2 of SEQ ID NO: 51 and the HCDR 3 of SEQ ID NO: 52, and a light chain variable region, particularly a humanized light chain variable region, comprising the light chain complementarity determining region (LCDR) 1 of SEQ ID NO: 53, the LCDR 2 of SEQ ID NO: 54 and the LCDR 3 of SEQ ID NO: 55; and
wherein (i) the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety, or (ii) the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety.

2. The T cell activating bispecific antigen binding molecule according to claim 1, wherein the first and the third antigen binding moiety comprise a heavy chain variable region comprising an amino acid sequence that is at least 95%, 96%, 97%, 98%, 99% or 100% identical to the amino acid sequence of SEQ ID NO: 56 and a light chain variable region comprising an amino acid sequence that is at least 95%, 96%, 97%, 98%, 99% or 100% identical to the amino acid sequence of SEQ ID NO: 57.

3. The T cell activating bispecific antigen binding molecule according to claim 1 or 2, wherein the second antigen binding moiety comprises a heavy chain variable region comprising the HCDR 1 of SEQ ID NO: 4, the HCDR 2 of SEQ ID NO: 5, the HCDR 3 of SEQ ID NO: 6, and a light chain variable region comprising the LCDR 1 of SEQ ID NO: 8, the LCDR 2 of SEQ ID NO: 9 and the LCDR 3 of SEQ ID NO: 10.

4. The T cell activating bispecific antigen binding molecule according to any one of claims 1-3, wherein the second antigen binding moiety comprises a heavy chain variable region comprising an amino acid sequence that is at least 95%, 96%, 97%, 98%, 99% or 100% identical to the amino acid sequence of SEQ ID NO: 3 and a light chain variable region comprising an amino acid sequence that is at least 95%, 96%, 97%, 98%, 99% or 100% identical to the amino acid sequence of SEQ ID NO: 7.

5. The T cell activating bispecific antigen binding molecule according to any one of claims 1-4, wherein in the first and the third antigen binding moiety
(i) in the constant domain CL the amino acid at position 124 is substituted by lysine (K), numbering according to Kabat, and the amino acid at position 123 is substituted by arginine (R), numbering according to Kabat, and in the constant domain CH1 the amino acid at position 147 is substituted by glutamic acid (E), numbering according to Kabat EU index, and the amino acid at position 213 is substituted by glutamic acid (E), numbering according to Kabat EU index; or
(ii) in the constant domain CL the amino acid at position 124 is substituted by lysine (K), numbering according to Kabat, and the amino acid at position 123 is substituted by lysine (K), numbering according to Kabat, and in the constant domain CH1 the amino acid at position 147 is substituted by glutamic acid (E), numbering according to Kabat EU index, and the amino acid at position 213 is substituted by glutamic acid (E), numbering according to Kabat EU index.

6. The T cell activating bispecific antigen binding molecule according to any one of claims 1-5, wherein the Fc domain is an IgG, specifically an IgG₁ or IgG₄, Fc domain, and/or wherein the Fc domain is a human Fc domain.

7. The T cell activating bispecific antigen binding molecule according to any one of claims 1-6, wherein the Fc domain comprises a modification promoting the association of the first and the second subunit of the Fc domain.

8. The T cell activating bispecific antigen binding molecule of claim 7, wherein in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable; and wherein optionally
(i) said amino acid residue having a larger side chain volume is selected from the group consisting of arginine (R), phenylalanine (F), tyrosine (Y), and tryptophan (W), and said amino acid residue having a smaller side chain volume is selected from the group consisting of alanine (A), serine (S), threonine (T), and valine (V);
(ii) in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V), and optionally in the second subunit of the Fc domain additionally the threonine residue at position 366 is replaced with a serine residue (T366S) and the leucine residue at position 368 is replaced with an alanine residue (L368A), numberings according to Kabat EU index;
(iii) in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C), numberings according to Kabat EU index; and/or
(iv) the first subunit of the Fc domain comprises amino acid substitutions S354C and T366W, and the second subunit of the Fc domain comprises amino acid substitutions Y349C, T366S, L368A and Y407V, numberings according to Kabat EU index.

9. The T cell activating bispecific antigen binding molecule according to any one of claims 1-8, wherein
(i) the Fc domain exhibits reduced binding affinity to an Fc receptor, particularly an Fcγ receptor, and/or reduced effector function, particularly antibody-dependent cell-mediated cytotoxicity (ADCC), as compared to a native IgG₁ Fc domain;
(ii) the Fc domain comprises one or more amino acid substitution that reduces binding to an Fc receptor and/or effector function, particularly wherein said one or more amino acid substitution is at one or more position selected from the group of L234, L235, and P329, numberings according to Kabat EU index; and/or
(iii) each subunit of the Fc domain comprises three amino acid substitutions that reduce binding to an activating Fc receptor and/or effector function wherein said amino acid substitutions are L234A, L235A and P329G, numberings according to Kabat EU index.

10. One or more isolated polynucleotide encoding the T cell activating bispecific antigen binding molecule of any one of claims 1 to 9.

11. One or more vector, particularly expression vector, comprising the polynucleotide(s) of claim 10.

12. A host cell comprising the polynucleotide(s) of claim 10 or the vector(s) of claim 11.

13. A method of producing a T cell activating bispecific antigen binding molecule capable of specific binding to CD19 and CD3, comprising the steps of a) culturing the host cell of claim 12 under conditions suitable for the expression of the T cell activating bispecific antigen binding molecule and b) optionally recovering the T cell activating bispecific antigen binding molecule.

14. A pharmaceutical composition comprising the T cell activating bispecific antigen binding molecule of any one of claims 1 to 9 and a pharmaceutically acceptable carrier.

15. The T cell activating bispecific antigen binding molecule of any one of claims 1 to 9 or the pharmaceutical composition of claim 14 for use
(i) as a medicament;
(ii) in the treatment of a disease in an individual in need thereof; or
(iii) in the treatment of a disease in an individual in need thereof, wherein the disease is cancer.

## Patentansprüche

1. Bispezifisches antigenbindendes Molekül zur T-Zellen-Aktivierung, umfassend
(a) eine erste antigenbindende Einheit, die spezifisch an CD19 bindet, wobei die erste antigenbindende Einheit ein herkömmliches Fab-Molekül ist;
(b) eine zweite antigenbindende Einheit, die spezifisch an CD3 bindet, wobei die zweite antigenbindende Einheit ein Crossover-Fab-Molekül ist, wobei die variablen Domänen VL und VH der leichten Fab-Kette und der schweren Fab-Kette gegeneinander ausgetauscht sind;
(c) eine dritte antigenbindende Einheit, die spezifisch an CD19 bindet, wobei die dritte antigenbindende Einheit ein herkömmliches Fab-Molekül ist;
(d) eine Fc-Domäne, die aus einer ersten und einer zweiten Untereinheit besteht, die zu stabiler Assoziation fähig sind;
wobei in der ersten und der dritten antigenbindenden Einheit in der konstanten Domäne CL die Aminosäure an Position 124 unabhängig durch Lysin (K), Arginin (R) oder Histidin (H), Nummerierung nach Kabat, substituiert ist und die Aminosäure an Position 123 unabhängig durch Lysin (K), Arginin (R) oder Histidin (H), Nummerierung nach Kabat, substituiert ist, und in der konstanten Domäne CH1 die Aminosäure an Position 147 unabhängig durch Glutaminsäure (E) oder Asparaginsäure (D), Nummerierung nach Kabat-EU-Index, substituiert ist und die Aminosäure an Position 213 unabhängig durch Glutaminsäure (E) oder Asparaginsäure (D), Nummerierung nach Kabat-EU-Index, substituiert ist; und
wobei die erste und die dritte antigenbindende Einheit eine variable Region der schweren Kette, insbesondere eine humanisierte variable Region der schweren Kette, umfassend die komplementaritätsbestimmende Region der schweren Kette (HCDR) 1 von SEQ ID NO: 50, die HCDR 2 von SEQ ID NO: 51 und die HCDR 3 von SEQ ID NO: 52, und eine variable Region der leichten Kette, insbesondere eine humanisierte variable Region der leichten Kette, umfassend die komplementaritätsbestimmende Region der leichten Kette (LCDR) 1 von SEQ ID NO: 53, die LCDR 2 von SEQ ID NO: 54 und die LCDR 3 von SEQ ID NO: 55, umfassen; und
wobei (i) die zweite und die dritte antigenbindende Einheit jeweils am C-Terminus der schweren Fab-Kette an den N-Terminus einer der Untereinheiten der Fc-Domäne fusioniert sind und die erste antigenbindende Einheit am C-Terminus der schweren Fab-Kette an den N-Terminus der schweren Fab-Kette der zweiten antigenbindenden Einheit fusioniert ist, oder (ii) die erste und die dritte antigenbindende Einheit jeweils am C-Terminus der schweren Fab-Kette an den N-Terminus einer der Untereinheiten der Fc-Domäne fusioniert sind und die zweite antigenbindende Einheit am C-Terminus der schweren Fab-Kette an den N-Terminus der schweren Fab-Kette der ersten antigenbindenden Einheit fusioniert ist.

2. Bispezifisches antigenbindendes Molekül zur T-Zellen-Aktivierung nach Anspruch 1, wobei die erste und die dritte antigenbindende Einheit eine variable Region der schweren Kette, umfassend eine Aminosäuresequenz, die zu mindestens 95 %, 96 %, 97 %, 98 %, 99 % oder 100 % mit der Aminosäuresequenz von SEQ ID NO: 56 identisch ist, und eine variable Region der leichten Kette, umfassend eine Aminosäuresequenz, die zu mindestens 95 %, 96 %, 97 %, 98 %, 99 % oder 100 % mit der Aminosäuresequenz von SEQ ID NO: 57 identisch ist, umfassen.

3. Bispezifisches antigenbindendes Molekül zur T-Zellen-Aktivierung nach Anspruch 1 oder 2, wobei die zweite antigenbindende Einheit eine variable Region der schweren Kette, umfassend die HCDR 1 von SEQ ID NO: 4, die HCDR 2 von SEQ ID NO: 5, die HCDR 3 von SEQ ID NO: 6, und eine variable Region der leichten Kette, umfassend die LCDR 1 von SEQ ID NO: 8, die LCDR 2 von SEQ ID NO: 9 und die LCDR 3 von SEQ ID NO: 10, umfasst.

4. Bispezifisches antigenbindendes Molekül zur T-Zellen-Aktivierung nach einem der Ansprüche 1-3, wobei die zweite antigenbindende Einheit eine variable Region der schweren Kette, umfassend eine Aminosäuresequenz, die zu mindestens 95 %, 96 %, 97 %, 98 %, 99 % oder 100 % mit der Aminosäuresequenz von SEQ ID NO: 3 identisch ist, und eine variable Region der leichten Kette, umfassend eine Aminosäuresequenz, die zu mindestens 95 %, 96 %, 97 %, 98 %, 99 % oder 100 % mit der Aminosäuresequenz von SEQ ID NO: 7 identisch ist, umfasst.

5. Bispezifisches antigenbindendes Molekül zur T-Zellen-Aktivierung nach einem der Ansprüche 1-4, wobei in der ersten und der dritten antigenbindenden Einheit
(i) in der konstanten Domäne CL die Aminosäure an Position 124 durch Lysin (K), Nummerierung nach Kabat, substituiert ist und die Aminosäure an Position 123 durch Arginin (R), Nummerierung nach Kabat, substituiert ist, und in der konstanten Domäne CH1 die Aminosäure an Position 147 durch Glutaminsäure (E), Nummerierung nach Kabat-EU-Index, substituiert ist und die Aminosäure an Position 213 durch Glutaminsäure (E), Nummerierung nach Kabat-EU-Index, substituiert ist; oder
(ii) in der konstanten Domäne CL die Aminosäure an Position 124 durch Lysin (K), Nummerierung nach Kabat, substituiert ist und die Aminosäure an Position 123 durch Lysin (K), Nummerierung nach Kabat, substituiert ist, und in der konstanten Domäne CH1 die Aminosäure an Position 147 durch Glutaminsäure (E), Nummerierung nach Kabat-EU-Index, substituiert ist und die Aminosäure an Position 213 durch Glutaminsäure (E), Nummerierung nach Kabat-EU-Index, substituiert ist.

6. Bispezifisches antigenbindendes Molekül zur T-Zellen-Aktivierung nach einem der Ansprüche 1-5, wobei die Fc-Domäne eine IgG-, spezifisch eine IgG₁- oder IgG₄-Fc-Domäne ist und/oder wobei die Fc-Domäne eine humane Fc-Domäne ist.

7. Bispezifisches antigenbindendes Molekül zur T-Zellen-Aktivierung nach einem der Ansprüche 1-6, wobei die Fc-Domäne eine Modifikation umfasst, die die Assoziation der ersten und der zweiten Untereinheit der Fc-Domäne fördert.

8. Bispezifisches antigenbindendes Molekül zur T-Zellen-Aktivierung nach Anspruch 7, wobei in der CH3-Domäne der ersten Untereinheit der Fc-Domäne ein Aminosäurerest durch einen Aminosäurerest mit einem größeren Seitenkettenvolumen ausgetauscht ist, wodurch eine Protuberanz innerhalb der CH3-Domäne der ersten Untereinheit erzeugt wird, die in einem Hohlraum innerhalb der CH3-Domäne der zweiten Untereinheit positionierbar ist, und in der CH3-Domäne der zweiten Untereinheit der Fc-Domäne ein Aminosäurerest durch einen Aminosäurerest mit einem kleineren Seitenkettenvolumen ausgetauscht ist, wodurch ein Hohlraum innerhalb der CH3-Domäne der zweiten Untereinheit erzeugt wird, innerhalb dessen die Protuberanz innerhalb der CH3-Domäne der ersten Untereinheit positionierbar ist; und wobei optional
(i) der Aminosäurerest mit einem größeren Seitenkettenvolumen ausgewählt ist aus der Gruppe bestehend aus Arginin (R), Phenylalanin (F), Tyrosin (Y) und Tryptophan (W) und der Aminosäurerest mit einem kleineren Seitenkettenvolumen ausgewählt ist aus der Gruppe bestehend aus Alanin (A), Serin (S), Threonin (T) und Valin (V);
(ii) in der CH3-Domäne der ersten Untereinheit der Fc-Domäne der Threoninrest an Position 366 durch einen Tryptophanrest ausgetauscht ist (T366W) und in der CH3-Domäne der zweiten Untereinheit der Fc-Domäne der Tyrosinrest an Position 407 durch einen Valinrest ausgetauscht ist (Y407V), und optional in der zweiten Untereinheit der Fc-Domäne zusätzlich der Threoninrest an Position 366 durch einen Serinrest ausgetauscht ist (T366S) und der Leucinrest an Position 368 durch einen Alaninrest ausgetauscht ist (L368A), Nummerierungen nach Kabat-EU-Index;
(iii) in der ersten Untereinheit der Fc-Domäne zusätzlich der Serinrest an Position 354 durch einen Cysteinrest ausgetauscht ist (S354C) oder der Glutaminsäurerest an Position 356 durch einen Cysteinrest ausgetauscht ist (E356C), und in der zweiten Untereinheit der Fc-Domäne zusätzlich der Tyrosinrest an Position 349 durch einen Cysteinrest ausgetauscht ist (Y349C), Nummerierungen nach Kabat-EU-Index; und/oder
(iv) die erste Untereinheit der Fc-Domäne die Aminosäuresubstitutionen S354C und T366W umfasst und die zweite Untereinheit der Fc-Domäne die Aminosäuresubstitutionen Y349C, T366S, L368A und Y407V umfasst, Nummerierungen nach Kabat-EU-Index.

9. Bispezifisches antigenbindendes Molekül zur T-Zellen-Aktivierung nach einem der Ansprüche 1-8, wobei
(i) die Fc-Domäne im Vergleich zu einer nativen IgG₁-Fc-Domäne reduzierte Bindungsaffinität zu einem Fc-Rezeptor, insbesondere einem Fcγ-Rezeptor, und/oder reduzierte Effektorfunktion, insbesondere antikörperabhängige zellvermittelte Zytotoxizität (ADCC), aufweist;
(ii) die Fc-Domäne eine oder mehrere Aminosäuresubstitutionen umfasst, die die Bindung an einen Fc-Rezeptor und/oder die Effektorfunktion reduzieren, insbesondere wobei die eine oder die mehreren Aminosäuresubstitutionen an einer oder mehreren Positionen, ausgewählt aus der Gruppe von L234, L235 und P329, Nummerierungen nach Kabat-EU-Index, vorliegen; und/oder
(iii) jede Untereinheit der Fc-Domäne drei Aminosäuresubstitutionen umfasst, die die Bindung an einen aktivierenden Fc-Rezeptor und/oder die Effektorfunktion reduzieren, wobei die Aminosäuresubstitutionen L234A, L235A und P329G, Nummerierungen nach Kabat-EU-Index, sind.

10. Ein oder mehrere isolierte Polynukleotide, die das bispezifische antigenbindende Molekül zur T-Zellen-Aktivierung nach einem der Ansprüche 1 bis 9 kodieren.

11. Ein oder mehrere Vektoren, insbesondere Expressionsvektoren, umfassend das/die Polynukleotid(e) nach Anspruch 10.

12. Wirtszelle, umfassend das/die Polynukleotid(e) nach Anspruch 10 oder den/die Vektor(en) nach Anspruch 11.

13. Verfahren zur Herstellung eines bispezifischen antigenbindenden Moleküls zur T-Zellen-Aktivierung, das zur spezifischen Bindung an CD19 und CD3 fähig ist, umfassend die Schritte a) Kultivieren der Wirtszelle nach Anspruch 12 unter Bedingungen, die für die Expression des bispezifischen antigenbindenden Moleküls zur T-Zellen-Aktivierung geeignet sind, und b) optionales Gewinnen des bispezifischen antigenbindenden Moleküls zur T-Zellen-Aktivierung.

14. Pharmazeutische Zusammensetzung, umfassend das bispezifische antigenbindende Molekül zur T-Zellen-Aktivierung nach einem der Ansprüche 1 bis 9 und einen pharmazeutisch verträglichen Träger.

15. Bispezifisches antigenbindendes Molekül zur T-Zellen-Aktivierung nach einem der Ansprüche 1 bis 9 oder pharmazeutische Zusammensetzung nach Anspruch 14 zur Verwendung
(i) als Medikament;
(ii) bei der Behandlung einer Krankheit bei einem Individuum, das dessen bedarf; oder
(iii) bei der Behandlung einer Krankheit bei einem Individuum, das dessen bedarf, wobei die Krankheit Krebs ist.

## Revendications

1. Molécule bispécifique de liaison à l'antigène activant les lymphocytes T comprenant
(a) une première fraction de liaison à l'antigène qui se lie spécifiquement à CD19, dans laquelle la première fraction de liaison à l'antigène est une molécule Fab classique ;
(b) une deuxième fraction de liaison à l'antigène qui se lie spécifiquement à CD3, dans laquelle la deuxième fraction de liaison à l'antigène est une molécule Fab croisée dans laquelle les domaines variables VL et VH de la chaîne légère de Fab et de la chaîne lourde de Fab sont remplacés l'un par l'autre ;
(c) une troisième fraction de liaison à l'antigène qui se lie spécifiquement à CD19, dans laquelle la troisième fraction de liaison à l'antigène est une molécule Fab classique ;
(d) un domaine Fc composé d'un premier et d'un second sous-motif capables d'une association stable ;
dans laquelle dans la première et la troisième fraction de liaison à l'antigène dans le domaine constant CL l'acide aminé en position 124 est substitué indépendamment par la lysine (K), l'arginine (R) ou l'histidine (H), numérotation selon Kabat, et l'acide aminé en position 123 est substitué indépendamment par la lysine (K), l'arginine (R) ou l'histidine (H), numérotation selon Kabat, et dans le domaine constant CH1 l'acide aminé en position 147 est substitué indépendamment par l'acide glutamique (E) ou l'acide aspartique (D), numérotation selon l'indice EU de Kabat, et l'acide aminé en position 213 est substitué indépendamment par l'acide glutamique (E) ou l'acide aspartique (D), numérotation selon l'indice EU de Kabat ; et dans laquelle la première et la troisième fraction de liaison à l'antigène comprennent une région variable de chaîne lourde, en particulier une région variable de chaîne lourde humanisée, comprenant la région déterminant la complémentarité de chaîne lourde (HCDR) 1 de SEQ ID NO : 50, la HCDR 2 de SEQ ID NO : 51 et la HCDR 3 de SEQ ID NO : 52, et une région variable de chaîne légère, en particulier une région variable de chaîne légère humanisée, comprenant la région déterminant la complémentarité de chaîne légère (LCDR) 1 de SEQ ID NO : 53, la LCDR 2 de SEQ ID NO : 54 et la LCDR 3 de SEQ ID NO : 55 ; et
dans laquelle (i) la deuxième et la troisième fraction de liaison à l'antigène sont chacune fusionnées au niveau de l'extrémité C-terminale de la chaîne lourde de Fab à l'extrémité N-terminale de l'un des sous-motifs du domaine Fc, et la première fraction de liaison à l'antigène est fusionnée au niveau de l'extrémité C-terminale de la chaîne lourde de Fab à l'extrémité N-terminale de la chaîne lourde de Fab de la deuxième fraction de liaison à l'antigène, ou (ii) la première et la troisième fraction de liaison à l'antigène sont chacune fusionnées au niveau de l'extrémité C-terminale de la chaîne lourde de Fab à l'extrémité N-terminale de l'un des sous-motifs du domaine Fc, et la deuxième fraction de liaison à l'antigène est fusionnée au niveau de l'extrémité C-terminale de la chaîne lourde de Fab à l'extrémité N-terminale de la chaîne lourde de Fab de la première fraction de liaison à l'antigène.

2. Molécule bispécifique de liaison à l'antigène activant les lymphocytes T selon la revendication 1, dans laquelle la première et la troisième fraction de liaison à l'antigène comprennent une région variable de chaîne lourde comprenant une séquence d'acides aminés qui est au moins 95 %, 96 %, 97 %, 98 %, 99 % ou 100 % identique à la séquence d'acides aminés de SEQ ID NO : 56 et une région variable de chaîne légère comprenant une séquence d'acides aminés qui est au moins 95 %, 96 %, 97 %, 98 %, 99 % ou 100 % identique à la séquence d'acides aminés de SEQ ID NO : 57.

3. Molécule bispécifique de liaison à l'antigène activant les lymphocytes T selon la revendication 1 ou 2, dans laquelle la deuxième fraction de liaison à l'antigène comprend une région variable de chaîne lourde comprenant la HCDR 1 de SEQ ID NO : 4, la HCDR 2 de SEQ ID NO : 5, la HCDR 3 de SEQ ID NO : 6, et une région variable de chaîne légère comprenant la LCDR 1 de SEQ ID NO : 8, la LCDR 2 de SEQ ID NO : 9 et la LCDR 3 de SEQ ID NO : 10.

4. Molécule bispécifique de liaison à l'antigène activant les lymphocytes T selon l'une quelconque des revendications 1 à 3, dans laquelle la deuxième fraction de liaison à l'antigène comprend une région variable de chaîne lourde comprenant une séquence d'acides aminés qui est au moins 95 %, 96 %, 97 %, 98 %, 99 % ou 100 % identique à la séquence d'acides aminés de SEQ ID NO : 3 et une région variable de chaîne légère comprenant une séquence d'acides aminés qui est au moins 95 %, 96 %, 97 %, 98 %, 99 % ou 100 % identique à la séquence d'acides aminés de SEQ ID NO : 7.

5. Molécule bispécifique de liaison à l'antigène activant les lymphocytes T selon l'une quelconque des revendications 1 à 4, dans laquelle dans la première et la troisième fraction de liaison à l'antigène
(i) dans le domaine constant CL l'acide aminé en position 124 est substitué par la lysine (K), numérotation selon Kabat, et l'acide aminé en position 123 est substitué par l'arginine (R), numérotation selon Kabat, et dans le domaine constant CH1 l'acide aminé en position 147 est substitué par l'acide glutamique (E), numérotation selon l'indice EU de Kabat, et l'acide aminé en position 213 est substitué par l'acide glutamique (E), numérotation selon l'indice EU de Kabat ; ou
(ii) dans le domaine constant CL l'acide aminé en position 124 est substitué par la lysine (K), numérotation selon Kabat, et l'acide aminé en position 123 est substitué par la lysine (K), numérotation selon Kabat, et dans le domaine constant CH1 l'acide aminé en position 147 est substitué par l'acide glutamique (E), numérotation selon l'indice EU de Kabat, et l'acide aminé en position 213 est substitué par l'acide glutamique (E), numérotation selon l'indice EU de Kabat.

6. Molécule bispécifique de liaison à l'antigène activant les lymphocytes T selon l'une quelconque des revendications 1 à 5, dans laquelle le domaine Fc est un domaine Fc d'une IgG, spécifiquement d'une IgG₁ ou d'une IgG₄, et/ou dans laquelle le domaine Fc est un domaine Fc humain.

7. Molécule bispécifique de liaison à l'antigène activant les lymphocytes T selon l'une quelconque des revendications 1 à 6, dans laquelle le domaine Fc comprend une modification favorisant l'association du premier et du second sous-motif du domaine Fc.

8. Molécule bispécifique de liaison à l'antigène activant les lymphocytes T selon la revendication 7, dans laquelle dans le domaine CH3 du premier sous-motif du domaine Fc un résidu d'acide aminé est remplacé par un résidu d'acide aminé ayant un plus grand volume de chaîne latérale, générant ainsi une protubérance au sein du domaine CH3 du premier sous-motif qui peut être positionnée dans une cavité au sein du domaine CH3 du second sous-motif, et dans le domaine CH3 du second sous-motif du domaine Fc un résidu d'acide aminé est remplacé par un résidu d'acide aminé ayant un plus petit volume de chaîne latérale, générant ainsi une cavité au sein du domaine CH3 du second sous-motif au sein de laquelle peut être positionnée la protubérance au sein du domaine CH3 du premier sous-motif ; et dans laquelle éventuellement (i) ledit résidu d'acide aminé ayant un plus grand volume de chaîne latérale est choisi dans le groupe constitué par l'arginine (R), la phénylalanine (F), la tyrosine (Y) et le tryptophane (W), et ledit résidu d'acide aminé ayant un plus petit volume de chaîne latérale est choisi dans le groupe constitué par l'alanine (A), la sérine (S), la thréonine (T) et la valine (V) ;
(ii) dans le domaine CH3 du premier sous-motif du domaine Fc le résidu thréonine en position 366 est remplacé par un résidu tryptophane (T366W), et dans le domaine CH3 du second sous-motif du domaine Fc le résidu tyrosine en position 407 est remplacé par un résidu valine (Y407V), et éventuellement dans le second sous-motif du domaine Fc en outre le résidu thréonine en position 366 est remplacé par un résidu sérine (T366S) et le résidu leucine en position 368 est remplacé par un résidu alanine (L368A), numérotations selon l'indice EU de Kabat ;
(iii) dans le premier sous-motif du domaine Fc en outre le résidu sérine en position 354 est remplacé par un résidu cystéine (S354C) ou le résidu acide glutamique en position 356 est remplacé par un résidu cystéine (E356C), et dans le second sous-motif du domaine Fc en outre le résidu tyrosine en position 349 est remplacé par un résidu cystéine (Y349C), numérotations selon l'indice EU de Kabat ; et/ou
(iv) le premier sous-motif du domaine Fc comprend les substitutions d'acides aminés S354C et T366W, et le second sous-motif du domaine Fc comprend les substitutions d'acides aminés Y349C, T366S, L368A et Y407V, numérotations selon l'indice EU de Kabat.

9. Molécule bispécifique de liaison à l'antigène activant les lymphocytes T selon l'une quelconque des revendications 1 à 8, dans laquelle
(i) le domaine Fc présente une affinité de liaison réduite vis-à-vis d'un récepteur Fc, en particulier d'un récepteur Fcγ, et/ou une fonction effectrice réduite, en particulier une cytotoxicité à médiation cellulaire dépendante des anticorps (ADCC), par comparaison avec un domaine Fc d'IgG₁ native ;
(ii) le domaine Fc comprend une ou plusieurs substitutions d'acides aminés qui réduisent la liaison à un récepteur Fc et/ou une fonction effectrice, en particulier dans laquelle lesdites une ou plusieurs substitutions d'acides aminés sont en une ou plusieurs positions choisies dans le groupe constitué par L234, L235 et P329, numérotations selon l'indice EU de Kabat ; et/ou
(iii) chaque sous-motif du domaine Fc comprend trois substitutions d'acides aminés qui réduisent la liaison à un récepteur Fc d'activation et/ou une fonction effectrice dans laquelle lesdites substitutions d'acides aminés sont L234A, L235A et P329G, numérotations selon l'indice EU de Kabat.

10. Polynucléotide(s) isolé(s) codant pour la molécule bispécifique de liaison à l'antigène activant les lymphocytes T selon l'une quelconque des revendications 1 à 9.

11. Vecteur(s), en particulier vecteur d'expression, comprenant le(s) polynucléotide(s) selon la revendication 10.

12. Cellule hôte comprenant le(s) polynucléotide(s) selon la revendication 10 ou le(s) vecteur(s) selon la revendication 11.

13. Procédé de production d'une molécule bispécifique de liaison à l'antigène activant les lymphocytes T capable de se lier spécifiquement à CD19 et CD3, comprenant les étapes de a) culture de la cellule hôte selon la revendication 12 dans des conditions appropriées pour l'expression de la molécule bispécifique de liaison à l'antigène activant les lymphocytes T et b) éventuellement récupération de la molécule bispécifique de liaison à l'antigène activant les lymphocytes T.

14. Composition pharmaceutique comprenant la molécule bispécifique de liaison à l'antigène activant les lymphocytes T selon l'une quelconque des revendications 1 à 9 et un véhicule pharmaceutiquement acceptable.

15. Molécule bispécifique de liaison à l'antigène activant les lymphocytes T selon l'une quelconque des revendications 1 à 9 ou composition pharmaceutique selon la revendication 14 pour une utilisation
(i) comme médicament ;
(ii) dans le traitement d'une maladie chez un individu en ayant besoin ; ou
(iii) dans le traitement d'une maladie chez un individu en ayant besoin, dans laquelle la maladie est un cancer.
